# EUROPEAN PATENT APPLICATION

(11) **EP 4 545 517 A1**
(43) Date of publication of application: **30.04.2025**
(21) Application number: 23826512.8
(22) Date of filing: 21.06.2023
(51) Int. Cl.: C07D 213/89, C07D 405/12, C07D 237/20, A61K 31/44, A61K 31/505, A61P 29/00

(54) **NAV1.8 INHIBITOR**

(30) Priority: 22.06.2022 CN 202210714208; 13.06.2023 CN 202310704035
(71) Applicant: Wuhan Humanwell Innovative Drug Research and Development Center Limited Company, Wuhan, Hubei 430075 (CN)
(72) Inventor: ZHANG, Xuejun, Wuhan, Hubei 430075 (CN); LI, Jinping, Wuhan, Hubei 430075 (CN); JIA, Yimin, Wuhan, Hubei 430075 (CN); CHEN, Haomin, Wuhan, Hubei 430075 (CN); LI, Qun, Wuhan, Hubei 430075 (CN); LI, Yang, Wuhan, Hubei 430075 (CN); YANG, Jun, Wuhan, Hubei 430075 (CN); LI, Li'e, Wuhan, Hubei 430075 (CN)
(74) Representative: Gille Hrabal Partnerschaftsgesellschaft mbB Patentanwälte
(86) International application number: PCT/CN2023/101712
(87) International publication number: WO 2023/246867

(57) **Abstract**

The present invention provides a Nav1.8 inhibitor. Specifically, the present invention provides a compound represented by formula I, a tautomer, a stereoisomer, a hydrate, a solvate, a pharmaceutically acceptable salt or a prodrug thereof, a preparation method therefor, and a use thereof in the preparation of a drug.

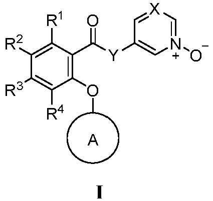

## Description

The present disclosure claims priority to:
A prior application with the application No. 202210714208.4 entitled "NAV1.8 INHIBITOR" and filed with China National Intellectual Property Administration on Jun. 22, 2022.

A prior application with the application No. 202310704035.2 entitled "NAV1.8 INHIBITOR" and filed with China National Intellectual Property Administration on Jun. 13, 2023.

The prior applications are incorporated herein by reference in their entirety.

### TECHNICAL FIELD

The present disclosure relates to the field of pharmaceuticals and a Nav1.8 inhibitor. Specifically, the present disclosure relates to use of a benzamide compound, or a tautomer, a stereoisomer, a hydrate, a solvate, a pharmaceutically acceptable salt, or a prodrug thereof, and a pharmaceutical composition thereof as a Nav1.8 inhibitor and for manufacturing a medicament for the treatment, alleviation, or prevention of pain.

### BACKGROUND

Pain is "an unpleasant and emotional sensation with substantial or potential tissue damage, which is a subjective sensation". Pain can be used as a warning signal to remind the body of potential danger, and has an indispensable protective effect on normal life activities of the body. Meanwhile, pain is also a common clinical symptom. After external stimulation causing pain disappears, strong or persistent pain can cause disorder of physiological functions and seriously affect the life quality of an organism. According to statistics, about one fifth of people worldwide have moderate to severe chronic pain. The global analgesic market was about $36 billion in 2018, and is expected to reach $56 billion in 2023. The acute moderate and severe pains will steadily increase at a compound annual growth rate of 2.5% in the future, and the chronic pain market will increase at a compound annual growth rate of about 18%. The chronic pain is the main driving force for the continuous growth of the global pain market in the next decade.

Pain originates in nociceptors of the peripheral nervous system. This is a free nerve ending widely distributed in the skin, muscle, joint and visceral tissues throughout the body, which can convert a sensed thermal, mechanical or chemical stimulus into nerve impulses (action potentials) and transmit them via afferent nerve fibers to its cell body part located in the dorsal root ganglia (DRG) and finally to the higher nerve center, causing pain sensation. The generation and conduction of action potentials in neurons depend on voltage-gated sodium channels (NaVs) on the cell membrane. When the cell membrane is depolarized, the sodium ion channel is activated and opens to cause the inflow of sodium ions, which further depolarizes the cell membrane, resulting in the generation of an action potential. Therefore, inhibition of aberrant sodium channel activity contributes to the treatment and alleviation of pain.

Human sodium ion is a transmembrane ion channel protein consisting of an α subunit with the molecular weight of 260 kD and a β subunit with the molecular weight of 30-40 kD, and can be divided into 9 subtypes according to the difference of the α subunit, i.e., Nav1.1-Nav1.9. Nav1.5, Nav1.8, and Nav1.9 are tetrodotoxin (TTX)-insensitive sodium channels, Nav1.5 mainly exists in cardiac muscle cells, and Nav1.8 and Navl.9 exist in peripheral nervous systems. Among them, Nav1.8 is an important ion channel involved in chronic pain, atrial fibrillation, and Budd-Chiari syndrome, and is a high-selectivity action target for treating pain.

The Nav1.8-encoding gene is SCN10A, which is located in the human chromosome 3p21-22 region, and mainly encodes α subunits. It was found that the identity of human and rat Nav1.8 genes is up to 93%. Nav1.8 mainly exists in trigeminal ganglion neurons and DRG neurons, and has the electrophysiological characteristics of slow inactivation and rapid recovery. In the neurons expressing Nav1.8, the increase of action potentials is mainly composed of Nav1.8 current. In neuropathic pain model, nerve injury will increase the expression level of Nav1.8 in axons and neuron cell bodies. The use of Nav1.8 antisense oligonucleotides reduces the expression of Nav1.8 and can also significantly reduce pain. After intra-paw injection of carrageenan in rats, the expression of Nav1.8 in DRG neurons is increased. Nav1.8-knockout mice can not exhibit normal visceral inflammatory pain. After a functional gain mutation of the human Nav1.8 gene, peripheral neuralgia is caused. According to a series of animal experiments and human genetic evidence, selective inhibition of Nav1.8 has the potential of becoming a novel analgesic therapy, and can be used for treating various pain types such as inflammatory pain, neuropathic pain, postsurgical pain, cancer pain, and the like.

A major drawback of some known Nav's inhibitors is their poor therapeutic window, which may be the result of their lack of isotype selectivity. Since Nav1.8 is primarily restricted to pain-sensing neurons, selective Nav1.8 blockers are unlikely to induce the adverse effects common to non-selective Nav's blockers. Therefore, the field still needs to develop new Nav1.8 selective inhibitors, preferably Nav channel inhibitors with better Nav1.8 selectivity, better effectiveness, increased metabolic stability, increased solubility, and fewer side effects.

### SUMMARY

The present disclosure aims to provide a Nav1.8 inhibitor, which can be used for manufacturing a medicament for the treatment, alleviation, or prevention of pain. The pain comprises acute pain, chronic pain, inflammatory pain, cancer pain, neuropathic pain, musculoskeletal pain, primary pain, intestinal pain, idiopathic pain, and the like.

In a first aspect of the present disclosure, the present disclosure provides a compound, which is a compound represented by formula (I), or a tautomer, a stereoisomer, a hydrate, a solvate, a pharmaceutically acceptable salt, or a prodrug thereof: 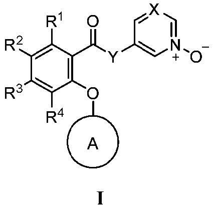 wherein
X is independently selected from N and CR^{A};
R^{A} is selected from H, D, C₁-C₆ alkyl, and C₃-C₆ cycloalkyl;
Y is independently selected from -NH- and 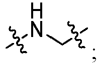
ring A is independently selected from 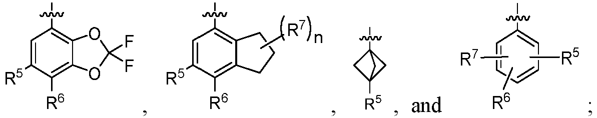 n is independently selected from 0, 1, 2, 3, 4, 5, and 6;
R¹, R², R³, R⁴, R⁵, R⁶, and R⁷ are each independently selected from H, D, C₁-C₆ alkyl, C₁-C₆ alkyl substituted with one or more R¹¹, C₂-C₆ alkenyl, C₂-C₆ alkenyl substituted with one or more R¹², C₂-C₆ alkynyl, C₂-C₆ alkynyl substituted with one or more R¹³, -O-(C₁-C₆ alkyl), -O-(C₁-C₆ alkyl) substituted with one or more R¹⁴, -S-(C₁-C₆ alkyl), -S-(C₁-C₆ alkyl) substituted with one or more R¹⁵, C₃-C₆ cycloalkyl, C₃-C₆ cycloalkyl substituted with one or more R¹⁶, 4-8 membered heterocycloalkyl, 4-8 membered heterocycloalkyl substituted with one or more R¹⁷, 6-10 membered aryl, 6-10 membered aryl substituted with one or more R¹⁸, 5-8 membered heteroaryl, 5-8 membered heteroaryl substituted with one or more R¹⁹, -NR^{1a}R^{1b}, halogen, hydroxy, cyano, nitro, and -SF₅, and when a plurality of the substituents R¹¹, R¹², R¹³, R¹⁴, R¹⁵, R¹⁶, R¹⁷, R¹⁸, and R¹⁹ are present, the substituents are the same or different; R^{1a} and R^{1b} are each independently selected from H, C₁-C₆ alkyl, and C₁-C₆ alkyl substituted with one or more R^{1a1}, and when a plurality of the substituents R^{1a}, R^{1b}, and R^{1a1} are present, the substituents are the same or different;
R¹¹, R¹², R¹³, R¹⁴, R¹⁵, R¹⁶, R¹⁷, R¹⁸, R¹⁹, and R^{1a1} are each independently selected from halogen, amino, hydroxy, cyano, deuterium, and nitro.

In an optional embodiment of the present disclosure, the compound represented by formula (I) is a compound represented by formula (II), 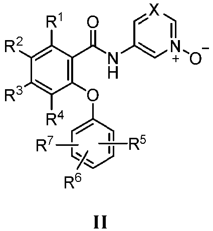 wherein
X is independently selected from N and CH;
R¹, R², R³, R⁴, R⁵, R⁶, and R⁷ are each independently selected from H, D, C₁-C₆ alkyl, C₁-C₆ alkyl substituted with one or more R¹¹, C₂-C₆ alkenyl, C₂-C₆ alkenyl substituted with one or more R¹², C₂-C₆ alkynyl, C₂-C₆ alkynyl substituted with one or more R¹³, -O-(C₁-C₆ alkyl), -O-(C₁-C₆ alkyl) substituted with one or more R¹⁴, -S-(C₁-C₆ alkyl), -S-(C₁-C₆ alkyl) substituted with one or more R¹⁵, C₃-C₆ cycloalkyl, C₃-C₆ cycloalkyl substituted with one or more R¹⁶, 4-8 membered heterocycloalkyl, 4-8 membered heterocycloalkyl substituted with one or more R¹⁷, 6-10 membered aryl, 6-10 membered aryl substituted with one or more R¹⁸, 5-8 membered heteroaryl, 5-8 membered heteroaryl substituted with one or more R¹⁹, -NR^{1a}R^{1b}, halogen, hydroxy, cyano, nitro, and -SF₅, and when a plurality of the substituents R¹¹, R¹², R¹³, R¹⁴, R¹⁵, R¹⁶, R¹⁷, R¹⁸, and R¹⁹ are present, the substituents are the same or different; R^{1a} and R^{1b} are each independently selected from H, C₁-C₆ alkyl, and C₁-C₆ alkyl substituted with one or more R^{1a1}, and when a plurality of the substituents R^{1a}, R^{1b}, and R^{1a1} are present, the substituents are the same or different;
R¹¹, R¹², R¹³, R¹⁴, R¹⁵, R¹⁶, R¹⁷, R¹⁸, R¹⁹, and R^{1a1} are each independently selected from halogen, amino, hydroxy, cyano, deuterium, and nitro.

In an optional embodiment of the present disclosure, the compound represented by formula (I) is a compound represented by formula (III), 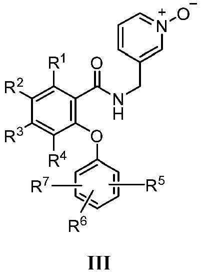 wherein
R¹, R², R³, R⁴, R⁵, R⁶, and R⁷ are each independently selected from H, D, C₁-C₆ alkyl, C₁-C₆ alkyl substituted with one or more R¹¹, C₂-C₆ alkenyl, C₂-C₆ alkenyl substituted with one or more R¹², C₂-C₆ alkynyl, C₂-C₆ alkynyl substituted with one or more R¹³, -O-(C₁-C₆ alkyl), -O-(C₁-C₆ alkyl) substituted with one or more R¹⁴, -S-(C₁-C₆ alkyl), -S-(C₁-C₆ alkyl) substituted with one or more R¹⁵, C₃-C₆ cycloalkyl, C₃-C₆ cycloalkyl substituted with one or more R¹⁶, 4-8 membered heterocycloalkyl, 4-8 membered heterocycloalkyl substituted with one or more R¹⁷, 6-10 membered aryl, 6-10 membered aryl substituted with one or more R¹⁸, 5-8 membered heteroaryl, 5-8 membered heteroaryl substituted with one or more R¹⁹, -NR^{1a}R^{1b}, halogen, hydroxy, cyano, nitro, and -SF₅, and when a plurality of the substituents R¹¹, R¹², R¹³, R¹⁴, R¹⁵, R¹⁶, R¹⁷, R¹⁸, and R¹⁹ are present, the substituents are the same or different; R^{1a} and R^{1b} are each independently selected from H, C₁-C₆ alkyl, and C₁-C₆ alkyl substituted with one or more R^{1a1}, and when a plurality of the substituents R^{1a}, R^{1b}, and R^{1a1} are present, the substituents are the same or different;
R¹¹, R¹², R¹³, R¹⁴, R¹⁵, R¹⁶, R¹⁷, R¹⁸, R¹⁹, and R^{1a1} are each independently selected from halogen, amino, hydroxy, cyano, deuterium, and nitro.

In an optional embodiment of the present disclosure, the compound represented by formula (I) is a compound represented by formula (IV), 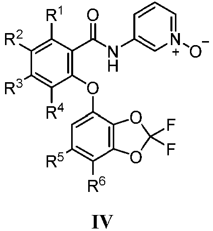 wherein
R¹, R², R³, R⁴, R⁵, and R⁶ are each independently selected from H, D, C₁-C₆ alkyl, C₁-C₆ alkyl substituted with one or more R¹¹, C₂-C₆ alkenyl, C₂-C₆ alkenyl substituted with one or more R¹², C₂-C₆ alkynyl, C₂-C₆ alkynyl substituted with one or more R¹³, -O-(C₁-C₆ alkyl), -O-(C₁-C₆ alkyl) substituted with one or more R¹⁴, -S-(C₁-C₆ alkyl), -S-(C₁-C₆ alkyl) substituted with one or more R¹⁵, C₃-C₆ cycloalkyl, C₃-C₆ cycloalkyl substituted with one or more R¹⁶, 4-8 membered heterocycloalkyl, 4-8 membered heterocycloalkyl substituted with one or more R¹⁷, 6-10 membered aryl, 6-10 membered aryl substituted with one or more R¹⁸, 5-8 membered heteroaryl, 5-8 membered heteroaryl substituted with one or more R¹⁹, -NR^{1a}R^{1b}, halogen, hydroxy, cyano, nitro, and -SF₅, and when a plurality of the substituents R¹¹, R¹², R¹³, R¹⁴, R¹⁵, R¹⁶, R¹⁷, R¹⁸, and R¹⁹ are present, the substituents are the same or different;
R^{1a} and R^{1b} are each independently selected from H, C₁-C₆ alkyl, and C₁-C₆ alkyl substituted with one or more R^{1a1}, and when a plurality of the substituents R^{1a}, R^{1b}, and R^{1a1} are present, the substituents are the same or different;
R¹¹, R¹², R¹³, R¹⁴, R¹⁵, R¹⁶, R¹⁷, R¹⁸, R¹⁹, and R^{1a1} are each independently selected from halogen, amino, hydroxy, cyano, deuterium, and nitro.

In an optional embodiment of the present disclosure, the compound represented by formula (I) is a compound represented by formula (V), 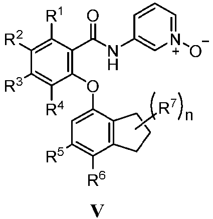 wherein
n is independently selected from 0, 1, 2, 3, 4, 5, and 6;
R¹, R², R³, R⁴, R⁵, R⁶, and R⁷ are each independently selected from H, D, C₁-C₆ alkyl, C₁-C₆ alkyl substituted with one or more R¹¹, C₂-C₆ alkenyl, C₂-C₆ alkenyl substituted with one or more R¹², C₂-C₆ alkynyl, C₂-C₆ alkynyl substituted with one or more R¹³, -O-(C₁-C₆ alkyl), -O-(C₁-C₆ alkyl) substituted with one or more R¹⁴, -S-(C₁-C₆ alkyl), -S-(C₁-C₆ alkyl) substituted with one or more R¹⁵, C₃-C₆ cycloalkyl, C₃-C₆ cycloalkyl substituted with one or more R¹⁶, 4-8 membered heterocycloalkyl, 4-8 membered heterocycloalkyl substituted with one or more R¹⁷, 6-10 membered aryl, 6-10 membered aryl substituted with one or more R¹⁸, 5-8 membered heteroaryl, 5-8 membered heteroaryl substituted with one or more R¹⁹, -NR^{1a}R^{1b}, halogen, hydroxy, cyano, nitro, and -SF₅, and when a plurality of the substituents R¹¹, R¹², R¹³, R¹⁴, R¹⁵, R¹⁶, R¹⁷, R¹⁸, and R¹⁹ are present, the substituents are the same or different; R^{1a} and R^{1b} are each independently selected from H, C₁-C₆ alkyl, and C₁-C₆ alkyl substituted with one or more R^{1a1}, and when a plurality of the substituents R^{1a}, R^{1b}, and R^{1a1} are present, the substituents are the same or different;
R¹¹, R¹², R¹³, R¹⁴, R¹⁵, R¹⁶, R¹⁷, R¹⁸, R¹⁹, and R^{1a1} are each independently selected from halogen, amino, hydroxy, cyano, deuterium, and nitro.

In an optional embodiment of the present disclosure, the compound represented by formula (I) is a compound represented by formula (VI), 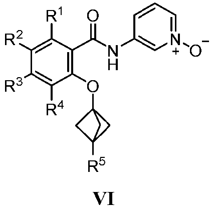 wherein
R¹, R², R³, R⁴, and R⁵ are each independently selected from H, D, C₁-C₆ alkyl, C₁-C₆ alkyl substituted with one or more R¹¹, C₂-C₆ alkenyl, C₂-C₆ alkenyl substituted with one or more R¹², C₂-C₆ alkynyl, C₂-C₆ alkynyl substituted with one or more R¹³, -O-(C₁-C₆ alkyl), -O-(C₁-C₆ alkyl) substituted with one or more R¹⁴, -S-(C₁-C₆ alkyl), -S-(C₁-C₆ alkyl) substituted with one or more R¹⁵, C₃-C₆ cycloalkyl, C₃-C₆ cycloalkyl substituted with one or more R¹⁶, 4-8 membered heterocycloalkyl, 4-8 membered heterocycloalkyl substituted with one or more R¹⁷, 6-10 membered aryl, 6-10 membered aryl substituted with one or more R¹⁸, 5-8 membered heteroaryl, 5-8 membered heteroaryl substituted with one or more R¹⁹, -NR^{1a}R^{1b}, halogen, hydroxy, cyano, nitro, and -SF₅, and when a plurality of the substituents R¹¹, R¹², R¹³, R¹⁴, R¹⁵, R¹⁶, R¹⁷, R¹⁸, and R¹⁹ are present, the substituents are the same or different;
R^{1a} and R^{1b} are each independently selected from H, C₁-C₆ alkyl, and C₁-C₆ alkyl substituted with one or more R^{1a1}, and when a plurality of the substituents R^{1a}, R^{1b}, and R^{1a1} are present, the substituents are the same or different;
R¹¹, R¹², R¹³, R¹⁴, R¹⁵, R¹⁶, R¹⁷, R¹⁸, R¹⁹, and R^{1a1} are each independently selected from halogen, amino, hydroxy, cyano, deuterium, and nitro.

In an optional embodiment of the present disclosure, 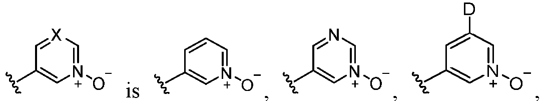 or

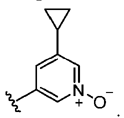

In an optional embodiment of the present disclosure, 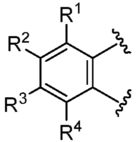 is selected from

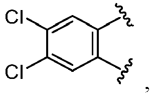

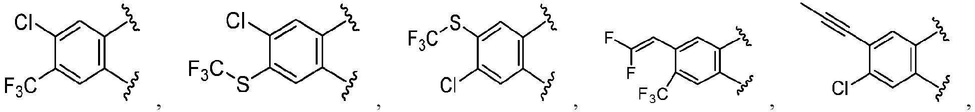

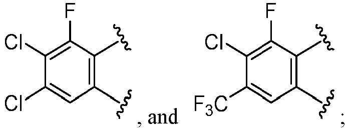

In an optional embodiment of the present disclosure,

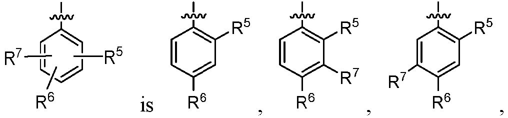

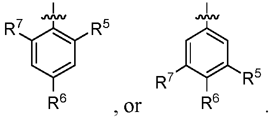

In an optional embodiment of the present disclosure, 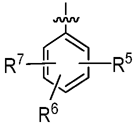 is selected from

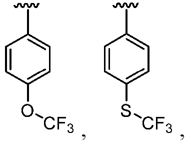

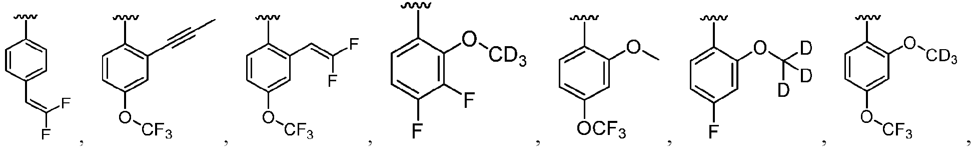

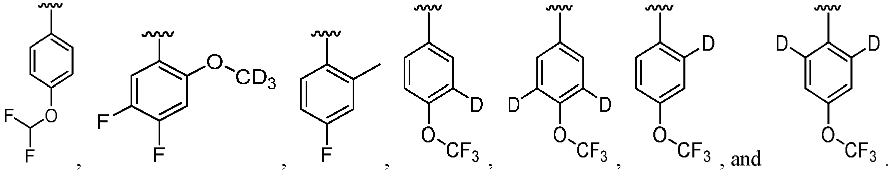

In an optional embodiment of the present disclosure, R¹ is H or halogen.

In an optional embodiment of the present disclosure, R¹ is H or F.

In an optional embodiment of the present disclosure, R² is halogen, C₁-C₆ alkyl substituted with one or more R¹¹, C₂-C₆ alkenyl substituted with one or more R¹², C₂-C₆ alkynyl, or -S-(C₁-C₆ alkyl) substituted with one or more R¹⁵.

In an optional embodiment of the present disclosure, R² is Cl, 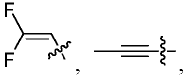 , trifluoromethyl, or

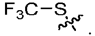

In an optional embodiment of the present disclosure, R³ is H, halogen, -S-(C₁-C₆ alkyl) substituted with one or more R¹⁵, or C₁-C₆ alkyl substituted with one or more R¹¹.

In an optional embodiment of the present disclosure, R³ is H, Cl, trifluoromethyl, or

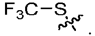

In an optional embodiment of the present disclosure, R⁴ is H.

In an optional embodiment of the present disclosure, R⁵ is H, D, C₁-C₆ alkyl substituted with one or more R¹¹, C₂-C₆ alkenyl substituted with one or more R¹², -O-(C₁-C₆ alkyl) substituted with one or more R¹⁴, or C₂-C₆ alkynyl.

In an optional embodiment of the present disclosure, R⁵ is H, D, methyl, 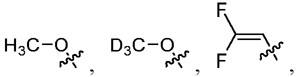 or

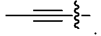

In an optional embodiment of the present disclosure, R⁶ is halogen, C₂-C₆ alkenyl substituted with one or more R¹², -O-(C₁-C₆ alkyl) substituted with one or more R¹⁴, or -S-(C₁-C₆ alkyl) substituted with one or more R¹⁵.

In an optional embodiment of the present disclosure, R⁶ is F, 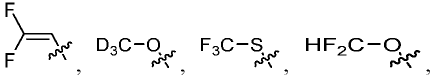 or

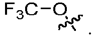

In an optional embodiment of the present disclosure, R⁷ is D or halogen; preferably, R⁷ is D or F.

In an optional embodiment of the present disclosure,

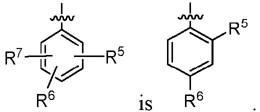

In an optional embodiment of the present disclosure, R¹ is H.

In an optional embodiment of the present disclosure, R² is halogen.

In an optional embodiment of the present disclosure, R² is Cl.

In an optional embodiment of the present disclosure, R³ is halogen.

In an optional embodiment of the present disclosure, R³ is Cl.

In an optional embodiment of the present disclosure, R⁴ is H.

In an optional embodiment of the present disclosure, R³ is H.

In an optional embodiment of the present disclosure, R⁶ is -O-(C₁-C₆ alkyl) substituted with one or more R¹⁴.

In an optional embodiment of the present disclosure, R⁶ is

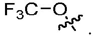

In an optional embodiment of the present disclosure, R¹ is halogen.

In an optional embodiment of the present disclosure, R¹ is F.

In an optional embodiment of the present disclosure, R² is halogen.

In an optional embodiment of the present disclosure, R² is Cl.

In an optional embodiment of the present disclosure, R³ is C₁-C₆ alkyl substituted with one or more R¹¹.

In an optional embodiment of the present disclosure, R³ is trifluoromethyl.

In an optional embodiment of the present disclosure, R⁴ is H.

In an optional embodiment of the present disclosure, R³ is H.

In an optional embodiment of the present disclosure, R⁶ is halogen.

In an optional embodiment of the present disclosure, R⁶ is F.

In an optional embodiment of the present disclosure,

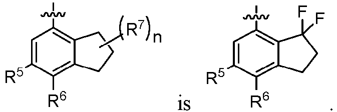

In an optional embodiment of the present disclosure, R¹ is halogen.

In an optional embodiment of the present disclosure, R¹ is F.

In an optional embodiment of the present disclosure, R² is halogen.

In an optional embodiment of the present disclosure, R² is Cl.

In an optional embodiment of the present disclosure, R³ is C₁-C₆ alkyl substituted with one or more R¹¹; preferably, R³ is trifluoromethyl.

In an optional embodiment of the present disclosure, R⁴ is H.

In an optional embodiment of the present disclosure, R⁵ is halogen.

In an optional embodiment of the present disclosure, R⁵ is F.

In an optional embodiment of the present disclosure, R⁶ is halogen.

In an optional embodiment of the present disclosure, R⁶ is F.

In an optional embodiment of the present disclosure, R¹ is H.

In an optional embodiment of the present disclosure, R² is halogen.

In an optional embodiment of the present disclosure, R² is Cl.

In an optional embodiment of the present disclosure, R³ is C₁-C₆ alkyl substituted with one or more R¹¹.

In an optional embodiment of the present disclosure, R³ is trifluoromethyl.

In an optional embodiment of the present disclosure, R⁴ is H.

In an optional embodiment of the present disclosure, R⁵ is -O-(C₁-C₆ alkyl) substituted with one or more R¹⁴.

In an optional embodiment of the present disclosure, R⁵ is

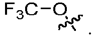

In an optional embodiment of the present disclosure, the compound represented by formula (I) is selected from the following compounds:

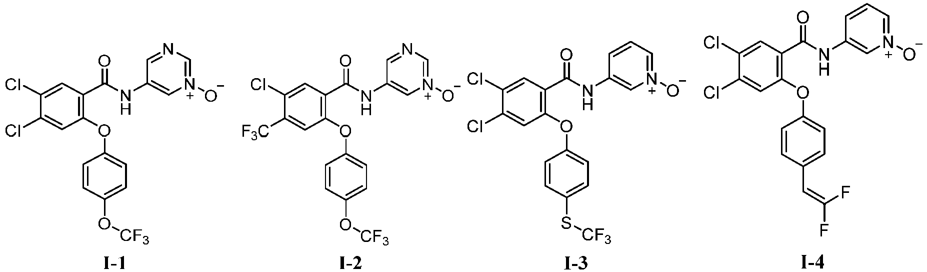

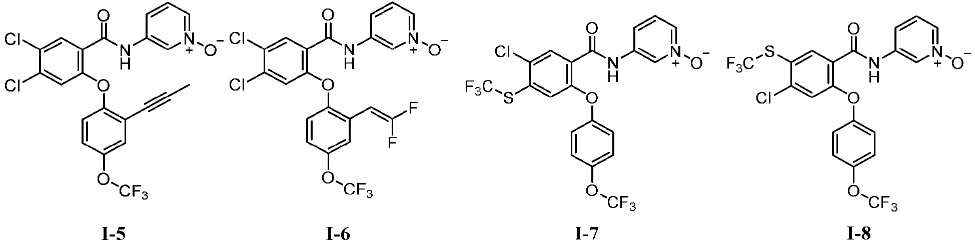

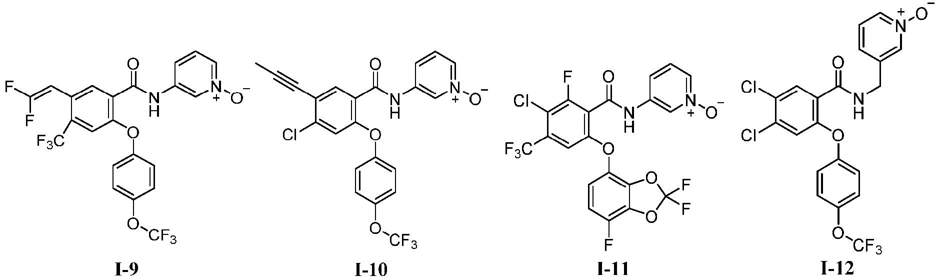

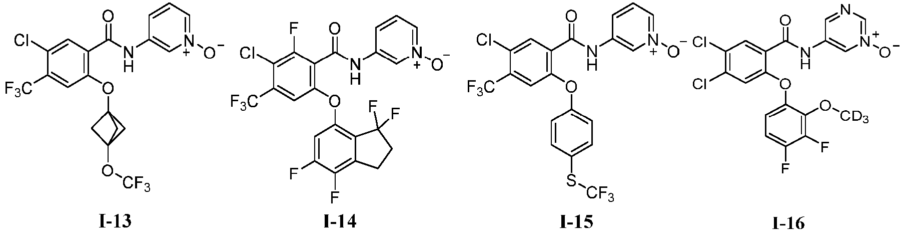

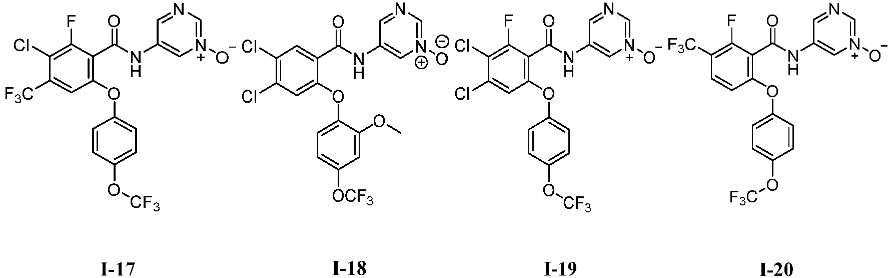

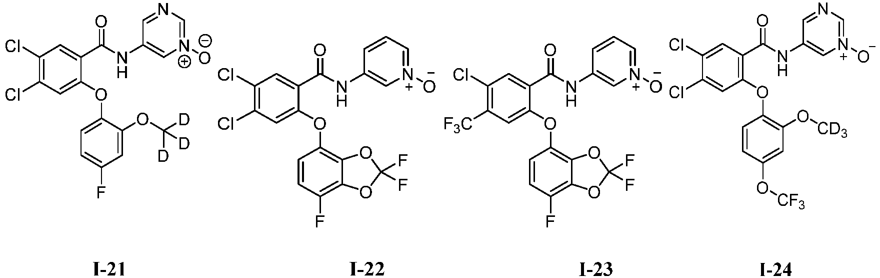

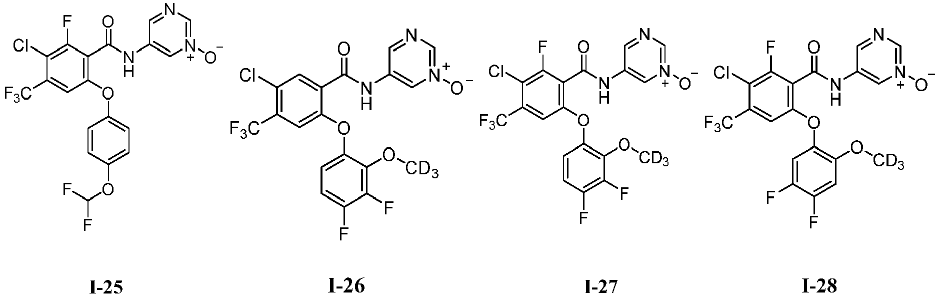

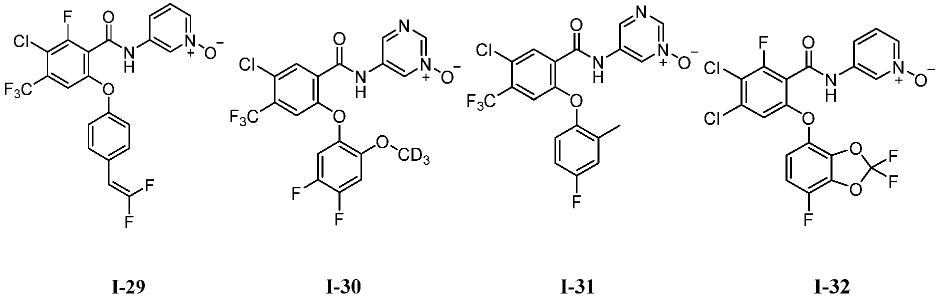

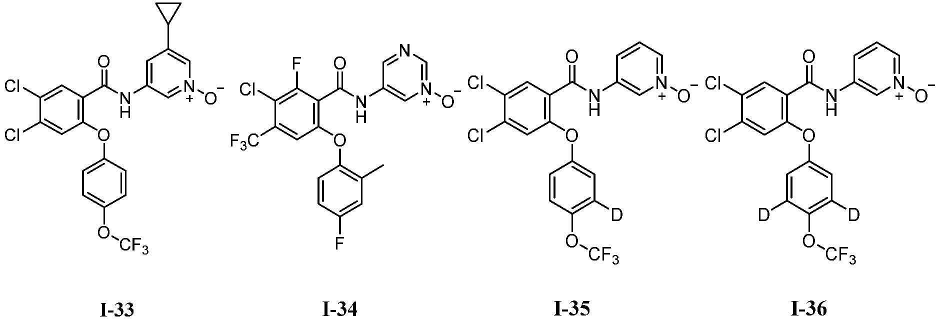

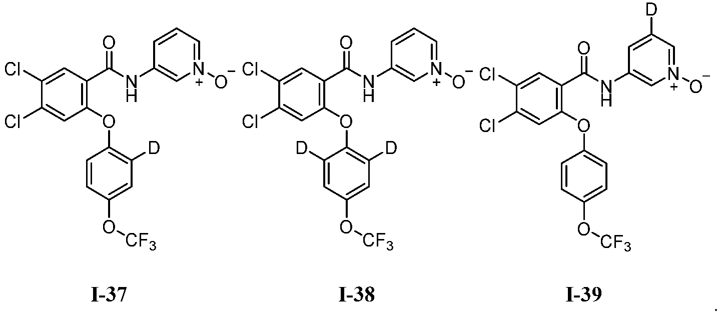

In a second aspect of the present disclosure, the present disclosure provides a pharmaceutical composition, and the pharmaceutical composition comprises a therapeutically effective amount of the compound, or the tautomer, the stereoisomer, the hydrate, the solvate, the pharmaceutically acceptable salt, or the prodrug thereof described above, and a pharmaceutically acceptable carrier, diluent, or excipient.

In a third aspect of the present disclosure, the present disclosure provides use of the compound, or the tautomer, the stereoisomer, the hydrate, the solvate, the pharmaceutically acceptable salt, or the prodrug thereof described above, or the pharmaceutical composition described above for manufacturing a medicament associated with the treatment and inhibition of a voltage-gated sodium channel. The inhibition of a voltage-gated sodium channel includes Nav1.1-Nav1.9, preferably Nav1.8.

According to a specific embodiment of the present disclosure, in the use of the compound, or the tautomer, the stereoisomer, the hydrate, the solvate, the pharmaceutically acceptable salt, or the prodrug thereof described above, or the pharmaceutical composition described above for manufacturing a medicament, the medicament can be used for the treatment, alleviation, or prevention of pain, and the pain comprises acute pain, chronic pain, inflammatory pain, cancer pain, neuropathic pain, musculoskeletal pain, primary pain, intestinal pain, and idiopathic pain.

### Beneficial Effects

According to an embodiment of the present disclosure, the present disclosure has at least one of the following technical effects:
1) A Nav1.8 inhibitor with a novel structure, excellent pharmacokinetic property, and good efficacy or druggability is provided, which can be used for effective treatment of Nav1.8-related diseases and symptoms.
2) The compound of the present disclosure has a relatively strong inhibitory activity on Nav1.8 ion channel.

Additional aspects and advantages of the present disclosure will be set forth in part in the following description and, in part, will be apparent from the following description, or may be learned by practice of the present disclosure.

### Definitions and Description

Unless otherwise indicated, the terms and definitions described in the specification and claims of the present application and used in the present disclosure are included as follows.

It will be understood by those skilled in the art that, according to conventions used in the art, in the structural formulas of the present application, 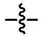 is used to depict a chemical bond that is the point at which a moiety or substituent is linked to a core structure or framework.

The term "pharmaceutically acceptable salt" refers to a pharmaceutically acceptable salt of a non-toxic acid or base, including salts of inorganic acids and bases, and organic acids and bases.

The term "pharmaceutical composition" refers to a mixture of one or more of the compounds described herein or a physiologically/pharmaceutically acceptable salt or prodrug thereof, and other chemical components, for example, physiologically/pharmaceutically acceptable carriers and excipients. The purpose of the pharmaceutical composition is to facilitate the administration of the compound to an organism.

The term "auxiliary material" refers to a pharmaceutically acceptable inert ingredient. Examples of types of the term "excipient" include, without limitation, adhesives, disintegrants, lubricants, glidants, stabilizers, fillers, diluents, and the like. Excipients are capable of enhancing the handling characteristics of the pharmaceutical preparation, i.e., making the preparation more amenable to direct compression by increasing flowability and/or adhesiveness.

The term "prodrug" can be converted to the compound disclosed herein with biological activity under physiological conditions or by solvolysis. The prodrug disclosed herein is prepared by modifying a functional group in the compound, wherein the modification may be removed by conventional procedures or be removed *in vivo* to give the parent compound. The prodrug includes a compound formed by linking a hydroxyl or amino group of the compound disclosed herein to any group. When the prodrug of the compound disclosed herein is administered to a mammalian individual, the prodrug is cleaved to form a free hydroxyl group or a free amino group.

The term "stereoisomer" refers to isomers resulting from different spatial arrangements of atoms in a molecule, including *cis-trans* isomers, enantiomers, diastereoisomers, and conformational isomers.

The term "tautomer" refers to functional isomers resulting from the rapid movement of an atom in a molecule between two positions. The compound disclosed herein may exhibit the tautomerism. A tautomeric compound may be present in two or more interconvertible forms. Prototropic tautomers result from the migration of a covalently bonded hydrogen atom between two atoms. Tautomers are generally present in an equilibrium form. Efforts to separate a single tautomer usually lead to a mixture, the physicochemical properties of which are consistent with those of the mixture of the compound. The position of the equilibrium depends on the chemical properties of the molecule. For example, in many aliphatic aldehydes and ketones such as acetaldehyde, the keto form predominates; whereas in phenols, the enol form predominates. In the present disclosure, all tautomeric forms of the compound are included.

Certain compounds of the present disclosure may have an asymmetric carbon atom (optical center) or double bond. Racemates, diastereoisomers, geometric isomers, and individual isomers are all included within the scope of the present disclosure.

The illustration of the racemic, ambiscalemic and scalemic, or enantiomerically pure compounds herein is from Machr, J. Chem. Ed. 1985, 62:114-120. Unless otherwise stated, the absolute configuration of a stereogenic center is represented by a wedged bond and a dashed bond. When the compounds described herein contain olefinic double bonds or other geometric asymmetric centers, they include E and Z geometric isomers unless otherwise specified. Likewise, all tautomeric forms are included within the scope of the present disclosure.

The compounds of the present disclosure may be in the form of a specific geometric isomer or stereoisomer. All such compounds are contemplated herein, including *cis* and *trans* isomers, (-)- and (+)-enantiomers, (R)- and (S)-enantiomers, diastereoisomers, D-isomers, L-isomers, and racemic mixtures and other mixtures thereof, such as an enantiomer or diastereoisomer enriched mixture, all of which are encompassed within the scope of the present disclosure. Substituents such as alkyl may have an additional asymmetric carbon atom. All these isomers and mixtures thereof are encompassed within the scope of the present disclosure.

Optically active (R)- and (S)-isomers and D- and L- isomers can be prepared by chiral synthesis or chiral reagents or other conventional techniques. If one enantiomer of a certain compound of the present disclosure is to be obtained, the desired pure enantiomer can be prepared by asymmetric synthesis or derivatization using a chiral auxiliary, wherein the resulting diastereoisomeric mixture is separated and the auxiliary group is cleaved. Alternatively, when the molecule contains a basic functional group (such as amino) or an acidic functional group (such as carboxyl), the compound reacts with an appropriate optically active acid or base to form a salt of the diastereoisomer, which is then subjected to resolution of diastereoisomers through fractional crystallization or chromatography known in the art to acquire the pure enantiomer. Furthermore, the enantiomer and the diastereoisomer are generally isolated through chromatography using a chiral stationary phase, optionally in combination with chemical derivatization (e.g., generating carbamate from amines).

The compound disclosed herein may contain an unnatural proportion of atomic isotope at one or more of the atoms that constitute the compound. For example, the compound may be labeled with a radioisotope, such as tritium (³H), iodine-125 (¹²⁵I) or C-14 (¹⁴C). All isotopic variations of the compound disclosed herein, whether radioactive or not, are encompassed within the scope of the present disclosure.

In the context of a drug or pharmacologically active agent, the term "effective amount" or "therapeutically effective amount" refers to an amount of the drug or medicament that is sufficient to achieve the desired effects without producing toxic effects. For oral dosage forms of the present disclosure, an "effective amount" of one active substance in the composition is the amount required to achieve the desired effect when the active substance is combined with another active substance in the composition. The effective amount varies from person to person, depending on the age and general condition of the subject and also on the particular active substance. The suitable effective amount in a case can be determined by those skilled in the art through routine tests.

The term "active ingredient", "therapeutic agent", "active substance", or "active agent" refers to a chemical entity that is effective in treating a target disorder, disease, or condition.

The term "substituted" means that one or more hydrogen atoms on a specific atom are substituted by substituents, including deuterium and hydrogen variants, as long as the valence of the specific atom is normal and the substituted compound is stable. When the substituent is keto (i.e., =O), it means that two hydrogen atoms are substituted. Substitution with keto does not occur on aromatic groups. The term "optionally substituted" means that an atom can be substituted with a substituent or not. Unless otherwise specified, the type and number of the substituent may be arbitrary as long as being chemically achievable.

The term "a plurality of' means two or more, including two, three, four, or more.

The prefix "Cᵤ₋Cᵥ" indicates that the following groups have from u to v carbon atoms. For example, "C₁₋C₆ alkyl" means that the alkyl has 1 to 6 carbon atoms.

The term "C₁-C₆ alkyl" should be understood to represent a linear or branched saturated monovalent hydrocarbyl group having 1, 2, 3, 4, 5, or 6 carbon atoms. The alkyl is, for example, methyl, ethyl, propyl, butyl, pentyl, hexyl, isopropyl, isobutyl, sec-butyl, *tert-butyl,* isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or an isomer thereof. In particular, the group has 1, 2, or 3 carbon atoms ("C₁-C₃ alkyl"), such as methyl, ethyl, *n*-propyl, or isopropyl.

The term "-O-(C₁-C₆ alkyl)" should be understood to mean that an alkyl group is connected with the rest of the molecule through an oxygen atom, wherein "C₁-C₆ alkyl" has the definition described above, e.g., -O-(methyl) and - O-(ethyl).

The term "--S-(C₁-C₆ alkyl)" should be understood to mean that an alkyl group is connected with the rest of the molecule through a sulfur atom, where "C₁-C₆ alkyl" has the definition described above, e.g., -S-(methyl) and -S-(ethyl).

The term "C₂-C₆ alkynyl" refers to a linear or branched unsaturated hydrocarbyl having at least 1 (e.g., 1 to 2, preferably 1) triple bond, and examples include, but are not limited to, C₂-C₆ alkynyl such as ethynyl, 1-propynyl or 2-propynyl, 1-butynyl, 2-butynyl or 3-butynyl, 1-methyl-2-propynyl, and the like.

The term "C₂₋C₆ alkenyl" should be understood to mean a linear or branched monovalent hydrocarbyl comprising one or more double bonds and having 2-6 carbon atoms, for example, having 2 or 3 carbon atoms (i.e., C₂-C₃ alkenyl). It should be understood that in the case that the alkenyl contains more than one double bond, the double bonds can be separated from one another or conjugated. The alkenyl is, for example, ethenyl, allyl, (*E*)-2-methylethenyl, (*Z*)-2-methylethenyl, (*E*)-but-2-enyl, (*Z*)-but-2-enyl, (*E*)-but-1-enyl, (Z)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, or 1-isopropylvinyl.

The term "C₃-C₆ cycloalkyl" should be understood to denote a saturated monovalent monocyclic or bicyclic hydrocarbon ring having 3-6 carbon atoms, including fused or bridged polycyclic ring systems, e.g., cyclopropyl, cyclobutyl, cyclopentyl, or cyclohexyl.

The term "4-8 membered heterocycloalkyl" refers to a monocyclic saturated heterocyclic ring having a total of 4, 5, 6, 7, or 8 ring atoms and containing one or two identical or different ring heteroatoms or heteroatom-containing groups. The ring heteroatoms or heteroatom-containing groups are selected from: N, NH, O, S, SO, and SO₂. The heterocycloalkyl group may be linked to the rest of the molecule through any carbon atom or (if present) a nitrogen atom. The heterocycloalkyl may be a 4-membered ring, such as azetidinyl, oxetanyl, or thietanyl; or a 5-membered ring, such as tetrahydrofuranyl, 1,3-dioxolanyl, thiacyclopentyl, pyrrolidinyl, imidazolidinyl, pyrazolidinyl, 1,1-dioxothiacyclopentyl, 1,2-oxazolidinyl, 1,3-oxazolidinyl, or 1,3-thiazolidinyl; or a 6-membered ring, such as tetrahydropyranyl, tetrahydrothiopyranyl, piperidinyl, morpholinyl, dithianyl, thiomorpholinyl, piperazinyl, 1,3-dioxanyl, 1,4-dioxanyl, or 1,2-oxazacyclohexyl.

The term "6-10 membered aryl" should be understood as an aromatic or partially aromatic monovalent monocyclic, bicyclic or tricyclic hydrocarbon ring having 6-10 carbon atoms, in particular a ring having 6 carbon atoms ("C₆ aryl"), such as phenyl; when the 6-10 membered aryl is substituted, it may be monosubstituted or polysubstituted. In addition, the substitution site is not limited, and may be, for example, ortho-substitution, para-substitution, or meta-substitution.

The term "5-8 membered heteroaryl" should be understood as a monovalent monocyclic, bicyclic or tricyclic aromatic ring group having 5-8 ring atoms, in particular 5 or 6 carbon atoms, and comprising 1-5 heteroatoms independently selected from N, O, and S. A monovalent monocyclic, bicyclic or tricyclic aromatic ring group having 1-3 heteroatoms independently selected from N, O, and S is preferred, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, and the like; or pyridinyl, pyridazinyl, pyrimidinyl, pyrazinyl, triazinyl, and the like; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

The term "halo" or "halogen" is fluorine, chlorine, bromine, and iodine.

The term "deuterium" ("D" and "d") refers to an isotope of hydrogen (H) with one proton and one neutron in the deuterium nucleus, and the natural abundance of the isotope is 0.015%.

The term "one or more" (e.g., in the definition of a substituent of the compound of the general formula of the present disclosure) means "one, two, three, four, or five, particularly one, two, three, or four, more particularly one, two, or three, even more particularly one or two".

The term "optional" or "optionally" means that the subsequently described event or circumstance may or may not occur, and that the description includes instances where the event or circumstance occurs and instances where it does not.

In addition, it should be noted that, unless otherwise explicitly indicated, the description of "... is independently" used herein is to be understood broadly and means that each individual group described is independent from the others and may be independently the same or different specific groups. In more detail, the description of "... is independently" can mean that the specific options expressed by the same symbols in different groups do not affect each other; it can also mean that the specific options expressed by the same symbols in the same group do not affect each other.

### DETAILED DESCRIPTION

The scheme of the present disclosure will be explained with reference to the following examples. Those skilled in the art will appreciate that the following examples are only for illustrating the present disclosure, and should not be construed as limitations to the scope of the present disclosure. Examples without specified techniques or conditions are implemented according to techniques or conditions described in the literature in the art or according to product instructions. Reagents or instruments without specified manufacturers used herein are conventional products that are commercially available.

Unless otherwise specified, the structure of the compound of the present disclosure is determined by nuclear magnetic resonance (NMR) and/or mass spectrometry (MS). NMR shifts are given in 10⁻⁶ (ppm). Solvents for NMR determination are deuterodimethylsulfoxide, deuterochloroform, deuteromethanol or the like, and internal standard is tetramethylsilane (TMS).

The abbreviations of the present disclosure are defined as follows:
M: molar concentration, e.g., 1 M hydrochloric acid for 1 mol/L hydrochloric acid solution
LC-MS: liquid chromatography-mass spectrometry
DMSO: dimethylsulfoxide
HATU: N,N,N',N'-tetramethyl-O-(7-azabenzotriazol-1-yl)urea hexafluorophosphate
DMF: N,N-dimethylformamide
DCM: dichloromethane
m-CPBA: m-chloroperoxybenzoic acid
DIPEA: also designated as DIEA, diisopropylethylamine, i.e., N,N-diisopropylethylamine
IC₅₀: the half maximal inhibitory concentration, referring to the concentration at which half of the maximal inhibitory effect is achieved

### Example 1: Preparation of Target Compound I-1

### 5-(4,5-Dichloro-2-(4-(trifluoromethoxy)phenoxy)benzamido)pyrimidine 1-oxide (target compound I-1)

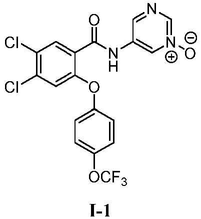

The synthetic route of compound I-1 was as follows:

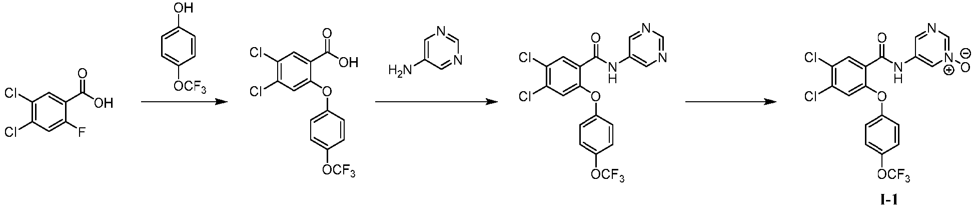

### Step 1: synthesis of 4,5-dichloro-2-(4-(trifluoromethoxy)phenoxy)benzoic acid

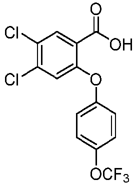

4,5-Dichloro-2-fluorobenzoic acid (1.0 g, 4.78 mmol), cesium carbonate (4.68 g, 14.35 mmol), and 4-(trifluoromethoxy)phenol (8 mL) were added to a 20 mL microwave tube at room temperature. The microwave tube was sealed, and the mixture was reacted at 150 °C for 1 h. The reaction liquid was cooled to room temperature, and water (10 mL) was added to the reaction liquid. The mixture was extracted with EtOAc (20 mL × 3), and the organic phase was washed 1 time with saturated brine (20 mL), dried over anhydrous sodium sulfate, filtered, and concentrated by rotary evaporation under reduced pressure. The crude product was purified by column chromatography (SiO₂; PE:EA = 10:1) to give 4,5-dichloro-2-(4-(trifluoromethoxy)phenoxy)benzoic acid (3) (1.0 g, yield: 56.9%).

### Step 2: synthesis of 4,5-dichloro-N-(pyrimidin-5-yl)-2-(4-(trifluoromethoxy)phenoxy)benzamide

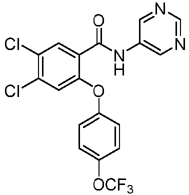

4,5-Dichloro-2-(4-(trifluoromethoxy)phenoxy)benzoic acid (1.0 g, 2.72 mmol), 5-aminopyrimidine (310.88 mg, 3.27 mmol), and HATU (2.07 g, 5.45 mmol) were added to DMF (10 mL), and DIPEA (1.06 g, 8.17 mmol) was then added. After the addition was completed, the reaction liquid was reacted at room temperature for 16 h. The reaction was completed as detected by LC-MS. A NH₄Cl solution (15 mL) was added to the reaction liquid, and the mixture was extracted with EtOAc (20 mL × 3). The organic phases were combined and separately washed with water (20 mL × 2) and saturated brine (10 mL). The organic phase was dried over anhydrous sodium sulfate and filtered. The filtrate was concentrated by rotary evaporation, and the crude product was separated by normal phase silica gel column chromatography (SiO₂; EtOAc/PE = 1:1) to give 4,5-dichloro-N-(pyrimidin-5-yl)-2-(4-(trifluoromethoxy)phenoxy)benzamide (1.10 g; yield: 90.9%).

### Step 3: 5-(4,5-dichloro-2-(4-(trifluoromethoxy)phenoxy)benzamido)pyrimidine 1-oxide (target compound I-1)

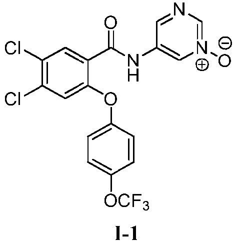

4,5-Dichloro-N-(pyrimidin-5-yl)-2-(4-(trifluoromethoxy)phenoxy)benzamide (1.0 g, 2.09 mmol) was weighed out and placed in DCM (10 mL) at room temperature, and m-CPBA (849.89 mg, 4.19 mmol; 85%) was then slowly added. The reaction liquid was reacted at room temperature for 16 h. After the reaction was completed, the reaction liquid was diluted with dichloromethane (20 mL), and then washed with a saturated aqueous sodium bicarbonate solution (15 mL × 2). The organic phase was dried over Na₂SO₄, concentrated, and sent to preparative high pressure liquid chromatography (aqueous ammonia-acetonitrile) to give 5-(4,5-dichloro-2-(4-(trifluoromethoxy)phenoxy)benzamido)pyrimidine 1-oxide (86.6 mg, yield: 8.38%).
¹H NMR (400 MHz, DMSO): δ 8.85-8.84 (m, 2H), 8.43-8.42 (m, 1H), 8.04 (s, 1H), 7.45 (s, 1H), 7.40-7.37 (m, 2H), 7.20-7.18 (m, 2H).
LC-MS, M/Z (ESI): 458.1 [M-H]⁻

### Example 2: Preparation of Target Compound I-2

### 5-(5-Chloro-2-(4-(trifluoromethoxy)phenoxy)-4-(trifluoromethyl)benzamido )pyrimidine-1-oxide (target compound I-2)

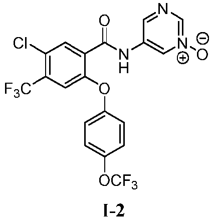

The synthetic route of compound **I-2** was as follows:

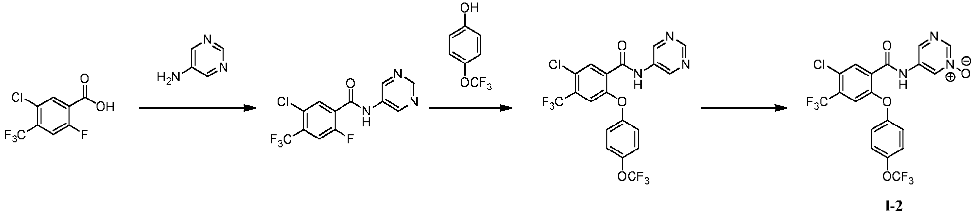

### Step 1: synthesis of 5-chloro-2-fluoro-N-(pyrimidin-5-yl)-4-(trifluoromethyl)benzamide

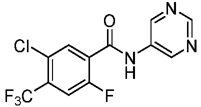

5-Chloro-2-fluoro-4-(trifluoromethyl)benzoic acid (1.60 g, 6.60 mmol) was added to DMF (20 mL) under an ice bath, and HATU (3.76 g, 9.89 mmol) was then added. The mixture was stirred at this condition for 10 min, and 5-aminopyrimidine (752.83 mg, 7.92 mmol) and DIPEA (2.56 g, 19.79 mmol) were then added. After the addition was completed, the reaction liquid was slowly warmed to room temperature and reacted for 16 h. The reaction was completed as detected by LC-MS. An aqueous NH₄Cl solution (30 mL) was added to the reaction liquid, and the mixture was extracted with EtOAc (30 mL × 3). The organic phases were combined and separately washed with water (30 mL × 2) and saturated brine (20 mL). The organic phase was dried over anhydrous sodium sulfate and filtered, the filtrate was concentrated by rotary evaporation, and the crude product was separated by normal phase silica gel preparative plate (EtOAc/PE = 1:2) to give 5-chloro-2-fluoro-N-(pyrimidin-5-yl)-4-(trifluoromethyl)benzamide (0.17 g; yield: 8.06%).

### Step 2: synthesis of 5-chloro-N-(pyrimidin-5-yl)-2-(4-(trifluoromethoxy)phenoxy)-4-(trifluoromethyl)benzamide (5)

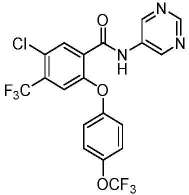

5-Chloro-2-fluoro-N-(pyrimidin-5-yl)-4-(trifluoromethyl)benzamide (170 mg, 0.53 mmol), cesium carbonate (519.85 mg, 1.60 mmol), and 4-(trifluoromethoxy)phenol (3 mL) were added to a 20 mL microwave tube at room temperature. The microwave tube was sealed, and the mixture was reacted at 150 °C for 1 h. The reaction liquid was cooled to room temperature, and water (10 mL) was added to the reaction liquid. The mixture was extracted with EtOAc (20 mL × 3), and the organic phase was washed 1 time with saturated brine (20 mL), dried over anhydrous sodium sulfate, filtered, and concentrated by rotary evaporation under reduced pressure. The crude product was purified by silica gel preparative plate to give 5-chloro-N-(pyrimidin-5-yl)-2-(4-(trifluoromethoxy)phenoxy)-4-(trifluoromethyl)benzamide (0.20 g, yield: 78.7%).

### Step 3: 5-(5-chloro-2-(4-(trifluoromethoxy)phenoxy)-4-(trifluoromethyl)benzamido)pyrimidine-1-oxide (target compound I-2)

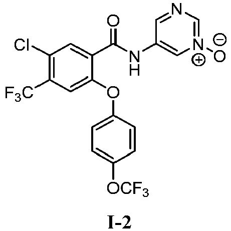

5-Chloro-N-(pyrimidin-5-yl)-2-(4-(trifluoromethoxy)phenoxy)-4-(trifluoromethyl)benzamide (0.20 mg, 0.42 mmol) was weighed out and placed in DCM (3 mL) at room temperature, and m-CPBA (169.98 mg, 0.84 mmol, 85%) was then slowly added. The reaction liquid was reacted at room temperature for 16 h. After the reaction was completed, the reaction liquid was diluted with dichloromethane (10 mL), and then washed with a saturated aqueous sodium bicarbonate solution (10 mL × 2). The organic phase was dried over anhydrous sodium sulfate, concentrated, and sent to preparative high pressure liquid chromatography (aqueous ammonia-acetonitrile) to give 5-(5-chloro-2-(4-(trifluoromethoxy)phenoxy)-4-(trifluoromethyl)benzamido)pyrimidine-1-oxide (10.4 mg, yield: 5.03%).
¹H NMR (400 MHz, DMSO-*d₆*): δ 8.84-8.82 (m, 1H), 8.52 (s, 1H), 8.10 (s, 1H), 7.96 (s, 1H), 7.48 (s, 1H), 7.33-7.31 (m, 2H), 7.09-7.07 (m, 2H).
LC-MS, M/Z (ESI): 492.0 [M-H]⁻

### Example 3: Preparation of Target Compound I-11

### 3-(3-Chloro-2-fluoro-6-((2,2,7-trifluorobenzo[d][1,3]dioxol-4-yl)oxy)-4-trifluoromethyl-benzamido)pyridine 1-oxide (target compound I-11)

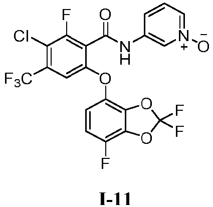

The synthetic route of compound **I-11** was as follows:

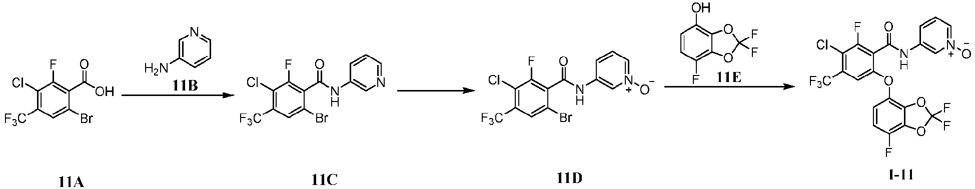

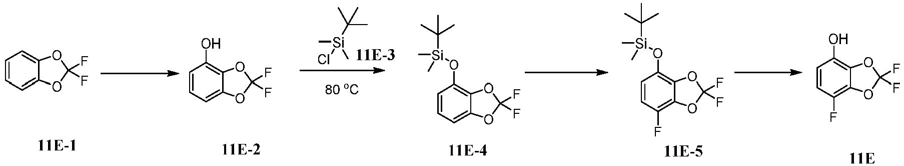

### Step 1: synthesis of 2,2-difluorobenzo[d][1,3]dioxol-4-ol (11E-2)

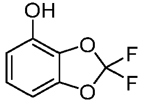

Compound 11E-1 (25.0 g, 8.20 mmol) was dissolved in anhydrous tetrahydrofuran (250 mL), and the system was vacuumized and purged with nitrogen, which was repeated three times. The reaction liquid was cooled to -78 °C under nitrogen atmosphere, sec-butyl lithium (134 mL, 173.94 mmol, 1.3 M) was slowly added, and the reaction temperature was maintained at no more than -70 °C. After the addition was completed, the reaction liquid was successively reacted at this temperature for 2 h. Trimethyl borate (19.72 g, 189.75 mmol) was slowly added dropwise, and the temperature was maintained at no more than -65 °C. After the addition was completed, the reaction liquid was slowly warmed to room temperature and successively reacted for 1 h. Hydrogen peroxide (10.76 g, 316.25 mmol, 30%) and sodium hydroxide (6.32 g, 158.12 mmol) were then separately added. After the addition was completed, the reaction liquid was reacted at room temperature for 16 h. The reaction was completed as detected by TLC (PE:EA = 5:1). Water (100 mL) was added for dilution, and the mixture was extracted with EtOAc (100 mL × 3). The organic phases were combined and washed with 2 N aqueous NaOH solution (50 mL × 2). The aqueous phases were combined, the pH was adjusted to about 3 with 2 N aqueous HCl solution, and the mixture was extracted with EtOAc (100 mL × 3). The organic phases were combined, washed with saturated brine (100 mL), dried over anhydrous sodium sulfate, and filtered. The filtrate was concentrated by rotary evaporation, and the crude product was separated by normal phase silica gel column chromatography (EtOAc/PE = 0%-20%) to give 2,2-difluorobenzo[d][1,3]dioxol-4-ol **(11E-2)** (31.6 g).

### Step 2: tert-butyl ((2,2-difluorobenzo[d][1,3]dioxol-4-yl)oxy)dimethylsilane (11E-4)

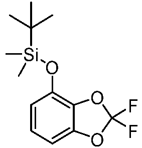

2,2-Difluorobenzo[d][1,3]dioxol-4-ol (31.6 g, 181.50 mmol) was dissolved in anhydrous DMF (300 mL). Imidazole (29.66 g, 435.60 mmol) and compound **11E-3** (32.83 g, 217.80 mmol) were then added. After the addition was completed, the reaction liquid was heated to 80 °C and reacted under the condition for 10 h. The reaction was completed as detected by TLC (PE:EA = 3:1). The reaction liquid was cooled to room temperature, added with water (300 mL) for dilution, and extracted with EtOAc (150 mL × 3). The organic phases were combined, separately washed with water (100 mL × 2) and washed with saturated brine (100 mL), dried over anhydrous sodium sulfate, and filtered. The filtrate was concentrated by rotary evaporation, and the crude product was separated by normal phase silica gel column chromatography (EtOAc/PE = 0%-20%) to give *tert-butyl* ((2,2-difluorobenzo[d][1,3]dioxol-4-yl)oxy)dimethylsilane **(11E-4)** (34.0 g; yield: 64.96%).

### Step 3: tert-butyldimethyl((2,2,7-trifluorobenzo[d][1,3]dioxol-4-yl)oxy)silane (11E-5)

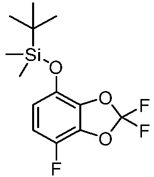

Compound **11E-4** (34.0 g, 117.91 mmol) was dissolved in anhydrous tetrahydrofuran (250 mL), and the system was vacuumized and purged with nitrogen, which was repeated three times. The reaction liquid was cooled to -78 °C under nitrogen atmosphere, *n*-butyl lithium (58.95 mL, 147.38 mmol, 2.5 M) was slowly added, and the reaction temperature was maintained at no more than -70 °C. After the addition was completed, the reaction liquid was successively reacted at this temperature for 2 h. A solution of NFSI (46.47 g, 147.38 mmol) in tetrahydrofuran (100 mL) was slowly added dropwise, and the temperature was maintained at no more than -65 °C. After the addition was completed, the reaction liquid was slowly warmed to room temperature and successively reacted for 10 h. A little starting material remained as detected by LCMS. A saturated aqueous ammonium chloride solution (100 mL) was added to quench the reaction, and the mixture was then extracted with EtOAc (100 mL × 3). The organic phases were combined, washed with saturated brine (100 mL), dried over anhydrous sodium sulfate, and filtered. The filtrate was concentrated by rotary evaporation, and the crude product was separated by normal phase silica gel column chromatography (EtOAc/PE = 0%-20%) to give *tert*-butyldimethyl((2,2,7-trifluorobenzo[d][1,3]dioxol-4-yl)oxy)silane **(11E-5)** (32.20 g; yield: 89.14%).

### Step 4: 2,2,7-trifluorobenzo[d][1,3]dioxol-4-ol (11E)

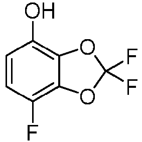

*tert*-Butyldimethyl((2,2,7-trifluorobenzo[d][1,3]dioxol-4-yl)oxy)silane (32.20 g, 105.11 mmol) was weighed out and dissolved in absolute ethanol (300 mL). Potassium hydroxide (8.85 g, 157.66 mmol) was then added. After the addition was completed, the reaction liquid was reacted at room temperature for 10 h. The reaction was completed as detected by TLC (PE:EA = 5:1). The pH was adjusted to about 3 by the addition of 2 N HCl under an ice bath, and the mixture was then extracted with EtOAc (150 mL × 3). The organic phases were combined, washed with saturated brine (100 mL), dried over anhydrous sodium sulfate, and filtered. The filtrate was concentrated by rotary evaporation, and the crude product was separated by reverse phase silica gel column chromatography (HCOOH; ACN/H₂O = 0%-30%) to give 2,2,7-trifluorobenzo[d][1,3]dioxol-4-ol **(11E)** (18.0 g; yield: 89.15%).

### Step 5: 6-bromo-3-chloro-2-fluoro-N-(pyridin-3-yl)-4-(trifluoromethyl)benzamide (11C)

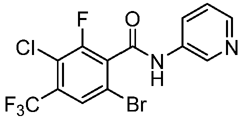

6-Bromo-3-chloro-2-fluoro-4-(trifluoromethyl)benzoic acid **(11A)** (10.0 g, 31.11 mmol) was dissolved in anhydrous DMF (100 mL) under an ice bath, and HATU (27.20 g, 62.22 mmol) was then added. After 10 min, 3-aminopyridine (3.51 g, 37.33 mmol) and DIPEA (12.06 g, 93.33 mmol) were added. After the addition was completed, the mixture was warmed to room temperature and reacted at room temperature for 2 h. The reaction was completed as detected by TLC (PE:EA = 3:1). Water (100 mL) was added to the reaction liquid, and the mixture was extracted with EtOAc (100 mL × 3). The organic phase was separately washed with water (100 mL × 2) and saturated brine (50 mL), dried over Na₂SO₄, filtered, and concentrated by rotary evaporation under reduced pressure. The crude product was separated by normal phase silica gel column chromatography (PE/EtOAc = 0%-50%) to give 6-bromo-3-chloro-2-fluoro-N-(pyridin-3-yl)-4-(trifluoromethyl)benzamide **(11C)** (9.50 g; yield: 76.81%).

### Step 6: 3-(6-bromo-3-chloro-2-fluoro-4-(trifluoromethyl)benzamido)pyridine 1-oxide (11D)

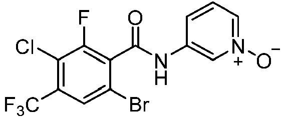

6-Bromo-3-chloro-2-fluoro-N-(pyridin-3-yl)-4-(trifluoromethyl)benzamide **(11C)** (5.0 g, 12.58 mmol) was weighed out and dissolved in DCM (50 mL) under an ice bath, and m-CPBA (6.38 g, 85%, 31.44 mmol) was then added in batches. The reaction liquid was reacted at room temperature for 2 h. The reaction was completed as detected by TLC (EtOAc). The reaction liquid was diluted with dichloromethane (20 mL) and washed with a saturated aqueous sodium bicarbonate solution (30 mL × 2) and saturated brine (30 mL). The organic phase was dried over anhydrous Na₂SO₄, filtered, and concentrated by rotary evaporation under reduced pressure. The crude product was separated by normal phase silica gel column chromatography (DCM/MeOH = 0%-10%) to give 3-(6-bromo-3-chloro-2-fluoro-4-(trifluoromethyl)benzamido)pyridine 1-oxide **(11D)** (2.50 g; yield: 48.07%).

### Step 7: 3-(3-chloro-2-fluoro-6-((2,2,7-trifluorobenzo[d][1,3]dioxol-4-yl)oxy)-4-trifluoromethyl-benzamido)pyridine 1-oxide (target compound I-11)

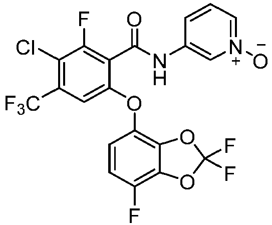

3-(6-Bromo-3-chloro-2-fluoro-4-(trifluoromethyl)benzamido)pyridine 1-oxide (0.50 g, 1.21 mmol) was weighed out and dissolved in toluene (5 mL) under nitrogen atmosphere, and compound **11E** (232.3 mg, 1.21 mmol), cesium carbonate (1.18 g, 3.63 mmol), and copper(I) iodide (46.1 mg, 0.24 mmol) were then sequentially added. After the addition was completed, the reaction liquid was heated to 100 °C and reacted under the condition for 20 min. The reaction was completed as detected by LCMS. The reaction liquid was cooled to room temperature, and a NH₄Cl solution (20 mL) was added to the reaction liquid. The mixture was extracted with EtOAc (30 mL × 3). The organic phase was washed with saturated brine (20 mL) 1 time, dried over Na₂SO₄, filtered, and concentrated by rotary evaporation under reduced pressure. The crude product was purified and separated by preparative reverse phase high pressure liquid chromatography (NH₃H₂O) to give 3-(3-chloro-2-fluoro-6-((2,2,7-trifluorobenzo[d][1,3]dioxol-4-yl)oxy)-4-trifluoromethyl-benzamido)pyridine 1-oxide (target compound **I-11**) (279 mg, yield: 43.9%).
¹H NMR (400 MHz, DMSO_*d₆*):δ 11.38 (s, 1H), 8.61 (s, 1H), 8.06 (d, 1H, J=4.0 Hz), 7.62 (s, 1H), 7.46-7.39 (m, 2H), 7.31-7.26 (m, 1H), 7.13-7.10 (m, 1H).
LC-MS, M/Z (ESI): 523.0 [M-H]⁻

### Example 4: Preparation of Target Compound I-16

### 5-(4,5-Dichloro-2-(3,4-difluoro-2-(deuteromethoxy)phenoxy)benzamido)pyrimidine 1-oxide (target compound I-16)

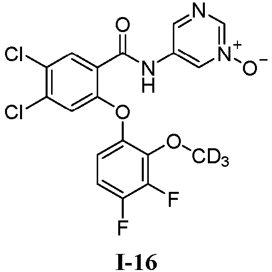

The synthetic route of compound **I-16** was as follows:

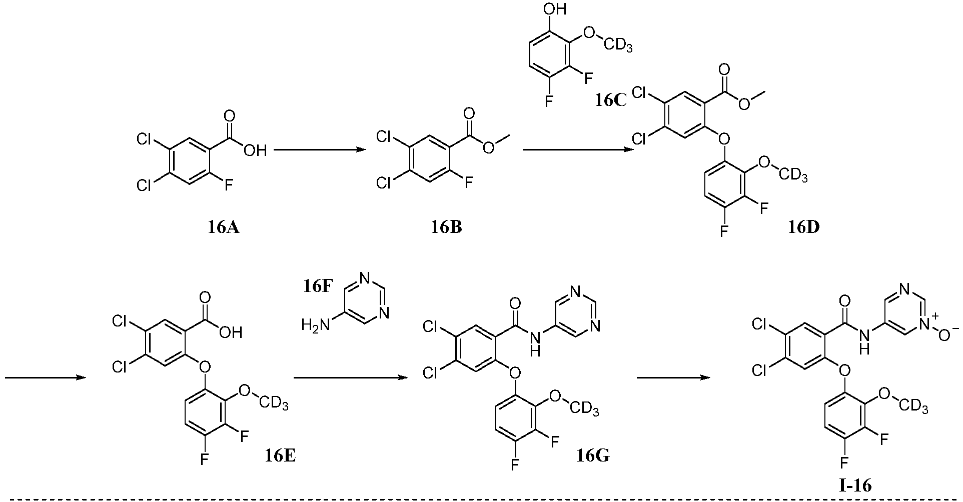

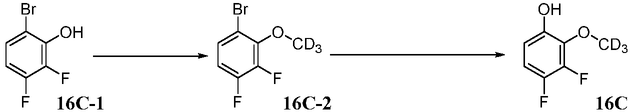

### Step 1: synthesis of 1-bromo-3,4-difluoro-2-deuteromethoxybenzene (16C-2)

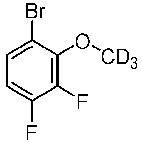

Intermediate **16C-1** (1.0 g, 4.8 mmol), potassium carbonate (0.99 g, 7.2 mmol), and deuteroiodomethane (0.69 g, 4.8 mmol) were added to anhydrous acetonitrile (25 mL) at room temperature, heated to 120 °C, and reacted overnight. After the reaction liquid was cooled to room temperature, water (20 mL) was added to the reaction liquid. The mixture was extracted with EtOAc (20 mL × 3), and the organic phase was washed 1 time with saturated brine (20 mL), dried over Na₂SO₄, filtered, and concentrated by rotary evaporation under reduced pressure. The crude product was purified by silica gel column chromatography (EtOAc/PE = 1:10) to give 1-bromo-3,4-difluoro-2-deuteromethoxybenzene (**16C-2**) (0.95 g; yield: 87.9%) as a yellow liquid.

### Step 2: synthesis of 3,4-difluoro-2-deuteromethoxyphenol (16C)

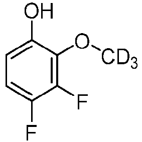

Compound **16C-2** (0.95 g, 4.2 mmol) and triisopropylborate (0.95 g, 5.0 mmol) were added to THF (10 mL), and the air in the reaction flask was purged with nitrogen three times. After the reaction system was cooled to -78 °C, n-BuLi (2 mL, 2.5 M, 5.0 mmol) was slowly added dropwise to the reaction flask. The reaction was then slowly warmed to room temperature and stirred overnight.

After the reaction system was cooled to 0 °C, MeOH (20 mL) and H₂O₂ (30 wt% 10 mL) were added to the reaction liquid, and an aqueous sodium hydroxide solution (10%, 40 mL) was then added dropwise. The reaction was stirred at room temperature for 1 h. After the reaction was completed, saturated sodium thiosulfate (20 mL) was added dropwise to quench the reaction, and the mixture was extracted with EtOAc (30 mL × 3). The organic phases were combined and separately washed with water (20 mL × 2) and saturated brine (20 mL). The organic phase was dried over anhydrous sodium sulfate and filtered. The filtrate was concentrated by rotary evaporation, and the crude product was separated by normal phase silica gel column chromatography (EtOAc/PE = 1:4) to give 3,4-difluoro-2-deuteromethoxyphenol (16C) (360 mg; yield: 52%) as a light yellow liquid.

¹H NMR (400 MHz, DMSO-d₆): δ 8.89 (s, 1H), 6.76-6.72 (m, 1H), 6.59-6.54 (m, 1H).

### Step 3: synthesis of methyl 4,5-dichloro-2-fluorobenzoate (16B)

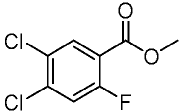

4,5-Dichloro-2-fluorobenzoic acid (1.0 g, 4.7 mmol) was added to MeOH (20 mL). Concentrated hydrochloric acid (5 drops) was then added dropwise to the reaction liquid, and the reaction liquid was reacted at 50 °C for 6 h. The reaction was completed as detected by LC-MS. The reaction liquid was concentrated by rotary evaporation. A NH₄Cl solution (10 mL) was added, and the mixture was extracted with EtOAc (20 mL × 3). The organic phases were combined and separately washed with water (20 mL × 2) and saturated brine (10 mL). The organic phase was dried over anhydrous sodium sulfate and filtered. The filtrate was concentrated by rotary evaporation, and the crude product was separated by normal phase silica gel column chromatography (EtOAc/PE = 1:20) to give methyl 4,5-dichloro-2-fluorobenzoate **(16B)** (1.1 g; yield: 95.4%) as a white solid.

### Step 4: synthesis of methyl 4,5-dichloro-2-(3,4-difluoro-2-(deuteromethoxy)phenoxy)benzoate (16D)

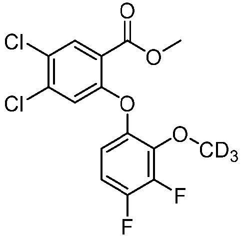

Intermediate **16C** (0.16 g, 1 mmol), methyl 4,5-dichloro-2-fluorobenzoate (0.22 g, 1 mmol), cesium carbonate (0.65 g, 2 mmol), and acetonitrile (5 mL) were added to a 25 mL round-bottom flask at room temperature and reacted at 80 °C for 5 h. The reaction liquid was cooled to room temperature, and a NH₄Cl solution (20 mL) was added to the reaction liquid. The mixture was extracted with EtOAc (20 mL × 3), and the organic phase was washed 1 time with saturated brine (20 mL), dried over Na₂SO₄, filtered, and concentrated by rotary evaporation under reduced pressure. The crude product was purified by silica gel column chromatography (EtOAc/PE = 1:10) to give methyl 4,5-dichloro-2-(3,4-difluoro-2-(deuteromethoxy)phenoxy)benzoate **(16D)** (340.0 mg; yield: 90.3%) as a light yellow solid.

### Step 5: synthesis of 4,5-dichloro-2-(3,4-difluoro-2-(deuteromethoxy)phenoxy)benzoic acid (16E)

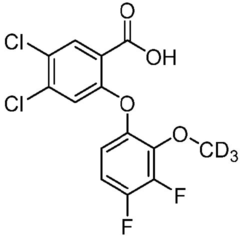

Intermediate **16D** (0.34 g, 0.95 mmol), lithium hydroxide monohydrate (0.07 g, 1.5 mmol), and methanol (10 mL) were added to a 25 mL round-bottom flask at room temperature and reacted for 3 h. A diluted hydrochloric acid solution (20 mL) was added to the reaction liquid. The mixture was extracted with EtOAc (20 mL × 3), and the organic phase was washed 1 time with saturated brine (20 mL), dried over Na₂SO₄, filtered, and concentrated by rotary evaporation under reduced pressure. The crude product was purified by silica gel column chromatography (MeOH/DCM = 1:10) to give 4,5-dichloro-2-(3,4-difluoro-2-(deuteromethoxy)phenoxy)benzoic acid (16E) (311.0 mg; yield: 95.3%) as a white solid.

LC-MS:m/z 352.02[M+H]⁺.

### Step 6: synthesis of 4,5-dichloro-2-(3,4-difluoro-2-(deuteromethoxy)phenoxy)-N-(pyrimidin-5-yl)benzamide (16G)

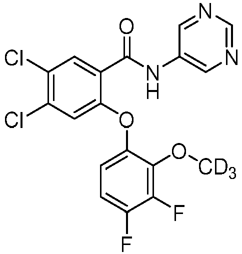

Compound **16F** (0.086 g, 0.9 mmol), intermediate **16E** (0.31 g, 0.89 mmol), DIEA (3.48 g, 2.7 mmol), and HATU (0.51 g, 1.35 mmol) were added to MeCN (10 mL) under an ice bath. The reaction liquid was reacted at room temperature overnight. The reaction was completed as detected by LC-MS. Water (20 mL) was added to the reaction liquid, and the mixture was extracted with EtOAc (30 mL × 3). The organic phases were combined and separately washed with water (20 mL × 2) and saturated brine (20 mL). The organic phase was dried over anhydrous sodium sulfate and filtered. The filtrate was concentrated by rotary evaporation, and the crude product was separated by normal phase silica gel column chromatography (EtOAc/PE = 1:1) to give 4,5-dichloro-2-(3,4-difluoro-2-(deuteromethoxy)phenoxy)-N-(pyrimidin-5-yl)benzamide **(16G)** (305 mg; yield: 81.3%) as a light yellow solid.

LC-MS:m/z 429.04[M+H]⁺.

### Step 7: synthesis of 5-(4,5-dichloro-2-(3,4-difluoro-2-(deuteromethoxy)phenoxy)benzatnido)pyrimidine 1-oxide (target compound 1-16)

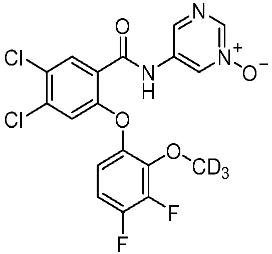

Intermediate **16G** (100 mg, 0.22 mmol) and m-CPBA (75.7 mg, 0.44 mmol) were separately weighed out at room temperature and added to DCM (10 mL). The reaction liquid was reacted at room temperature overnight. After the reaction was completed, the reaction liquid was diluted with dichloromethane (20 mL), and then washed with a saturated aqueous sodium bicarbonate solution (10 mL × 2). The organic phase was dried over Na₂SO₄, concentrated, and sent to preparative high performance liquid chromatography to give 5-(4,5-dichloro-2-(3,4-difluoro-2-(deuteromethoxy)phenoxy)benzamido)pyrimidine 1-oxide (target compound **I-16**) (31.1 mg, yield: 28.9%).
¹H NMR (400 MHz, CDCl₃): δ 9.64 (s, 1H), 9.22 (t, J = 1.8 Hz, 1H), 8.76 (d, J = 1.6 Hz, 1H), 8.32 (d, J = 2.1 Hz, 1H), 8.28 (s, 1H), 7.02 (dd, J = 7.7, 4.9 Hz, 2H), 6.91 (s, 1H).
LC-MS, M/Z (ESI): 445.04 [M+H]⁺

### Example 5: Preparation of Target Compound I-17

### Synthesis of 5-(3-chloro-2-fluoro-6-(4-(trifluoromethoxy)phenoxy)-4-(trifluoromethyl)benzamido)pyrimidine 1-oxide (I-17)

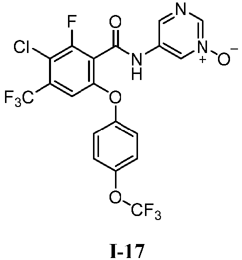

The synthetic route of the target compound I was as follows:

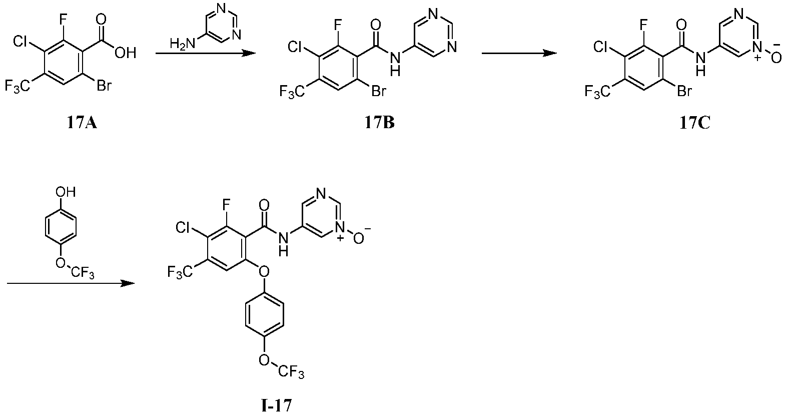

### Step 1: synthesis of 6-bromo-3-chloro-2-fluoro-N-(pyrimidin-5-yl)-4-(trifluoromethyl)benzamide (17B)

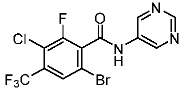

6-Bromo-3-chloro-2-fluoro-4-(trifluoromethyl)benzoic acid (2.00 g, 3.22 mmol) and 3-aminopyrimidine (710 mg, 7.46 mmol) were dissolved in N,N-dimethylamide (20.0 mL) at room temperature, and N,N-diisopropylethylamine (1.61 g, 12.4 mmol) and O-(7-azabenzotriazol-1-yl)-N,N,N,N-tetratnethyluronium hexafluorophosphate (HATU, 3.08 g, 8.08 mmol) were then added. The reaction liquid was stirred at 25 °C for 12 h. After the reaction was completed, the mixture was added into water (40.0 mL) to quench the reaction, and extracted with ethyl acetate (20.0 mL × 3). The organic phase was washed with a saturated sodium chloride solution (40.0 mL), dried over anhydrous sodium sulfate, filtered, and concentrated to give a crude product. The crude product was obtained by direct concentration, and was separated by column chromatography (petroleum ether:ethyl acetate 5:1 to 1:1) to give 6-bromo-3-chloro-2-fluoro-N-(pyrimidin-5-yl)-4-(trifluoromethyl)benzamide **(17B)** (1.70 g, yield: 68.5%) as a yellow solid.
LC-MS, M/Z (ESI): 399.9 [M+H]⁺

### Step 2: synthesis of 5-(6-bromo-3-chloro-2-fluoro-4-(trifluoromethyl)benzamido)pyrimidine 1-oxide (17C)

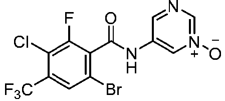

6-Bromo-3-chloro-2-fluoro-N-(pyrimidin-5-yl)-4-(trifluoromethyl)benzamide **(17B)** (1.20 g, 3.01 mmol) was dissolved in dichloromethane (24.0 mL) at 0 °C, and m-chloroperoxybenzoic acid (1.83 g, 9.03 mmol) was added in batches. The reaction liquid was stirred at 25 °C for 12 h. After the reaction was completed, the mixture was added into a saturated sodium sulfite solution (50.0 mL) to quench the reaction, and extracted with dichloromethane (30.0 mL × 3). The organic phase was washed with a saturated sodium chloride solution (50.0 mL), dried over anhydrous sodium sulfate, filtered, and concentrated to give a crude product. The crude product was obtained by direct concentration, and was purified by column chromatography (petroleum ether:ethyl acetate 5:1 to 0:1) to give 5-(6-bromo-3-chloro-2-fluoro-4-(trifluoromethyl)benzamido)pyrimidine 1-oxide **(17C)** (500 mg, yield: 40%) as a white solid.
LC-MS, M/Z (ESI): 415.9 [M+H]⁺
¹H NMR (400 MHz, CDCl₃-*d*) δ 9.66 (br s, 1H), 9.15 (br s, 1H), 8.87 (s, 1H), 8.70 (s, 1H), 7.79 (s, 1H).

### Step 3: synthesis of 5-(3-chloro-2-fluoro-6-(4-(trifluoromethoxy)phenoxy)-4-(trifluoromethyl)benzamido)pyrimidine 1-oxide (I-17)

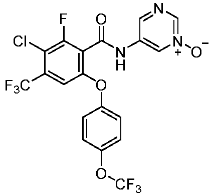

5-(6-Bromo-3-chloro-2-fluoro-4-(trifluoromethyl)benzamido)pyrimidine 1-oxide **(17C)** (400 mg, 965 µmol) and 4-trifluoromethoxyphenol (258 mg, 1.45 mmol) were dissolved in toluene (8.00 mL) at room temperature, and copper(I) iodide (36.7 mg, 193 µmol) and cesium carbonate (629 mg, 1.93 mmol) were then added. The reaction liquid was stirred at 90 °C for 1 h under nitrogen atmosphere. After the reaction was completed, water (20.0 mL) was added to the reaction liquid, and the mixture was extracted with ethyl acetate (10.0 mL × 3). The organic phase was washed with a saturated sodium chloride solution (20.0 mL) and concentrated to give a crude product. The crude product was subjected to preparative reverse phase chromatography (YMC-Actus Triart C18 150 × 30 mm × 7 µm; mobile phase: [water (0.225% formic acid)-acetonitrile]; B%: 53%-83%, 10 min) to give 5-(3-chloro-2-fluoro-6-(4-(trifluoromethoxy)phenoxy)-4-(trifluoromethyl)benzamido)pyrimidine 1-oxide **(I-17,** 95.4 mg, yield: 18.5%).
¹H NMR (400 MHz, DMSO-d6) δ 11.57 (br s, 1H), 8.89 (d, 1H), 8.82 (t, 1H), 8.37 (d, 1H), 7.40 - 7.46 (m, 3H), 7.26 - 7.31 (m, 2H).
LC-MS, M/Z (ESI): 512.1 [M+H]⁺

### Example 6: Preparation of Target Compound I-18

### Synthesis of 5-(4,5-dichloro-2-(2-methoxy-4-(trifluoromethoxy)phenoxy)benzamido)pyrimidine 1-oxide (I-18)

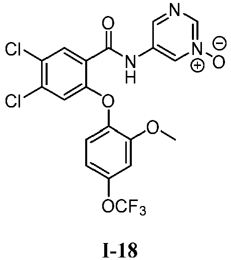

The synthetic route of the target compound **I-18** was as follows:

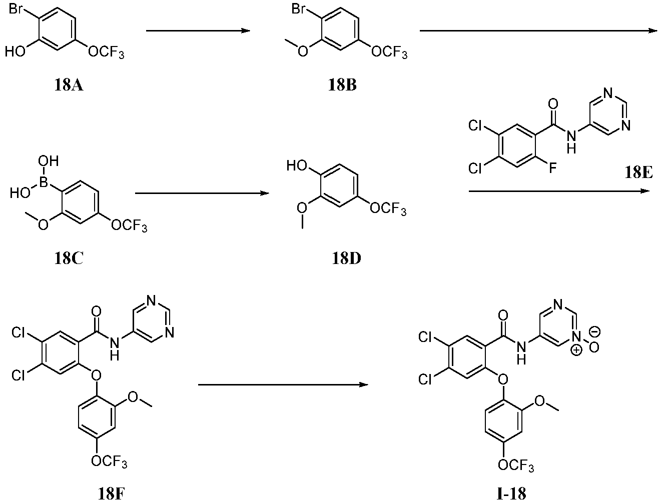

### Step 1: 1-bromo-2-methoxy-4-(trifluoromethoxy)benzene

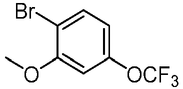

The starting materials 2-bromo-5-(trifluoromethoxy)phenol (5.0 g, 19.45 mmol), iodomethane (4.14 g, 29.18 mmol), and potassium carbonate (8.07 g, 58.36 mmol) were dissolved in DMF (60 mL) and reacted at room temperature for 2 h. After the reaction was completed, water (50 mL) and ethyl acetate (100 mL) were added for liquid separation. The organic phases were combined, added with brine (30 mL × 3) for washing, dried over anhydrous sodium sulfate, filtered, and concentrated to give a crude product as a yellow oily substance (18B, 5.0 g, yield: 94.8%).

¹H NMR (400 MHz, cdcl3) δ 7.56 - 7.52 (m, 1H), 6.77 - 6.72 (m, 2H), 3.90 (s, 3H).

### Step 2: (2-methoxy-4-(trifluoromethoxy)phenyl)boronic acid

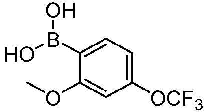

The starting material 1-bromo-2-methoxy-4-(trifluoromethoxy)benzene (2.0 g, 7.38 mmol) was dissolved in tetrahydrofuran (20 mL), and a solution (2.5 M, 3.5 mL, 8.86 mmol) of n-butyl lithium in tetrahydrofuran was slowly added dropwise to the solution at -78 °C under nitrogen atmosphere. After the dropwise addition was completed, the mixture was successively stirred at the temperature for 30 min. Triisopropylborate (1.94 g, 10.33 mmol) was added dropwise to the reaction liquid described above at -78 °C and successively stirred for 2 h. After the reaction was completed, the pH was adjusted to 4-5 with 1 N hydrochloric acid (10 mL). Ethyl acetate (20 mL) was added for liquid separation. The aqueous phase was extracted twice with ethyl acetate, and the organic phases were combined, dried over anhydrous sodium sulfate, filtered, and concentrated under reduced pressure to give a crude product (18C, 1.7 g, yield: 97.6%).

### Step 3: 2-methoxy-4-(trifluoromethoxy)phenol

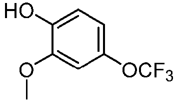

The starting material (2-methoxy-4-(trifluoromethoxy)phenyl)boronic acid (1.0 g, 4.24 mmol) was dissolved in ethanol (10 mL). Hydrogen peroxide (2.5 mL) was slowly added dropwise, and the mixture was reacted at room temperature for 2 h. After the reaction was completed, water (10 mL) and ethyl acetate (30 mL) were added for liquid separation. The organic phase was dried over anhydrous sodium sulfate, filtered, and concentrated under reduced pressure to give a crude product, which was separated and purified by column chromatography (petroleum ether:ethyl acetate (V/V) = 5:1) to give the title compound (18D, 800 mg, yield: 90%).

¹H NMR (400 MHz, cdcl3) δ 6.91 - 6.87 (m, 1H), 6.78 - 6.73 (m, 2H), 3.90 (s, 3H).

### Step 4: 4,5-dichloro-2-(2-methoxy-4-(trifluoromethoxy)phenoxy)-N-(pyrimidin-5-yl)benzamide

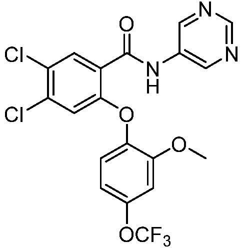

The starting materials 4,5-dichloro-2-fluoro-N-(pyrimidin-5-yl)benzamide (800 mg, 2.8 mmol), 2-methoxy-4-(trifluoromethoxy)phenol (465 mg, 2.24 mmol), and cesium carbonate (2.73 g, 8.39 mmol) were dissolved in DMF (10 mL), and the reaction liquid was reacted at 100 °C for 2 h. After the reaction was completed, water (10 mL) and ethyl acetate (30 mL) were added for liquid separation. The organic phase was dried over anhydrous sodium sulfate, filtered, and concentrated under reduced pressure to give a crude product, which was separated and purified by column chromatography (petroleum ether:ethyl acetate (V/V) = 5:1) to give the title compound (18F, 200 mg, yield: 15%).

LC-MS, M/Z (ESI): 473.98 [M+H]⁺.

### Step 5: 5-(4,5-dichloro-2-(2-methoxy-4-(trifluoromethoxy)phenoxy)benzamido)pyrimidine 1-oxide (I-18)

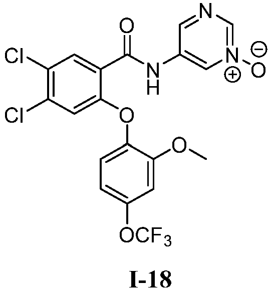

The starting materials 4,5-dichloro-2-(2-methoxy-4-(trifluoromethoxy)phenoxy)-N-(pyrimidin-5-yl)benzamide (100 mg, 0.210 mmol) and m-CPBA (100 mg, 0.63 mmol) were dissolved in dichloromethane (2 mL) and reacted at room temperature for 10 h. After the reaction was completed, an aqueous sodium bicarbonate solution was added for washing, and dichloromethane (10 mL) was added for liquid separation. The organic phases were combined, dried over anhydrous sodium sulfate, and concentrated to give a crude product. The crude product was then purified by preparative reverse phase high performance liquid chromatography (column: SunFileTM Prep C18 OBDTM 5 µm 30 mm × 150 mm); mobile phase: A = acetonitrile, B = water; gradient: 1%-35%, 10 min) to give the title compound 5-(4,5-dichloro-2-(2-methoxy-4-(trifluoromethoxy)phenoxy)benzamido)pyrimidine 1-oxide (I-18, 30 mg, yield: 29%).

LC-MS, M/Z (ESI): 490.0 [M+H]⁺.

¹H NMR (400 MHz, cdcl3) δ 9.73 (s, 1H), 9.21 (t, 1H), 8.77 (d, 1H), 8.31 (d, 1H), 8.30 (s, 1H), 7.28 (d, 1H), 7.01 - 6.97 (m, 2H), 6.88 (s, 1H), 3.87 (s, 3H).

### Example 7: Preparation of Target Compound I-19

### 5-(3,4-Dichloro-2-fluoro-6-(4-(trifluoromethoxy)phenoxy)benzamido)pyrimidine 1-oxide (I-19)

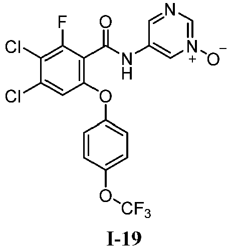

The synthetic route of the target compound **I-19** was as follows:

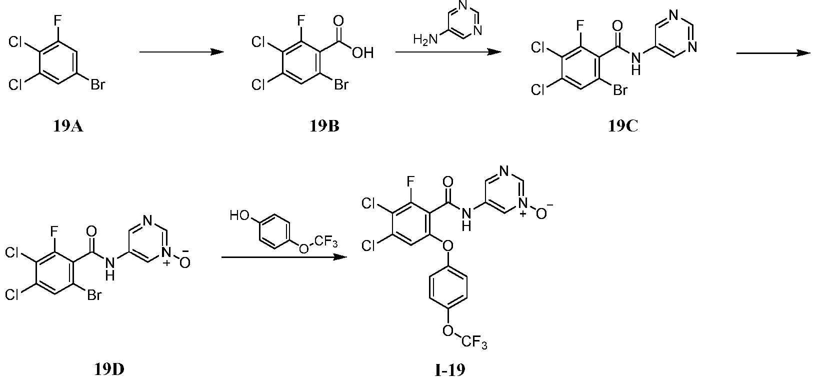

### Step 1: synthesis of 6-bromo-3,4-dichloro-2-fluorobenzoic acid (19B)

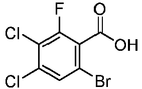

5-Bromo-1,2-dichloro-3-fluorobenzene (19A) (10.0 g, 41.0 mmol) was dissolved in tetrahydrofuran (100 mL) under nitrogen atmosphere, and the reaction liquid was then cooled to -70 °C. A 2 M solution (30.7 mL, 61.5 mmol) of lithium diisopropylamide in tetrahydrofuran was slowly added dropwise, and the reaction liquid was stirred at -70 °C for 0.5 h. Dry ice (20.0 g) was then added to the reaction liquid in batches, and the reaction liquid was successively stirred at 25 °C for 0.5 h. After the reaction was completed, 1 M hydrochloric acid was added to adjust the pH to 3-4, and the mixture was extracted with ethyl acetate. The organic phase was concentrated to give a crude product. The crude product was slurried with petroleum ether (50.0 mL), filtered, and dried with an oil pump to give 6-bromo-3,4-dichloro-2-fluorobenzoic acid **(19B)** (10.0 g, yield: 84.7%) as a brown solid.
LC-MS, M/Z (ESI): 301.0 [M-H]⁻
¹H NMR (400 MHz, DMSO-d6) δ 8.01 (d, 1H).

### Step 2: synthesis of 6-bromo-3,4-dichloro-2-fluoro-N-(pyrimidin-5-yl)benzamide (19C)

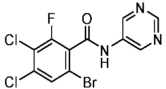

6-Bromo-3,4-dichloro-2-fluorobenzoic acid (3.00 g, 10.4 mmol) and 3-aminopyrimidine (1.19 g, 12.5 mmol) were dissolved in N,N-dimethylamide (30.0 mL) at room temperature, and N,N-diisopropylethylamine (2.69 g, 20.8 mmol) and O-(7-azabenzotriazol-1-yl)-N,N,N,N-tetratnethyluronium hexafluorophosphate (HATU, 5.15 g, 13.5 mmol) were then added. The reaction liquid was stirred at 25 °C for 12 h. After the reaction was completed, the mixture was added into water (50.0 mL) to quench the reaction, and extracted with ethyl acetate (30.0 mL × 3). The organic phase was washed with a saturated sodium chloride solution (50.0 mL), dried over anhydrous sodium sulfate, filtered, and concentrated to give a crude product. The crude product was obtained by direct concentration, and was separated by column chromatography (petroleum ether:ethyl acetate 5:1 to 1:1) to give 6-bromo-3,4-dichloro-2-fluoro-N-(pyrimidin-5-yl)benzamide **(19C)** (2.60 g, yield: 68.4%) as a yellow solid.
LC-MS, M/Z (ESI): 365.9 [M+H]⁺

### Step 3: synthesis of 5-(6-bromo-3,4-dichloro-2-fluorobenzamido)pyrimidine 1-oxide (19D)

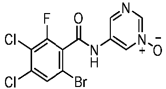

6-Bromo-3,4-dichloro-2-fluoro-N-(pyrimidin-5-yl)benzamide (2.40 g, 6.58 mmol) was dissolved in dichloromethane (48.0 mL) at 0 °C, and m-chloroperoxybenzoic acid (2.67 g, 13.2 mmol) was added in batches. The reaction liquid was stirred at 25 °C for 12 h. After the reaction was completed, the mixture was added into a saturated sodium sulfite solution (50.0 mL) to quench the reaction, and extracted with dichloromethane (30.0 mL × 3). The organic phase was washed with a saturated sodium chloride solution (50.0 mL), dried over anhydrous sodium sulfate, filtered, and concentrated to give a crude product. The crude product was obtained by direct concentration, and was purified by column chromatography (petroleum ether:ethyl acetate 5:1 to 0:1) to give 5-(6-bromo-3,4-dichloro-2-fluorobenzamido)pyrimidine 1-oxide **(19D)** (700 mg, yield: 27.9%) as a white solid.
LC-MS, M/Z (ESI): 381.9 [M+H]⁺
¹H NMR (400 MHz, DMSO-d6) δ 11.59 (br s, 1H), 8.92 (s, 2H), 8.44 (d, 1H), 8.14 (d, 1H).

### Step 4: synthesis of 5-(3,4-dichloro-2-fluoro-6-(4-(trifluoromethoxy)phenoxy)benzamido)pyrimidine 1-oxide (1-19)

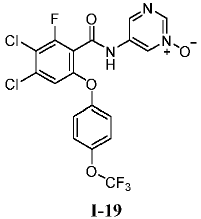

5-(6-Bromo-3,4-dichloro-2-fluorobenzamido)pyrimidine 1-oxide (650 mg, 1.71 mmol) and 4-trifluoromethoxyphenol (365 mg, 2.05 mmol) were dissolved in N,N-dimethylformamide (14.0 mL) at room temperature, and copper(I) iodide (65.0 mg, 341 µmol) and cesium carbonate (1.11 mg, 3.41 mmol) were then added. The reaction liquid was stirred at 90 °C for 1 h under nitrogen atmosphere. After the reaction was completed, water (50.0 mL) was added to the reaction liquid, and the mixture was extracted with ethyl acetate (30.0 mL × 3). The organic phase was washed with a saturated sodium chloride solution (50.0 mL) and concentrated to give a crude product. The crude product was subjected to preparative reverse phase chromatography (Phenomenex luna C18 150 × 25 mm × 10 µm; mobile phase: [water (0.225% formic acid)-acetonitrile]; B%: 45%-75%, 10 min) to give 5-(3,4-dichloro-2-fluoro-6-(4-(trifluoromethoxy)phenoxy)benzamido)pyrimidine 1-oxide (I-19, 200 mg, yield: 24.3%).
LC-MS, M/Z (ESI): 478.0 [M+H]⁺
¹H NMR (400 MHz, DMSO-d6) δ 11.30 - 11.71 (m, 1H), 8.87 (d, 1H), 8.83 (t, 1H), 8.38 (d, 1H), 7.42 (d, 2H), 7.33 (d, 1H), 7.23 - 7.28 (m, 2H).

### Example 8: Preparation of Target Compound I-20

### Synthesis of 5-(2-fluoro-6-(4-(trifluoromethoxy)phenoxy)-3-(trifluoromethyl)benzamido)pyrimidine 1-oxide (I-20)

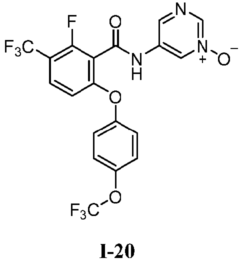

The synthetic route of the target compound **I-20** was as follows:

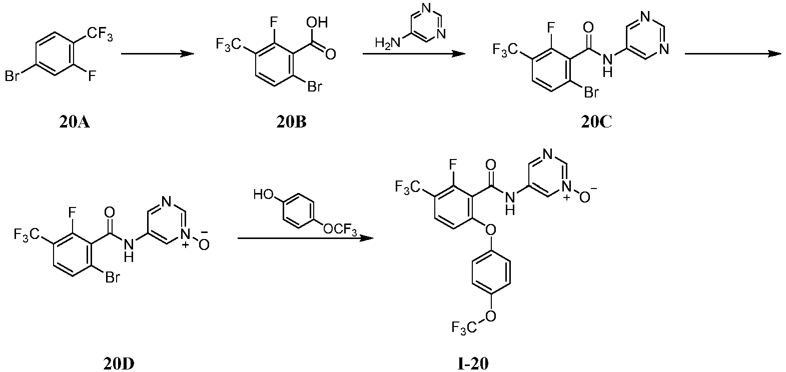

### Step 1: synthesis of 6-bromo-2-fluoro-3-(trifluoromethyl)benzoic acid (20B)

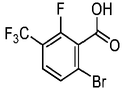

4-Bromo-2-fluoro-1-(trifluoromethyl)benzene (4.00 g, 16.4 mmol) was dissolved in tetrahydrofuran (40.0 mL) under nitrogen atmosphere, and the reaction liquid was then cooled to -70 °C. A 2 M solution (9.84 mL, 19.6 mmol) of lithium diisopropylamide in tetrahydrofuran was slowly added dropwise, and the reaction liquid was stirred at -70 °C for 1 h. Dry ice (30.0 g) was then added to the reaction liquid in batches, and the reaction liquid was successively stirred at 25 °C for 1 h. After the reaction was completed, 1 M hydrochloric acid was added to adjust the pH to 3-4, and the mixture was extracted with ethyl acetate. The organic phase was concentrated to give a crude product. The crude product was slurried with petroleum ether (50.0 mL), filtered, and dried with an oil pump to give 6-bromo-2-fluoro-3-(trifluoromethyl)benzoic acid **(20B)** (3.20 g, yield: 67.8%) as a brown solid.
LC-MS, M/Z (ESI): 284.9 [M-H]⁻
¹H NMR (400 MHz, CDCl₃) δ 9.05 (br s, 1H), 7.53 (d, 2H).

### Step 2: synthesis of 6-bromo-2-fluoro-N-(pyrimidin-5-yl)-3-(trifluoromethyl)benzamide (20C)

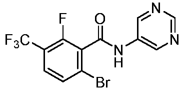

6-Bromo-2-fluoro-3-(trifluoromethyl)benzoic acid (3.00 g, 10.4 mmol) and 3-aminopyrimidine (1.19 mg, 12.5 mmol) were dissolved in N,N-dimethylamide (30.0 mL) at room temperature, and N,N-diisopropylethylamine (2.70 g, 20.8 mmol) and O-(7-azabenzotriazol-1-yl)-N,N,N,N-tetratnethyluronium hexafluorophosphate (HATU, 5.16 g, 13.6 mmol) were then added. The reaction liquid was stirred at 25 °C for 12 h. After the reaction was completed, the mixture was added into water (50.0 mL) to quench the reaction, and extracted with ethyl acetate (30.0 mL × 3). The organic phase was washed with a saturated sodium chloride solution (50.0 mL), dried over anhydrous sodium sulfate, filtered, and concentrated to give a crude product. The crude product was obtained by direct concentration, and was separated by column chromatography (petroleum ether:ethyl acetate 5:1 to 1:1) to give 6-bromo-2-fluoro-N-(pyrimidin-5-yl)-3-(trifluoromethyl)benzamide **(20C)** (3.20 g, yield: 84.2%) as a yellow solid.
LC-MS, M/Z (ESI): 364.0 [M+H]⁺

### Step 3: synthesis of 5-(6-bromo-2-fluoro-3-(trifluoromethyl)benzamido)pyrimidine 1-oxide (20D)

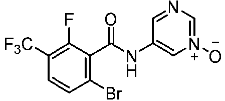

6-Bromo-2-fluoro-N-(pyrimidin-5-yl)-3-(trifluoromethyl)benzamide (2.50 g, 6.87 mmol) was dissolved in dichloromethane (50.0 mL) at 0 °C, and *m*-chloroperoxybenzoic acid (4.18 g, 19.8 mmol) was added in batches. The reaction liquid was stirred at 25 °C for 12 h. After the reaction was completed, the mixture was added into a saturated sodium sulfite solution (50.0 mL) to quench the reaction, and extracted with dichloromethane (30.0 mL × 3). The organic phase was washed with a saturated sodium chloride solution (50.0 mL), dried over anhydrous sodium sulfate, filtered, and concentrated to give a crude product. The crude product was obtained by direct concentration, and was purified by column chromatography (petroleum ether:ethyl acetate 5:1 to 0:1) to give 5-(6-bromo-2-fluoro-3-(trifluoromethyl)benzamido)pyrimidine 1-oxide **(20D)** (650 mg, yield: 24.9%) as a white solid.
LC-MS, M/Z (ESI): 380.0 [M+H]⁺

### Step 4: synthesis of 5-(2-fluoro-6-(4-(trifluoromethoxy)phenoxy)-3-(trifluoromethyl)benzamido)pyrimidine 1-oxide (I-20)

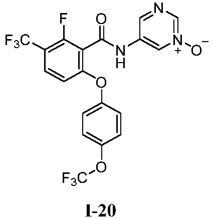

5-(6-Bromo-2-fluoro-3-(trifluoromethyl)benzamido)pyrimidine 1-oxide (400 mg, 1.05 mmol) and 4-trifluoromethoxyphenol (281 mg, 1.58 mmol) were dissolved in toluene (8.00 mL) at room temperature, and copper(I) iodide (40.1 mg, 210 µmol) and cesium carbonate (685 mg, 2.10 mmol) were then added. The reaction liquid was stirred at 90 °C for 1 h under nitrogen atmosphere. After the reaction was completed, water (50.0 mL) was added to the reaction liquid, and the mixture was extracted with ethyl acetate (30.0 mL × 3). The organic phase was washed with a saturated sodium chloride solution (50.0 mL) and concentrated to give a crude product. The crude product was subjected to preparative reverse phase chromatography (YMC-Actus Triart C18 150 × 30 mm × 7 µm; mobile phase: [water (0.225% formic acid)-acetonitrile]; B%: 50%-80%, 10 min) to give 5-(2-fluoro-6-(4-(trifluoromethoxy)phenoxy)-3-(trifluoromethyl)benzamido)pyrimidine 1-oxide (**I-20**, 155 mg, yield: 30.7%).
LC-MS, M/Z (ESI): 478.1 [M+H]⁺
¹H NMR (400 MHz, DMSO-*d₆*) δ 11.56 (s, 1H), 8.86 - 8.92 (m, 2H), 8.43 (d, 1H), 7.91 (t, 1H), 7.49 (d, 2H), 7.32 - 7.38 (m, 2H), 6.94 (d, 1H).

### Example 9: Preparation of Target Compound I-21

### Synthesis of 5- (4,5-dichloro-2-(4-fluoro-2-(trideuteromethoxy)phenoxy)benzamido)pyrimidine 1-oxide (I-21)

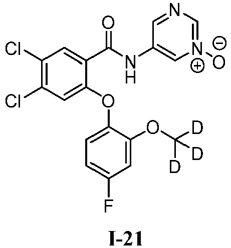

The synthetic route of the target compound **I-21** was as follows:

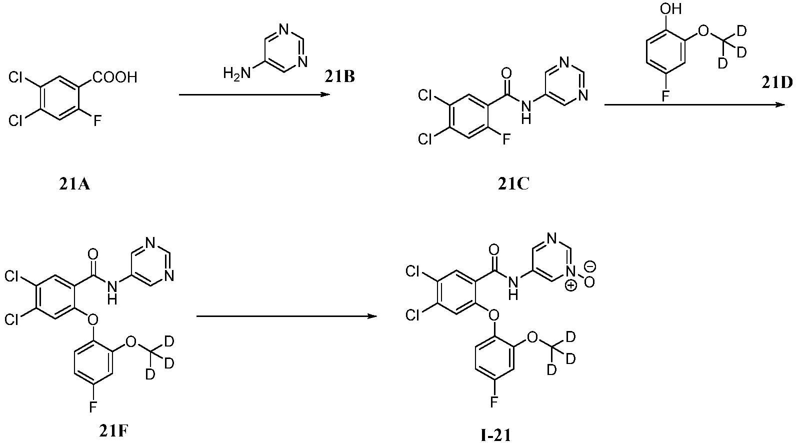

### Step 1: synthesis of 4,5-dichloro-2-fluoro-N-(pyrimidin-5-yl)benzamide (21C)

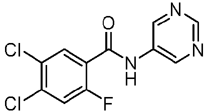

The starting materials 4,5-dichloro-2-fluorobenzoic acid (3.0 g, 14.35 mmol), 5-aminopyrimidine (1.64 g, 17.23 mmol), N,N-diisopropylethylamine (5.57 g, 43.06 mmol), and HATU (10.9 g, 28.7 mmol) were dissolved in DMF (30 mL) and reacted overnight at room temperature. After the reaction was completed, water (20 mL) and ethyl acetate (50 mL) were added for liquid separation. The organic phases were combined, added with brine (20 mL × 3) for washing, dried over anhydrous sodium sulfate, filtered, and concentrated to give a crude product, which was slurried with (petroleum ether:ethyl acetate (V/V) = 1:1) and filtered to give a filter cake, i.e., the title compound (21C, 4.0 g, yield: 97.4%).
LC-MS, M/Z (ESI): 285.6 [M+H]⁺

### Step 2: synthesis of 4,5-dichloro-2-(4-fluoro-2-(trideuteromethoxy)phenoxy)-N-(pyrimidin-5-yl)benzamide (21F)

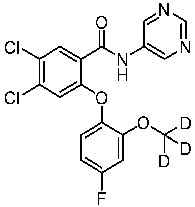

The starting materials 4,5-dichloro-2-fluoro-N-(pyrimidin-5-yl)benzamide (1.0 g, 3.5 mmol), 4-fluoro-2-(trideuteromethoxy)phenol, and cesium carbonate (761 mg, 5.24 mmol) were dissolved in DMF (10 mL) and reacted at 100 °C for 2 h. After the reaction was completed, the mixture was separated and purified by column chromatography (petroleum ether:ethyl acetate (V/V) = 1:1) to give the title compound (21F, 300 mg, yield: 56%).
LC-MS, M/Z (ESI): 411.0 [M+H]⁺

### Step 3: synthesis of 5-(4,5-dichloro-2-(4-fluoro-2-(trideuteromethoxy)phenoxy)benzamido)pyrimidine 1-oxide (target compound 1-21)

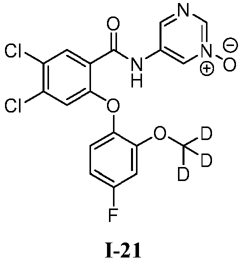

The starting materials 4,5-dichloro-2-(4-fluoro-2-(trideuteromethoxy)phenoxy)-N-(pyrimidin-5-yl)benzamide (100 mg, 0.243 mmol) and m-CPBA (62.9 mg, 0.364 mmol) were dissolved in dichloromethane (2 mL) and reacted at 25 °C for 10 h. After the reaction was completed, an aqueous sodium bicarbonate solution was added for washing, and dichloromethane (5 mL) was added for liquid separation. The organic phases were combined, dried over anhydrous sodium sulfate, and concentrated to give a crude product. The crude product was then purified by preparative reverse phase high performance liquid chromatography (column: SunFileTM Prep C18 OBDTM 5 µm 30 mm × 150 mm); mobile phase: A = acetonitrile, B = water; gradient: 1%-25%, 8 min) to give the title compound 5-(4,5-dichloro-2-(4-fluoro-2-(trideuteromethoxy)phenoxy)benzamido)pyrimidine 1-oxide (I-21) (20 mg, yield: 19.2%).
LC-MS, M/Z (ESI): 426.6 [M+H]⁺
¹H NMR (400 MHz, cdcl3) δ 9.80 (s, 1H), 9.21 (t, 1H), 8.77 (d, 1H), 8.31 (d, 1H), 8.28 (s, 1H), 7.22 (dd, 1H), 6.85 (td, 2H), 6.82 - 6.79 (m, 1H).

### Example 10: Preparation of Target Compound 1-22

### 3-(4,5-Dichloro-2-((2,2,7-trifluorobenzo[d][1,3]dioxol-4-yl)oxy)benzatnido)-pyridine 1-oxide (target compound I-22)

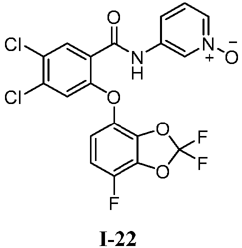

The synthetic route of compound **1-22** was as follows:

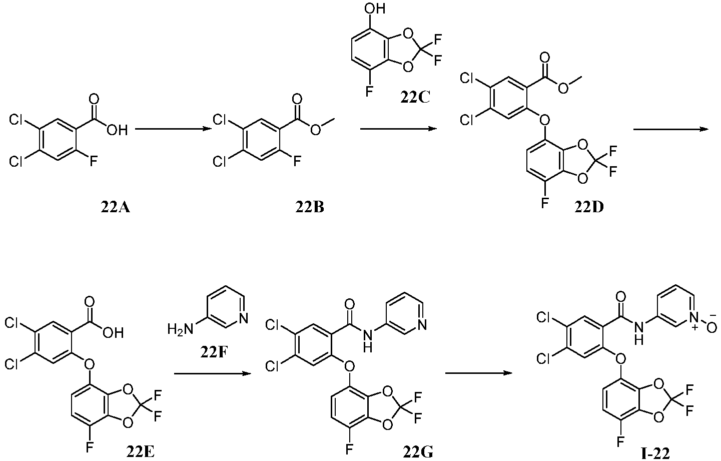

### Step 1: synthesis of methyl 4,5-dichloro-2-fluorobenzoate (22B)

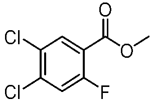

4,5-Dichloro-2-fluorobenzoic acid (1.0 g, 4.7 mmol) was added to MeOH (20 mL). Concentrated hydrochloric acid (5 drops) was then added dropwise to the reaction liquid, and the reaction liquid was reacted at 50 °C for 6 h. The reaction was completed as detected by LC-MS. The reaction liquid was concentrated by rotary evaporation. A NH₄Cl solution (10 mL) was added, and the mixture was extracted with EtOAc (20 mL × 3). The organic phases were combined and separately washed with water (20 mL × 2) and saturated brine (10 mL). The organic phase was dried over anhydrous sodium sulfate and filtered. The filtrate was concentrated by rotary evaporation, and the crude product was separated by normal phase silica gel column chromatography (EtOAc/PE = 1:20) to give methyl 4,5-dichloro-2-fluorobenzoate **(22B)** (1.1 g; yield: 95.4%) as a white solid.

### Step 2: synthesis of methyl 4,5-dichloro-2-(2,2,7-trifluorobenzo[d][1,3]dioxol-4-yl)oxy)benzoate (22D)

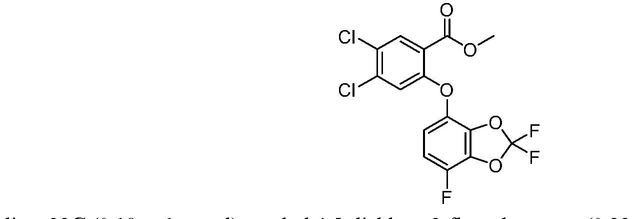

Intermediate **22C** (0.19 g, 1 mmol), methyl 4,5-dichloro-2-fluorobenzoate (0.22 g, 1 mmol), cesium carbonate (0.65 g, 2 mmol), and acetonitrile (5 mL) were added to a 25 mL round-bottom flask at room temperature and reacted at 80 °C for 8 h. The reaction liquid was cooled to room temperature, and a NH₄Cl solution (20 mL) was added to the reaction liquid. The mixture was extracted with EtOAc (20 mL × 3), and the organic phase was washed 1 time with saturated brine (20 mL), dried over Na₂SO₄, filtered, and concentrated by rotary evaporation under reduced pressure. The crude product was purified by silica gel column chromatography (EtOAc/PE = 1:10) to give methyl 4,5-dichloro-2-(3,4-difluoro-2-(deuteromethoxy)phenoxy)benzoate **(22D)** (364.0 mg; yield: 92.3%) as a light yellow solid.

### Step 3: synthesis of 4,5-dichloro-2-(2,2,7-trifluorobenzo[d][1,3]dioxol-4-yl)oxy)benzoic acid (22E)

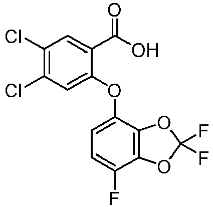

Methyl 4,5-dichloro-2-(2,2,7-trifluorobenzo[d][1,3]dioxol-4-yl)oxy)benzoate (364 mg, 0.93 mmol) was dissolved in anhydrous tetrahydrofuran (4 mL) and methanol (1 mL). Lithium hydroxide monohydrate (0.12 g, 2.77 mmol) was then weighed out and added to water (2 mL), and added to the solution described above. After the addition was completed, the reaction liquid was reacted at room temperature for 16 h. The reaction was completed as detected by LC-MS. The reaction liquid was extracted with EtOAc (15 mL × 3). The organic phases were combined and washed twice with water (10 mL × 2). The aqueous phases were combined, adjusted to pH 1 with 6 N hydrochloric acid, and then extracted with EtOAc (15 mL × 3). The organic phases were combined, washed with saturated brine (10 mL), dried over anhydrous sodium sulfate, and filtered. The filtrate was concentrated by rotary evaporation to give 5-chloro-2-(4-fluoro-2-methylphenoxy)-4-(trifluoromethyl)benzoic acid **(22E)** (320 mg; crude product). The crude product was used directly in the next step.

### Step 4: synthesis of 4,5-dichloro-N-(pyridin-3-yl)-2-(2,2,7-trifluorobenzo[d][1,3]dioxol-4-yl)oxy)benzatnide (22G)

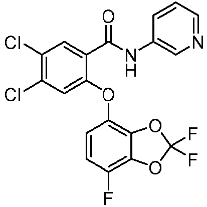

5-Chloro-2-(4-fluoro-2-methylphenoxy)-4-(trifluoromethyl)benzoic acid (320 mg, 0.84 mmol) was dissolved in acetonitrile (10 mL) under an ice bath, and TCFH (0.35 g, 1.27 mmol) and NMI (0.26 g, 3.09 mmol) were then added. After 10 min, intermediate 6 (0.08 g, 0.84 mmol) was added, and the mixture was warmed to room temperature and reacted for 10 h. Water (10 mL) was added to the reaction liquid. The mixture was extracted with EtOAc (20 mL × 3), and the organic phase was washed 1 time with saturated brine (20 mL), dried over Na₂SO₄, filtered, and concentrated by rotary evaporation under reduced pressure. The crude product was purified by normal phase silica gel column chromatography (EtOAc/PE = 1:1) to give 4,5-dichloro-N-(pyridin-3-yl)-2-(2,2,7-trifluorobenzo[d][1,3]dioxol-4-yl)oxy)benzamide **(22G)** (0.29 g; yield: 75.5%) as a light yellow solid.
LC-MS, M/Z (ESI): 457.00 [M+H]⁺

### Step 5: synthesis of 3-(4,5-dichloro-2-((2,2,7-trifluorobenzo[d][1,3]dioxol-4-yl)oxy)benzamido)-pyridine 1-oxide (target compound 1-22)

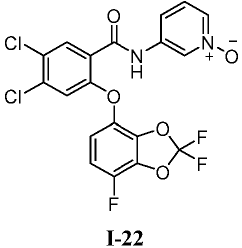

4,5-Dichloro-N-(pyridin-3-yl)-2-(2,2,7-trifluorobenzo[d][1,3]dioxol-4-yl)oxy)benzamide (7) (0.29 g, 0.63 mmol) was weighed out and dissolved in DCM (10 mL) under an ice bath, and m-CPBA (274.5 mg, 1.27 mmol) was then added. The reaction liquid was reacted at room temperature for 10 h. The reaction was incomplete, some starting materials remaining as detected by LCMS. The reaction liquid was diluted with dichloromethane (20 mL) and then washed with a saturated aqueous sodium bicarbonate solution (10 mL × 2). The organic phase was dried over Na₂SO₄, filtered, concentrated by rotary evaporation under reduced pressure, concentrated, and sent to high pressure liquid chromatography to give 3-(4,5-dichloro-2-((2,2,7-trifluorobenzo[d][1,3]dioxol-4-yl)oxy)benzamido)-pyridine 1-oxide (target compound **I-22**) (122.1 mg, yield: 40.9%).
¹H NMR (400 MHz, DMSO-d₆): δ 10.92 (s, 1H), 8.61 (s, 1H), 8.10 - 7.92 (m, 2H), 7.61 (s, 1H), 7.47 (d, J = 8.4 Hz, 1H), 7.40 - 7.33 (m, 1H), 7.24 (t, J = 9.7 Hz, 1H), 7.00 (dd, J = 9.5, 3.6 Hz, 1H).
LC-MS, M/Z (ESI): 472.99 [M+H]⁺

### Example 11: Preparation of Target Compound I-24

### Synthesis of 5-(4,5-dichloro-2-(2-(methoxy-d3)-4-(trifluoromethoxy)phenoxy)benzamido)pyrimidine 1-oxide (I-24)

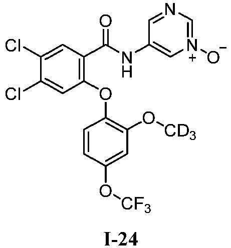

The synthetic route of the target compound **I-24** was as follows:

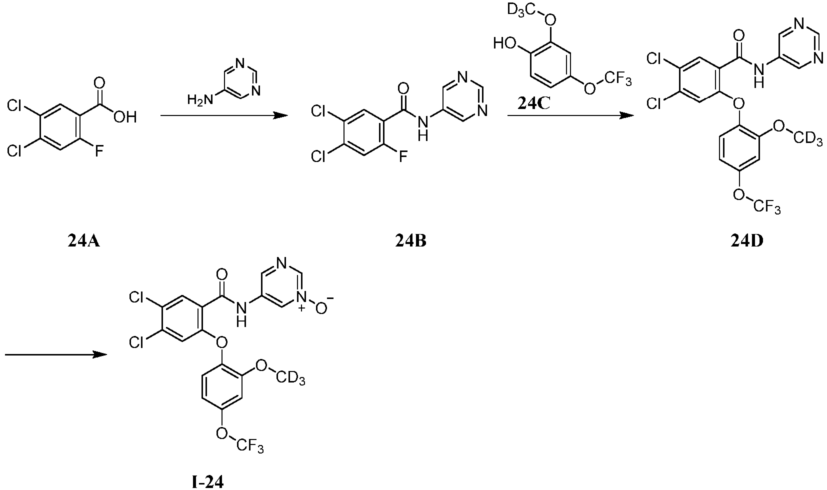

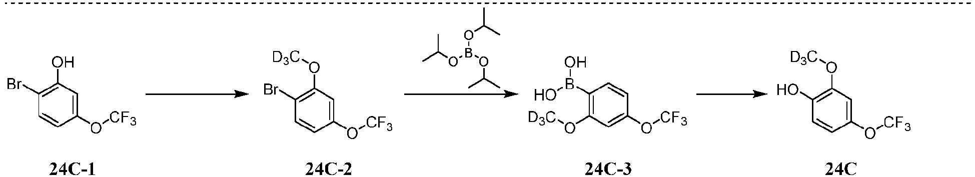

### Step 1: synthesis of 4,5-dichloro-2-fluoro-N-(pyrimidin-5-yl)benzamide (24B)

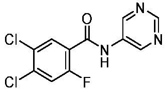

4,5-Dichloro-2-fluorobenzoic acid (2.00 g, 9.57 mmol) and 3-aminopyrimidine (1.09 g, 11.5 mmol) were dissolved in N,N-dimethylamide (20.0 mL) at room temperature, and N,N-diisopropylethylamine (2.47 g, 19.1 mmol) and O-(7-azabenzotriazol-1-yl)-N,N,N,N-tetramethyluronium hexafluorophosphate (HATU, 3.08 g, 12.4 mmol) were then added. The reaction liquid was stirred at 25 °C for 12 h. After the reaction was completed, the mixture was added into water (40.0 mL) to quench the reaction, and extracted with ethyl acetate (20.0 mL × 3). The organic phase was washed with a saturated sodium chloride solution (40.0 mL), dried over anhydrous sodium sulfate, filtered, and concentrated to give 4,5-dichloro-2-fluoro-N-(pyrimidin-5-yl)benzamide **(24B)** (2.50 g, crude product) as a yellow solid.
LC-MS, M/Z (ESI): 286.0 [M+H]⁺

### Step 2: synthesis of 1-bromo-2-deuteromethoxy-4-(trifluoromethoxy)benzene (24C-2)

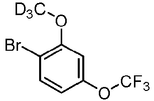

2-Bromo-5-(trifluoromethoxy)phenol (5.00 g, 19.4 mmol) was dissolved in N,N-dimethylamide (50.0 mL) at room temperature, and potassium carbonate (5.38 g, 38.9 mmol) and deuteroiodomethane (5.52 g, 38.9 mmol) were added. The reaction liquid was stirred at 25 °C for 12 h. After the reaction was completed, the mixture was added into water (100 mL) to quench the reaction, and extracted with ethyl acetate (30.0 mL × 3). The organic phase was washed with a saturated sodium chloride solution (50.0 mL), dried over anhydrous sodium sulfate, filtered, and concentrated to give 1-bromo-2-deuteromethoxy-4-(trifluoromethoxy)benzene **(24C-2)** (4.60 g, crude product) as a yellow liquid.

### Step 3: synthesis of [2-(deutero)methoxy-4-(trifluoromethoxy)phenyl] boronic acid (24C-3)

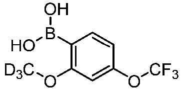

1-Bromo-2-deuteromethoxy-4-(trifluoromethoxy)benzene (4.00 g, 14.6 mmol) and triisopropyl borate were dissolved in tetrahydrofuran (40.0 mL) at room temperature. The reaction liquid was cooled to -70 °C. A solution (7.01 mL, 17.5 mmol, 2.5 M) of *n*-butyl lithium in tetrahydrofuran was slowly added dropwise. After the dropwise addition was completed, the reaction was slowly warmed to 25 °C and stirred for 12 h. After the reaction was completed, the mixture was added into a saturated ammonium chloride solution (40 mL) to quench the reaction, adjusted to pH 3-4 with 1 M diluted hydrochloric acid, and extracted with ethyl acetate (20.0 mL × 3). The organic phase was washed with a saturated sodium chloride solution (50.0 mL), dried over anhydrous sodium sulfate, filtered, and concentrated to give [2-(deutero)methoxy-4-(trifluoromethoxy)phenyl]boronic acid **(24C-3)** (3.85 g, crude product) as a yellow liquid.

### Step 4: synthesis of 2-(deutero)methoxy-4-(trifluoromethoxy)phenol (24C)

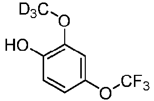

[2-(Deutero)methoxy-4-(trifluoromethoxy)phenyl]boronic acid (3.80 g, 15.9 mmol) was dissolved in dioxane (38.0 mL) at room temperature, and the reaction liquid was cooled to 0 °C. A 30% hydrogen peroxide solution (4.58 g, 47.7 mmol) was then slowly added dropwise. After the dropwise addition was completed, the reaction was slowly warmed to 25 °C and stirred for 12 h. After the reaction was completed, the mixture was slowly added to a saturated sodium thiosulfate solution (50.0 mL) to quench the reaction, and extracted with ethyl acetate (30.0 mL × 3). The organic phase was washed with a saturated sodium chloride solution (50.0 mL), dried over anhydrous sodium sulfate, filtered, and concentrated to give 2-(deutero)methoxy-4-(trifluoromethoxy)phenol **(24C)** (3.20 g, crude product) as a yellow liquid.

### Step 5: synthesis of 4,5-dichloro-2-[2-(deutero)methoxy-4-(trifluoromethoxy)phenoxy]-N-(pyrimidin-5-yl)benzamide (24D)

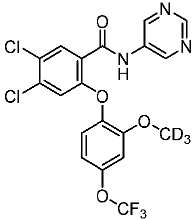

4,5-Dichloro-2-fluoro-N-(pyrimidin-5-yl)benzamide (1.00 g, 3.50 mmol) and 2-(deutero)methoxy-4-(trifluoromethoxy)phenol (886 mg, 4.19 mmol) were dissolved in N,N-dimethylamide (10.0 mL) at room temperature, and cesium carbonate (2.28 g, 6.99 mmol) was added. The reaction liquid was stirred at 80 °C for 12 h. After the reaction was completed, the mixture was added into water (20.0 mL) to quench the reaction, and extracted with ethyl acetate (10.0 mL × 3). The organic phase was washed with a saturated sodium chloride solution (20.0 mL), dried over anhydrous sodium sulfate, filtered, and concentrated to give a crude product. The crude product was separated by column chromatography (silica, petroleum ether:ethyl acetate = 5:1 to 1:1) to give 4,5-dichloro-2-[2-(deutero)methoxy-4-(trifluoromethoxy)phenoxy]-N-(pyrimidin-5-yl)benzamide (24D) (140 mg, yield: 8.38%) as a yellow solid.
LC-MS, M/Z (ESI): 477.1 [M+H]⁺
¹H NMR (400 MHz, CDCl₃) δ 9.60 (br s, 1H), 9.07 (br s, 2H), 9.00 (s, 1H), 8.32 (s, 1H), 7.25 (d, 1H), 6.94 - 7.00 (m, 2H), 6.88 (s, 1H).

### Step 6: synthesis of 5-{4,5-dichloro-2-[2-deuteromethoxy-4-(trifluoromethoxy)phenoxy]benzatnido}pyrimidine nitrogen oxide (I-24)

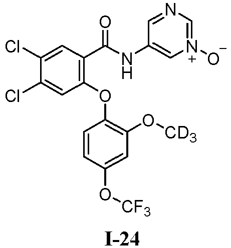

4.5-Dichloro-2-|2-(deutero)methoxy-4-(trifluoromethoxy)phenoxy]-N-(pyrimidin-5-yl)benzamide (140 mg, 293 µmol) was dissolved in dichloromethane (4.00 mL) at 0 °C, and m-chloroperoxybenzoic acid (71.5 mg, 352 µmol) was added in batches. The reaction liquid was stirred at 25 °C for 12 h. After the reaction was completed, the mixture was added into a saturated sodium sulfite solution (5.00 mL) to quench the reaction, and extracted with dichloromethane (5.00 mL × 3). The organic phase was washed with a saturated sodium chloride solution (10.0 mL), dried over anhydrous sodium sulfate, filtered, and concentrated to give a crude product. The crude product was obtained by direct concentration, and was subjected to preparative reverse phase chromatography column: Phenomenex luna C18 150 × 25 mm × 10 µm; (mobile phase: [water (0.225% formic acid)-acetonitrile]; B%: 58%-88%, 7 min) to give 5-(4,5-dichloro-2-(2-(methoxy-d3)-4-(trifluoromethoxy)phenoxy)benzamido)pyrimidine 1-oxide (**I-24**) (20.5 mg, yield: 13.9%).
LC-MS, M/Z (ESI): 492.9 [M+H]⁺
¹H NMR (400 MHz, DMSO-d6) δ 11.02 (s, 1H), 8.90 (s, 1H), 8.86 (d, 1H), 8.47 (d, 1H), 7.99 (s, 1H), 7.28 (d, 1H), 7.19 (d, 1H), 7.07 (s, 1H), 6.99 (dd, 1H).

### Example 12: Preparation of Target Compound I-25

### 5-(3-Chloro-6-(4-(difluoromethoxy)phenoxy)-2-fluoro-4-(trifluoromethyl)benzamido)pyrimidine 1-oxide (I-25).

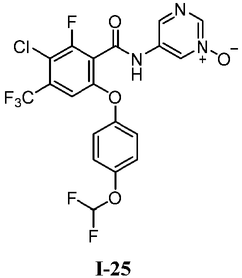

The synthetic route of the target compound **I-25** was as follows:

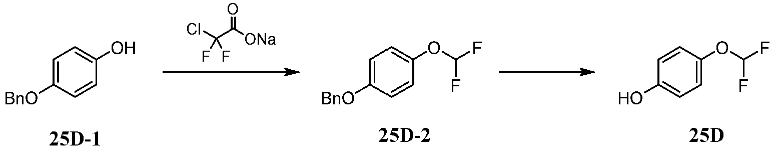

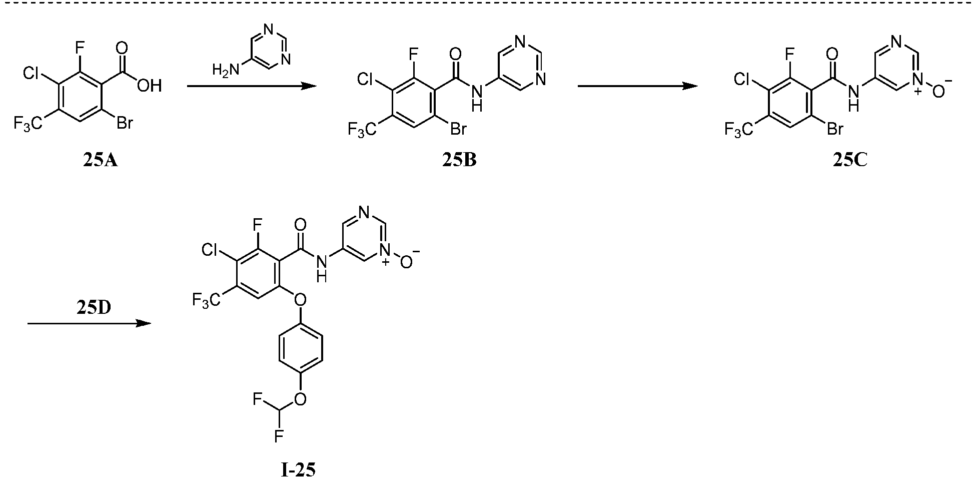

### Step 1: 1-(benzyloxy)-4-(difluoromethoxy)benzene (25D-2)

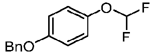

4-(Benzyloxy)phenol (5.00 g, 24.9 mmol) and sodium 2-chloro-2,2-difluoroacetate (5.71 g, 37.4 mmol) were dissolved in N,N-dimethylformamide (50.0 mL). Cesium carbonate (16.3 g, 49.9 mmol) was then added, and the reaction liquid was stirred at 100 °C for 3 h under nitrogen atmosphere. After the reaction was completed, the reaction liquid was extracted with ethyl acetate (50.0 mL × 3) and water (100 mL). The organic phase was washed with a saturated sodium chloride solution (100 mL), dried over anhydrous sodium sulfate, filtered, and concentrated to give a yellow oily crude product. The crude product was separated and purified by column chromatography (silica, petroleum ether:ethyl acetate = 20:1-10:1) to give a white solid: (1-(benzyloxy)-4-(difluoromethoxy)benzene (2.40 g, yield: 38.4%).

¹H NMR (400 MHz, DMSO-*d₆*) *δ* 7.43 - 7.48 (m, 2 H) 7.36 - 7.42 (m, 2 H) 7.30 - 7.36 (m, 1 H) 7.27 - 7.29 (m, 1 H) 7.10 - 7.15 (m, 2 H) 7.09 (s, 1 H) 7.02 - 7.08 (m, 2 H) 6.90 (s, 1 H) 5.06 - 5.14 (m, 2 H).

### Step 2: 4-(difluoromethoxy)phenol (25D)

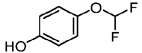

(1-(Benzyloxy)-4-(difluoromethoxy)benzene (1.00 g, 4.00 mmol) was dissolved in methanol (5.00 mL). Wet palladium on carbon (127 mg, 119 µmol, 10% purity) was then added. The reaction flask was purged three times with hydrogen, and the mixture was stirred at 25 °C for 12 h under hydrogen (50 Psi) stream. After the reaction was completed, the mixture was subjected to suction filtration and concentrated to give a black semi-oily substance: 4-(difluoromethoxy)phenol (530 mg, yield: 82.8%).

¹H NMR (400 MHz, DMSO-*d₆*) δ 9.53 (br s, 1 H) 6.74 - 7.20 (m, 6 H).

### Step 3: 6-bromo-3-chloro-2-fluoro-N-(pyrimidin-5-yl)-4-(trifluoromethyl)benzamide (25B)

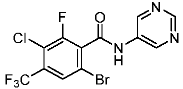

6-Bromo-3-chloro-2-fluoro-4-(trifluoromethyl)benzoic acid (10.0 g, 31.1 mmol) was dissolved in N,N-dimethylformamide (100 mL), and O-(7-azabenzotriazol-1-yl)-N,N,N,N-tetramethyluronium hexafluorophosphate (15.4 g, 40.4 mmol) was then added. The reaction liquid was stirred at 25 °C for 0.5 h. Pyrimidin-5-amine (3.55 g, 37.3 mmol) and N,N-diisopropylethylamine (8.04 g, 62.2 mmol) were then added, and the reaction liquid was stirred at 25 °C for 2.5 h. After the reaction was completed, water (200 mL) was added to the reaction liquid, and the mixture was extracted with ethyl acetate (100 mL × 3). The organic phase was washed with water (200 mL) and a saturated sodium chloride solution (100 mL), dried over anhydrous sodium sulfate, filtered, and concentrated to give a crude product as a yellow liquid. The crude product was separated and purified by column chromatography (silica, petroleum ether:ethyl acetate = 5:1-1:1) to give a yellow solid: 6-bromo-3-chloro-2-fluoro-N-(pyrimidin-5-yl)-4-(trifluoromethyl)benzamide (8.70 g, yield: 70.1%).
LC-MS, M/Z (ESI):499.9 [M+H]⁺
¹H NMR (400 MHz, DMSO-*d₆*) *δ* 11.38 - 11.55 (m, 1 H) 9.09 (s, 2 H) 9.03 (s, 1 H) 8.17 - 8.24 (m, 1 H) 2.69 (s, 2 H).

### Step 4: 5-(6-bromo-3-chloro-2-fluoro-4-(trifluoromethyl)benzamido)pyrimidine 1-oxide (25C)

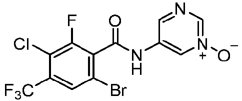

6-Bromo-3-chloro-2-fluoro-N-(pyrimidin-5-yl)-4-(trifluoromethyl)benzamide (5.00 g, 12.5 mmol) was dissolved in N,N-dimethylformamide (50.0 mL), and m-chloroperoxybenzoic acid (10.2 g, 50.2 mmol, 85% purity) was then added at 0 °C under nitrogen atmosphere. The reaction liquid was stirred at 25 °C for 12 h. After the reaction was completed, an anhydrous sodium sulfite solution (150 mL) and a sodium bicarbonate solution (150 mL) were added to the reaction liquid, and the mixture was extracted with ethyl acetate (50.0 mL × 3 mL). The organic phase was washed with a saturated sodium chloride solution (100 mL), dried over anhydrous sodium sulfate, filtered, and concentrated to give a crude product as a yellow liquid. The crude product was separated and purified by column chromatography (silica, petroleum ether:ethyl acetate = 5:1-0:1) to give a light white solid: 5-(6-bromo-3-chloro-2-fluoro-4-(trifluoromethyl)benzamido)pyrimidine 1-oxide (700 mg, yield: 13.5%).
LC-MS, M/Z (ESI):415.9 [M+H]⁺
¹H NMR (400 MHz, DMSO-*d₆*) *δ* 11.66 (s, 1 H) 8.94 (d, *J*=1.75 Hz, 1 H) 8.90 - 8.93 (m, 1 H) 8.44 (d, *J*=2.00 Hz, 1 H) 8.22 (d, *J*=1.13 Hz, 1 H).

### Step 5: 5-(3-chloro-6-(4-(difluoromethoxy)phenoxy)-2-fluoro-4-(trifluoromethyl)benzamido)pyrimidine 1-oxide (I-25)

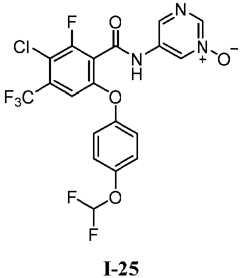

5-{3-Chloro-6-[4-(difluoromethoxy)phenoxy]-2-fluoro-4-(trifluoromethyl)benzamido}pyrimidin-1-cation-1-ol anion (100 mg, 240 µmol) and 4-(difluoromethoxy)phenol (42.4 mg, 264 µmol) were dissolved in N,N-dimethylformamide (2.00 mL). Cesium carbonate (157 mg, 482 µmol) and copper(I) iodide (4.60 mg, 24.1 µmol) were then separately added, and the reaction liquid was stirred at 100 °C for 10 min. After the reaction was completed, an organic phase crude product was obtained by filtration. The crude product was separated and purified by reverse phase high performance liquid chromatography, the separation method being: column: YMC-Actus Triart C18 150 × 30 mm × 7 µm; mobile phase: [water (formic acid)-acetonitrile]; gradient: 48%-78% B over 7 min, to give 5-(3-chloro-6-(4-(difluoromethoxy)phenoxy)-2-fluoro-4-(trifluoromethyl)benzamido)pyrimidine 1-oxide (33.6 mg, yield: 27.7%).

¹H NMR (400 MHz, DMSO-*d₆*) δ 11.55 - 11.65 (m, 1 H) 8.89 (d, *J*=1.75 Hz, 1 H) 8.85 (t, *J*=1.94 Hz, 1 H) 8.40 (d, *J=2.00* Hz, 1 H) 7.39 (s, 1 H) 7.26 (s, 5 H) 7.21 (s, 1 H) 7.03 (s, 1 H).

### Example 13: Preparation of Target Compound 1-26

### 5-Chloro-(5-chloro-2-(3,4-difluoro-2-(deuteromethoxy)phenoxy)-4-(trifluoromethyl)benzamido)pyrimidine 1-oxide (target compound 1-26)

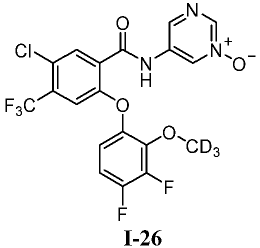

The synthetic route of compound **1-26** was as follows:

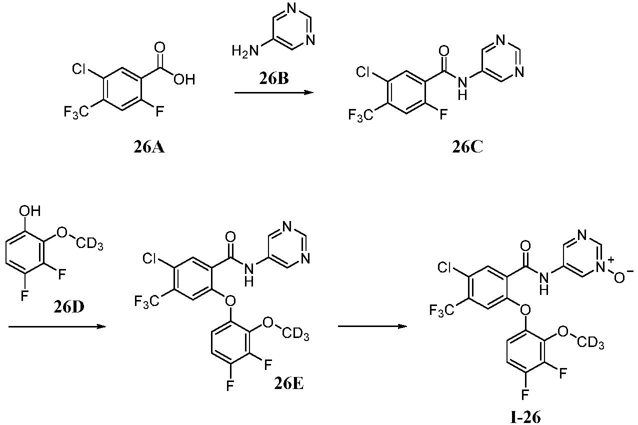

### Step 1: synthesis of 5-chloro-2-fluoro-N-(pyrimidin-5-yl)-4-(trifluoromethyl)benzamide (26C)

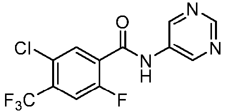

Compound **26A** (0.5 g, 2 mmol), compound **26B** (0.22 g, 2.2 mmol), DIEA (0.78 g, 6 mmol), and HATU (1.14 g, 3 mmol) were added to acetonitrile (20 mL) under an ice bath. The reaction liquid was reacted at room temperature overnight. The reaction was completed as detected by LC-MS. Water (20 mL) was added to the reaction liquid, and the mixture was then extracted with EtOAc (30 mL × 3). The organic phases were combined and separately washed with water (20 mL × 2) and saturated brine (20 mL). The organic phase was dried over anhydrous sodium sulfate and filtered, the filtrate was concentrated by rotary evaporation, and the crude product was separated by normal phase silica gel column chromatography (EtOAc/PE = 1:1) to give 5-chloro-2-fluoro-N-(pyrimidin-5-yl)-4-(trifluoromethyl)benzamide **(26C)** (0.48 g; yield: 76.3%) as a light yellow solid.

LC-MS, M/Z (ESI): 320.0 [M+H]⁺.

### Step 2: synthesis of 5-chloro-2-(3,4-difluoro-2-(deuteromethoxy)phenoxy)-N-(pyrimidin-5-yl)-4-(trifluoromethyl)benzamide (26E)

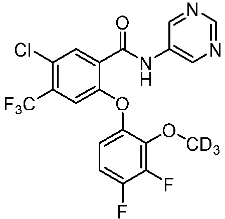

Intermediate **26C** (0.48 g, 1.5 mmol), intermediate **26D** (0.25 g, 1.5 mmol), cesium carbonate (0.73 mg, 2.25 mmol), and acetonitrile (10 mL) were added to a 25 mL eggplant-shaped bottle at room temperature and reacted at 80 °C for 5 h. The reaction liquid was cooled to room temperature, and water (10 mL) was added to the reaction liquid. The mixture was extracted with EtOAc (20 mL × 3), and the organic phase was washed 1 time with saturated brine (20 mL), dried over Na₂SO₄, filtered, and concentrated by rotary evaporation under reduced pressure. The crude product was separated by normal phase silica gel column chromatography (EtOAc/PE = 1:1) to give 5-chloro-2-(3,4-difluoro-2-(deuteromethoxy)phenoxy)-N-(pyrimidin-5-yl)-4-(trifluoromethyl)benzamide (26E) (70.0 mg; yield: 22.24%) as a light yellow solid.

LC-MS, M/Z (ESI): 463.07 [M+H]⁺.

### Step 3: synthesis of 5-chloro-(5-chloro-2-(3,4-difluoro-2-(deuteromethoxy)phenoxy)-4-(trifluoromethyl)benzamido)pyrimidine-1-oxide (target compound I-26)

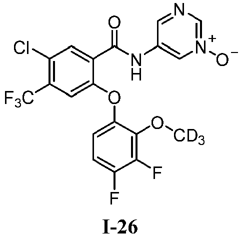

Intermediate 26E (100 mg, 0.22 mmol) was weighed out and dissolved in DCM (3 mL) under an ice bath, and m-CPBA (91.5 mg, 0.45 mmol) was then added. The reaction liquid was reacted at room temperature for 10 h. The reaction was incomplete, some starting materials remaining as detected by LCMS. The reaction liquid was diluted with dichloromethane (20 mL) and then washed with a saturated aqueous sodium bicarbonate solution (10 mL × 2). The organic phase was dried over Na₂SO₄, filtered, concentrated by rotary evaporation under reduced pressure, concentrated, and sent to high pressure liquid chromatography to give 5-chloro-(5-chloro-2-(3,4-difluoro-2-(deuteromethoxy)phenoxy)-4-(trifluoromethyl)benzamido)pyrimidine-1-oxide (target compound **I-26**) (26.1 mg; yield: 24.7%).
¹H NMR (400 MHz, CDCl₃): δ 9.69 (s, 1H), 9.23 (t, J = 1.9 Hz, 1H), 8.79 (d, J = 1.7 Hz, 1H), 8.35 - 8.30 (m, 2H), 7.13 (s, 1H), 7.07 - 6.99 (m, 2H).
LC-MS, M/Z (ESI): 479.06 [M+H]⁺

### Example 14: Preparation of Target Compound I-28

### Synthesis of 5-(3-chloro-6-(4,5-difluoro-2-(methoxy-d3)phenoxy)-2-fluoro-4-(trifluoromethyl)benzamido)pyrimidine 1-oxide (I-28)

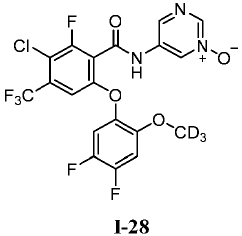

The synthetic route of the target compound **I-28** was as follows:

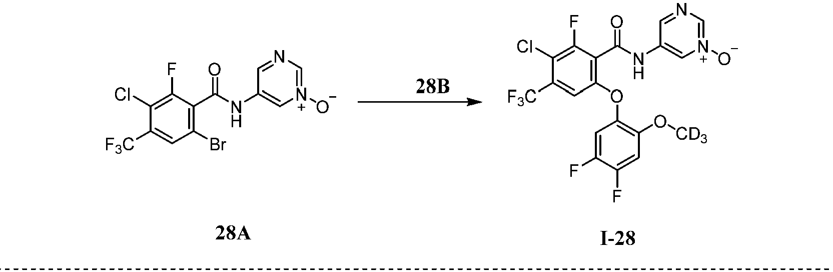

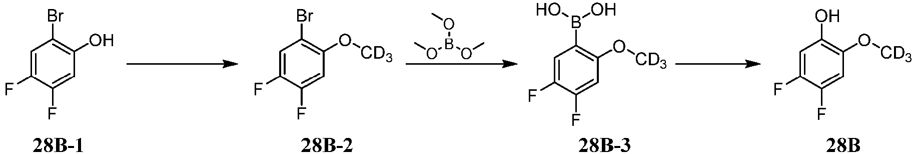

### Step 1: synthesis of 1-bromo-4,5-difluoro-2-(methoxy-d3)benzene (28B-2)

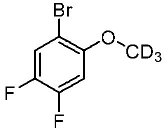

2-Bromo-4,5-difluorophenol (1.00 g, 4.78 mmol) was dissolved in N,N-dimethylamide (10.0 mL) at room temperature, and potassium carbonate (793 mg, 5.74 mmol) and deuteroiodomethane (815 mg, 5.74 mmol) were then added. The reaction liquid was stirred at 25 °C for 12 h. After the reaction was completed, water (20.0 mL) was added at 0 °C to quench the reaction, and the mixture was extracted with ethyl acetate (10.0 mL × 3). The organic phase was washed with a saturated sodium chloride solution (20.0 mL), dried over anhydrous sodium sulfate, filtered, and concentrated to give 1-bromo-4,5-difluoro-2-(methoxy-d3)benzene (800 mg, crude product) as a yellow oily product. Step 2: synthesis of (4,5-difluoro-2-(methoxy-d3)phenyl)boronic acid **(28B-3)**

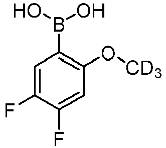

1-Bromo-4,5-difluoro-2-(methoxy-d3)benzene (700 mg, 3.10 mmol) and trimethyl borate (640 mg, 3.41 mmol) were dissolved in tetrahydrofuran (14.0 mL) at room temperature, and the reaction was then cooled to -70 °C. n-Butyl lithium (2.50 M, 1.49 mL) was slowly added dropwise, and the reaction was then warmed to 25 °C and stirred for 12 h. After the reaction was completed, the reaction liquid was adjusted to pH 3-4 with hydrochloric acid (1.00 M, 5.00 mL) at 0 °C, and the mixture was extracted with ethyl acetate (10.0 mL × 3). The organic phase was washed with a saturated sodium chloride solution (20.0 mL), dried over anhydrous sodium sulfate, filtered, and concentrated to give (4,5-difluoro-2-(methoxy-d3)phenyl)boronic acid (530 mg, crude product) as a yellow oily product.

### Step 3: synthesis of 4,5-difluoro-2-(methoxy-d3)phenol (28B)

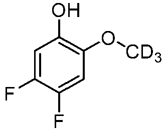

(4,5-Difluoro-2-(methoxy-d3)phenyl)boronic acid (500 mg, 2.62 mmol) was dissolved in dioxane (5.00 mL) at room temperature, and the reaction was then cooled to 0 °C. Hydrogen peroxide (890 mg, 7.86 mmol) was then slowly added dropwise. After the dropwise addition was completed, the reaction was slowly warmed to 25 °C and stirred for 2 h. After the reaction was completed, a sodium sulfite solution (20.0 mL) was added at 0 °C to quench the reaction, and the mixture was then extracted with ethyl acetate (10.0 mL × 3). The organic phase was washed with a saturated sodium chloride solution (20.0 mL), dried over anhydrous sodium sulfate, filtered, and concentrated to give 4,5-difluoro-2-(methoxy-d3)phenol (400 mg, crude product) as a yellow oily product.

### Step 4: synthesis of 5-(3-chloro-6-(4,5-difluoro-2-(methoxy-d3)phenoxy)-2-fluoro-4-(trifluoromethyl)benzamido) pyrimidine 1-oxide (I-28)

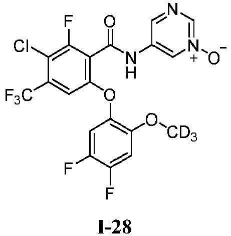

5-(6-Bromo-3-chloro-2-fluoro-4-(trifluoromethyl)benzamido)pyrimidine 1-oxide (150 mg, 361 µmol) and 4,5-difluoro-2-(methoxy-d3)phenol (147 mg, 904 µmol) were dissolved in N,N-dimethylamide (3.00 mL) at room temperature. Copper(I) iodide (13.7 mg, 72.3 µmol) and cesium carbonate (235 mg, 723 µmol) were added, and the reaction was then stirred at 100 °C for 10 min. After the reaction was completed, the mixture was subjected to preparative reverse phase column chromatography (C18 150 × 30 mm; mobile phase: [water (formic acid)-acetonitrile]; gradient 48%-78% B over 7 min) to give 5-(3-chloro-6-(4,5-difluoro-2-(methoxy-d3)phenoxy)-2-fluoro-4-(trifluoromethyl)benzamido)pyrimidine 1-oxide (**I-28**) (50.0 mg, yield: 27.8%).
LC-MS, M/Z (ESI): 497.1 [M+H]⁺
¹H NMR (400 MHz, DMSO-*d₆*) δ 11.58 (s, 1 H) 8.91 (*d, J =* 1.88 Hz, 1 H) 8.87 - 8.90 (m, 1 H) 8.44 (d, *J* = 2.00 Hz, 1 H) 7.48 - 7.55 (m, 1 H) 7.42 (dd, *J =* 12.44, 7.69 Hz, 1 H) 7.05 (s, 1 H)

### Example 15: Preparation of Target Compound 1-31

### Synthesis of 5-(5-chloro-2-(4-fluoro-2-methylphenoxy)-4-(trifluoromethyl)benzamido)pyrimidine 1-oxide (I-31)

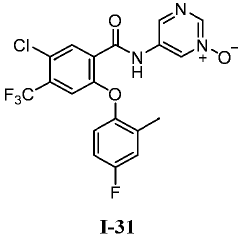

The synthetic route of the target compound **I-31** was as follows:

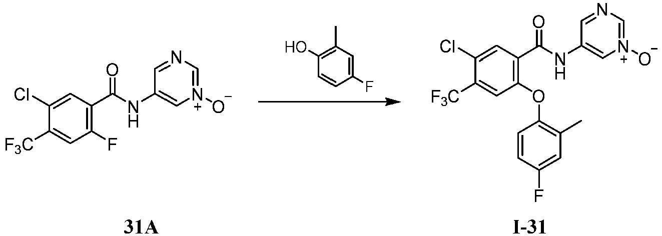

### Step 1: synthesis of 5-(5-chloro-2-(4-fluoro-2-methylphenoxy)-4-(trifluoromethyl)benzamido)pyrimidine 1-oxide (I-31)

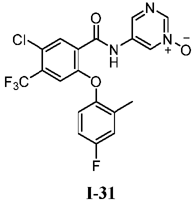

5-(5-Chloro-2-fluoro-4-(trifluoromethyl)benzamido)pyrimidine 1-oxide (25.0 mg, 74.4 µmol) (synthesized referring to Example 5) and 4-fluoro-2-methylphenol (12.2 mg, 96.8 µmol) were dissolved in N,N-dimethylamide (1.00 mL) at room temperature. Cesium carbonate (48.5 mg, 148 µmol) was then added, and the reaction was stirred at 120 °C for 12 h. After the reaction was completed, the mixture was subjected to preparative reverse phase column chromatography (Phenomenex luna C18 150 × 25 mm × 10 µm; mobile phase: [water (formic acid)-acetonitrile]; gradient 45%-75% B over 10 min) to give 5-(5-chloro-2-(4-fluoro-2-methylphenoxy)-4-(trifluoromethyl)benzamido)pyrimidine 1-oxide (**I-31**) (11.7 mg, yield: 35.6%).
LC-MS, M/Z (ESI): 441.9 [M+H]⁺
¹H NMR (400 MHz, DMSO-*d₆*) *δ* 11.23 (br s, 1 H) 8.86 - 8.92 (m, 2 H) 8.45 (d, *J =* 1.63 Hz, 1 H) 8.10 (s, 1 H) 7.22 (br d, *J =* 8.88 Hz, 1 H) 7.13 (s, 1 H) 7.08 - 7.11 (m, 2 H) 2.17 (s, 3 H)

### Example 16: Preparation of Target Compound 1-32

### 3-(3,4-Dichloro-2-fluoro-6-((2,2,7-trifluorobenzo[d][1,3]dioxol-4-yl)oxy)benzamido)pyridine-1-oxide (target compound I-32)

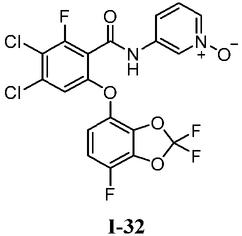

The synthetic route of compound 1-32 was as follows:

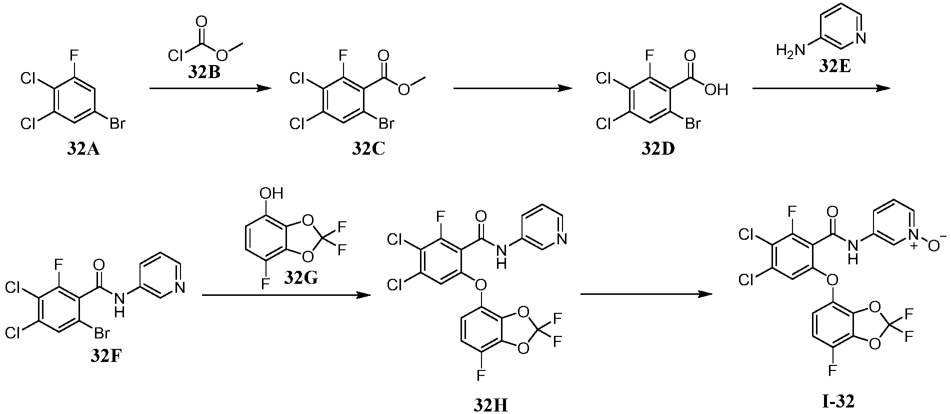

### Step 1: synthesis of methyl 6-bromo-3,4-dichloro-2-fluorobenzoate (32C)

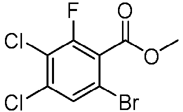

5-Bromo-1,2-dichloro-3-fluorobenzene (2.0 g, 8.20 mmol) was dissolved in anhydrous tetrahydrofuran (20 mL), and the system was vacuumized and purged with nitrogen, which was repeated three times. The reaction liquid was cooled to -78 °C under nitrogen atmosphere, and LDA (4.51 mL, 9.02 mmol, 2.0 M) was added. After the addition was completed, the reaction liquid was successively reacted at this temperature for 30 min. Methyl chloroformate (0.92 g, 9.84 mmol) was slowly added dropwise, and the temperature was maintained at no more than -65 °C. After the addition was completed, the reaction liquid was slowly warmed to room temperature and successively reacted for 10 h. The reaction was completed as detected by TLC (PE:EA = 5:1). A saturated aqueous ammonium chloride solution (10 mL) was added to quench the reaction. Water (20 mL) was added for dilution, and the mixture was then extracted with EtOAc (25 mL × 3). The organic phases were combined, washed with saturated brine (25 mL), dried over anhydrous sodium sulfate, and filtered. The filtrate was concentrated by rotary evaporation, and the crude product was separated by normal phase silica gel column chromatography (EtOAc/PE = 0%-20%) to give methyl 6-bromo-3,4-dichloro-2-fluorobenzoate **(32C)** (2.20 g; yield: 88.9%).

### Step 2: 6-bromo-3,4-dichloro-2-fluorobenzoic acid (32D)

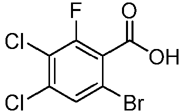

Methyl 6-bromo-3,4-dichloro-2-fluorobenzoate (1.0 g, 3.31 mmol) was dissolved in anhydrous tetrahydrofuran (6 mL) and methanol (1 mL). Lithium hydroxide monohydrate (0.42 g, 9.93 mmol) was then weighed out and added to water (3 mL), and added to the solution described above. After the addition was completed, the reaction liquid was reacted at room temperature for 16 h. The reaction was completed as detected by TLC (PE:EA = 3:1). The reaction liquid was extracted with EtOAc (15 mL × 3). The organic phases were combined and washed twice with water (10 mL × 2). The aqueous phases were combined, adjusted to pH 1 with 6 N hydrochloric acid, and then extracted with EtOAc (15 mL × 3). The organic phases were combined, washed with saturated brine (10 mL), dried over anhydrous sodium sulfate, and filtered. The filtrate was concentrated by rotary evaporation to give 6-bromo-3,4-dichloro-2-fluorobenzoic acid **(32D)** (0.50 g; crude product). The crude product was used directly in the next step.

### Step 3: 6-bromo-3,4-dichloro-2-fluoro-N-(pyridin-3-yl)benzamide (32F)

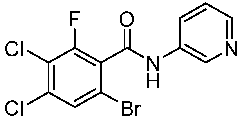

6-Bromo-3,4-dichloro-2-fluorobenzoic acid **(32D)** (0.50 g, 1.74 mmol) was dissolved in anhydrous DMF (5 mL) under an ice bath, and HATU (0.99 g, 2.61 mmol) was then added. After 10 min, 3-aminopyridine (0.20 g, 2.08 mmol) and DIPEA (0.67 g, 5.21 mmol) were added. After the addition was completed, the mixture was warmed to room temperature and reacted at room temperature for 2 h. The reaction was completed as detected by TLC (PE:EA = 3:1). Water (10 mL) was added to the reaction liquid, and the mixture was extracted with EtOAc (20 mL × 3). The organic phase was washed 1 time with saturated brine (20 mL), dried over Na₂SO₄, filtered, and concentrated by rotary evaporation under reduced pressure. The crude product was separated by normal phase silica gel column chromatography (PE/EtOAc = 0%-50%) to give 6-bromo-3,4-dichloro-2-fluoro-N-(pyridin-3-yl)benzamide (32F) (0.60 g; yield: 94.91%).

### Step 4: 3,4-dichloro-2-fluoro-N-(pyn*din-3-yl)-6-((2,2,7-trifluorobenzo[d][1,3]dloxan-4-yl)oxy)benzamide (32H)

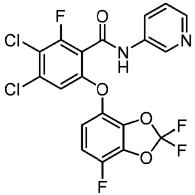

6-Bromo-3,4-dichloro-2-fluoro-N-(pyridin-3-yl)benzamide (0.15 g, 0.41 mmol) was weighed out and dissolved in ACN (3 mL) under nitrogen atmosphere, and compound **32G** (85.8 mg, 0.41 mmol), potassium carbonate (170.1 mg, 1.24 mmol), and copper(I) iodide (15.7 mg, 0.08 mmol) were then sequentially added. After the addition was completed, the reaction liquid was heated to 80 °C and reacted under the condition for 2 h. The reaction was completed as detected by LCMS. The reaction liquid was cooled to room temperature, and a NH₄Cl solution (20 mL) was added to the reaction liquid. The mixture was extracted with EtOAc (20 mL × 3), and the organic phase was washed with saturated brine (20 mL), dried over Na₂SO₄, filtered, and concentrated by rotary evaporation under reduced pressure. The crude product was purified and separated by silica gel preparative plate ((DCM: MeOH = 10:1):(PE: EtOAc = 10:1) = 3:1) to give 3,4-dichloro-2-fluoro-N-(pyridin-3-yl)-6-((2,2,7-trifluorobenzo[d][1,3]dloxan-4-yl)oxy)benzamide **(32H)** (140 mg; yield: 71.5%).

### Step 5: 3-(3.4-dichloro-2-fluoro-6-((2,2,7-trifluorobenzo[d][1,3]dioxol-4-yl)oxy)benzamido)pyridine-1-oxide (target compound I-32)

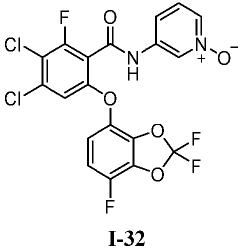

3,4-Dichloro-2-fluoro-N-(pyridin-3-yl)-6-((2,2,7-trifluorobenzo[d][1,3]dioxan-4-yl)oxy)benzamide (0.14 g, 0.29 mmol) was separately weighed out and dissolved in DCM (3 mL) at room temperature, and m-CPBA (0.15 g, 85%, 0.74 mmol) was then added. The reaction liquid was reacted at room temperature for 2 h. The reaction was completed as detected by TLC (EtOAc). The reaction liquid was diluted with dichloromethane (20 mL) and then washed with a saturated aqueous sodium bicarbonate solution (10 mL × 2). The organic phase was dried over Na₂SO₄, concentrated, and sent to high pressure liquid chromatography to give 3-(3,4-dichloro-2-fluoro-6-((2,2,7-trifluorobenzo[d][1,3]dioxol-4-yl)oxy)benzamido)pyridine 1-oxide (target compound 1-32) (30.7 mg, yield: 21.2%). ¹H NMR (400 MHz, DMSO_*d₆*):δ 11.29 (s, 1H), 8.62 (s, 1H), 8.04 (d, 1H, *J=8.0* Hz), 7.53 (s, 1H), 7.47-7.45 (m, 2H), 7.42-7.32 (m, 1H), 7.29-7.09 (m, 1H).
LC-MS, M/Z (ESI): 491.1[M+H]⁺

### Example 17: Preparation of Target Compound I-33

### 3-Cyclopropyl-5-(4,5-dichloro-2-(4-(trifluoromethoxy)phenoxy)benzamido)pyridine 1-oxide (I-33).

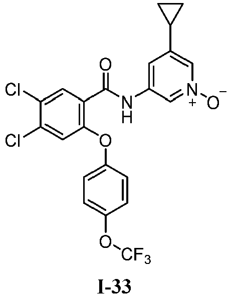

The synthetic route of the target compound **I-33** was as follows:

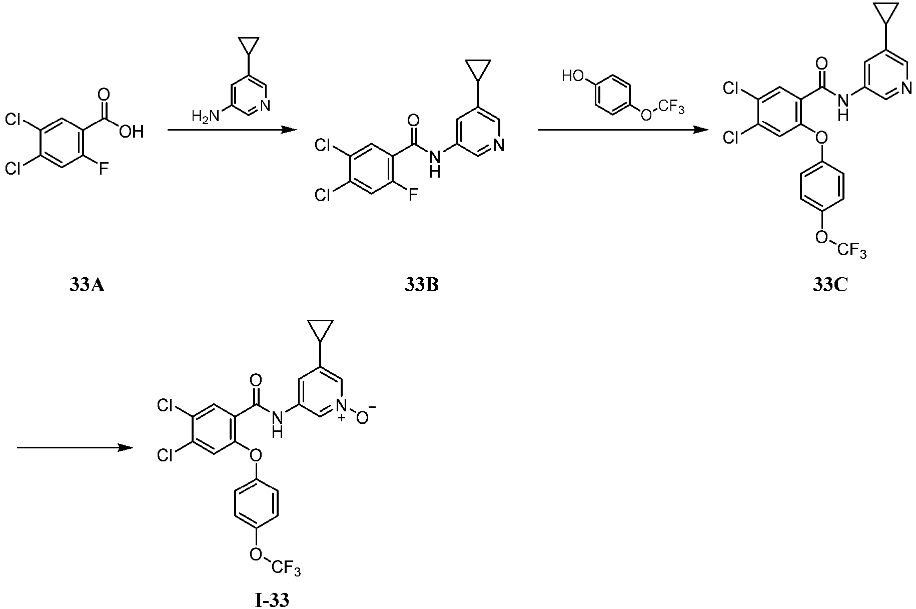

### Step 1: 4,5-dichloro-N-(5-cyclopropylpyridin-3-yl)-2-fluorobenzamide (33B)

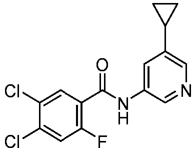

4,5-Dichloro-2-fluorobenzoic acid (300 mg, 1.44 mmol) and N,N-dimethylformamide (10.5 mg, 143 µmol) were dissolved in dichloromethane (6.00 mL). Oxalyl chloride (364 mg, 2.87 mmol) was then added dropwise at 0 °C. The reaction liquid was stirred at 0 °C for 1 h and then directly concentrated. Dichloromethane (3.00 mL) was then added, and triethylamine (217 mg, 2.15 mmol) and 5-cyclopropylpyridin-3-amine (211 mg, 1.58 mmol) were then added to the reaction liquid at 0 °C. After the reaction liquid was purged with nitrogen three times, the reaction liquid was stirred at 25 °C for 11 h. After the reaction was completed, a crude product as yellow oily substance was obtained by direct concentration. The crude product was separated and purified by column chromatography (silica, petroleum ether:ethyl acetate = 1:1) to give a pure product of 4,5-dichloro-N-(5-cyclopropylpyridin-3-yl)-2-fluorobenzoyl (300 mg, yield: 64.3%).
LC-MS, M/Z (ESI):325.1 (M+H⁺)

### Step 2: 4,5-dichloro-N-(5-cyclopropylpyridin-3-yl)-2-(4-(trifluoromethoxy)phenoxy)benzamide (33C)

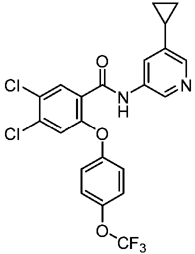

4,5-Dichloro-N-(5-cyclopropylpyridin-3-yl)-2-fluorobenzoyl (150 mg, 461 µmol) and 4-trifluoromethoxyphenol (106 mg, 599 µmol) were dissolved in N,N-dimethylformamide (3.00 mL). Cesium carbonate (300 mg, 922 µmol) was then added, and the reaction liquid was stirred at 80 °C for 12 h. After the reaction was completed, the mixture was diluted with water (30.0 mL) and then extracted with ethyl acetate (10 mL × 3). The organic phase was washed with a saturated sodium chloride solution (30.0 mL), dried over anhydrous sodium sulfate, filtered, and concentrated to give a crude product. The crude product was separated and purified by column chromatography (silica, petroleum ether:ethyl acetate = 1:1) to give a pure product of 4,5-dichloro-N-(5-cyclopropylpyridin-3-yl)-2-(4-(trifluoromethoxy)phenoxy)benzamide (200 mg, yield: 89.7%).
LC-MS, M/Z (ESI): 483.2 (M+H⁺)

### Step 3: 3-cyclopropyl-5-(4,5-dichloro-2-(4-(trifluoromethoxy)phenoxy)benzamido)pyridine 1-oxide (I-33).

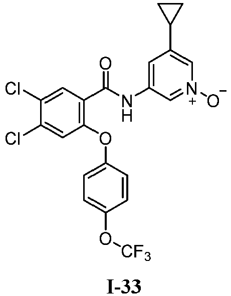

4,5-Dichloro-N-(5-cyclopropylpyridin-3-yl)-2-(4-(trifluoromethoxy)phenoxy)benzamide (200 mg, 413 µmol) was dissolved in dichloromethane (4.00 mL). m-Chloroperoxybenzoic acid (168 mg, 827 µmol, 85%) was then added at 0 °C, and the reaction liquid was stirred at 25 °C for 16 h. After the reaction was completed, the mixture was quenched with sodium sulfite (20 mL) at 0 °C, then extracted with dichloromethane (10 mL × 3), then washed with a saturated sodium bicarbonate solution (20 mL × 3), then washed with a saturated sodium chloride solution (30.0 mL), dried over anhydrous sodium sulfate, filtered, and concentrated to give a crude product. The crude product was separated and purified by reverse phase high performance liquid chromatography, the separation method being: column: YMC Triart 30 × 150 mm × 7 µm; mobile phase: [water (formic acid)-acetonitrile]; gradient: 55%-85% B over 8 min, to give 3-cyclopropyl-5-(4,5-dichloro-2-(4-(trifluoromethoxy)phenoxy)benzamido)pyridine 1-oxide (51.2 mg, yield: 24.5%).
¹H NMR (400 MHz, DMSO-*d₆*)*δ* 10.7 - 10.9 (m, 1 H) 7.99 - 8.23 (m, 2 H) 7.63 - 7.78 (m, 1 H) 7.33 - 7.59 (m, 4 H) 7.01 (br s, 1 H) 6.90 (s, 1 H) 1.78 - 2.01 (m, 1 H) 0.98 (br d, J=6.64 Hz, 2 H) 0.51 - 0.78 (m, 2 H)

Example 18: obtained referring to the synthetic methods of Examples 1-17

| **No.** | **Structural formula** | **Name** | **LC-MS, M/Z (ESI)** |
|---|---|---|---|
| I-3 | 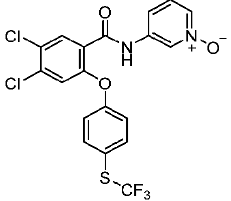 | 3-(4,5-dichloro-2-(4-((trifluoromethyl)thio)phenoxy)benzamido)pyridine 1-oxide | 473.0 [M-H]⁻ |
| I-4 | 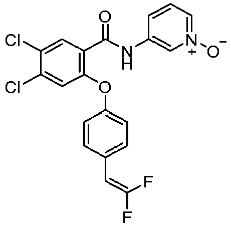 | 3-(4,5-dichloro-2-(4-(2,2-difluorovinyl)phenoxy)benzamido)pyridine 1-oxide | 435.0 [M-H]⁻ |
| I-5 | 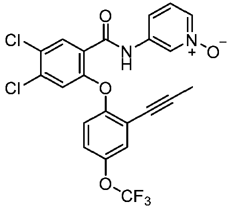 | 3-(4,5-dichloro-2-(2-(propynyl)-4-(trifluoromethoxy)phenoxy)benzamido)pyridine 1-oxide | 495.1 [M-H]⁻ |
| I-6 | 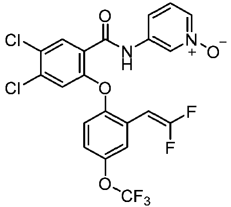 | 3-(4,5-dichloro-2-(2-(2,2-difluorovinyl)-4-(trifluoromethoxy)phenoxy)benzamido)pyridine 1-oxide | 519.0 [M-H]⁻ |
| I-7 | 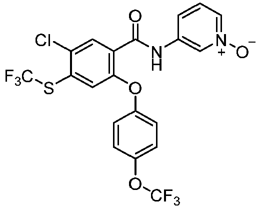 | 3-(5-chloro-2-(4-(trifluoromethoxy)phenoxy)-4-((trifluoromethyl)thio)benzamido)pyridine 1-oxide | 523.0 [M-H]⁻ |
| I-8 | 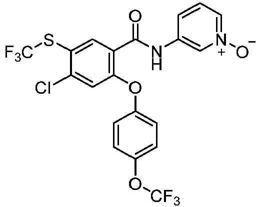 | 3-(4-chloro-2-(4-(trifluoromethoxy)phenoxy)-5-((trifluoromethyl)thio)benzamido)pyridine 1-oxide | 523.0 [M-H]⁻ |
| I-9 | 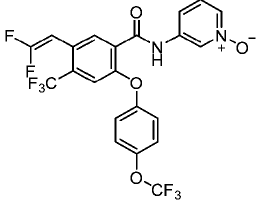 | 3-(5-(2,2-difluorovinyl)-2-(4-(trifluoromethoxy)phenoxy)-4-(trifluoromethyl)benzamido)pyridine 1-oxide | 519.1 [M-H]⁻ |
| I-10 | 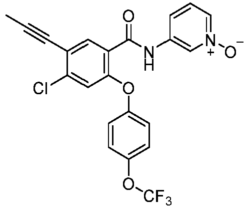 | 3-(4-chloro-5-(propynyl)-2-(4-(trifluoromethoxy)phenoxy)benzamido)pyridine 1-oxide | 461.1 [M-H]⁻ |
| I-12 | 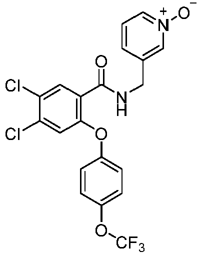 | 3-((4,5-dichloro-2-(4-(trifluoromethoxy)phenoxy)benzamido)methyl)pyridine 1-oxide | 471.1 [M-H]⁻ |
| I-13 | 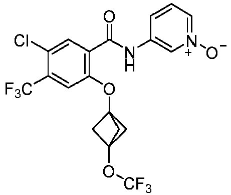 | 3-(5-chloro-2-(3-(trifluoromethoxy)bicyclo[1.1.1]pent-1-yl)oxy)-4-(trifluoromethyl)benzamide)pyridine 1-oxide | 481.1 [M-H]⁻ |
| I-14 | 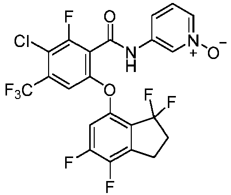 | 3-(3-chloro-2-fluoro-6-((3,3,6,7-tetrafluoro-2,3-dihydro-1H-inden-4-yl)oxy)-4-(trifluoromethyl)benzamide)pyridine 1-oxide | 537.0 [M-H]⁻ |
| I-15 | 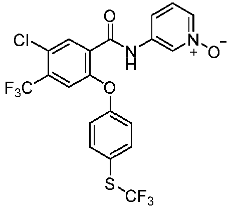 | 3-(5-chloro-4-(trifluoromethyl)-2-(4-(trifluoromethyl)thio)phenoxy)benzamido)pyridine 1-oxide | 507.1 [M-H]⁻ |
| I-23 | 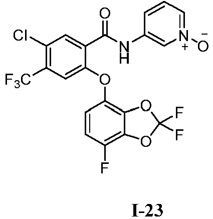 | 3-(5-chloro-2-((2,2,7-trifluorobenzo[d][1,3]dihydroxy-4-yl)oxy)-4-(trifluoromethyl)benzamido)pyridine 1-oxide | 507.0[M+H]⁺ |
| I-27 | 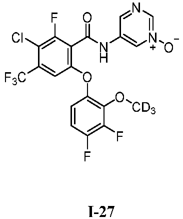 | 5-(3-chloro-6-(3,4-difluoro-2-(methoxy-d3)phenoxy)-2-fluoro-4-(trifluoromethyl)benzamido)pyrimidine 1-oxide | 497.0[M+H]⁺ |
| I-29 | 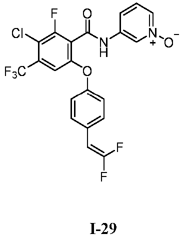 | 3-(3-chloro-6-(4-(2,2-difluorovinyl)phenoxy)-2-fluoro-4-(trifluoromethyl)benzamido)pyridine 1-oxide | 489.0[M+H]⁺ |
| I-30 | 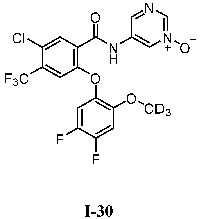 | 5-(5-chloro-2-(4,5-difluoro-2-(methoxy-d3)phenoxy)-4-(trifluoromethyl)benzamido)pyrimidine 1-oxide | 479. 0 [M+H]⁺ |
| I-34 | 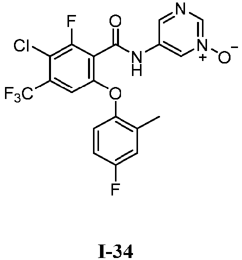 | 5-(3-chloro-2-fluoro-6-(4-fluoro-2-methylphenoxy)-4-(trifluoromethyl)benzamido)pyrimidine 1-oxide | 460.0[M+H]⁺ |
| I-35 | 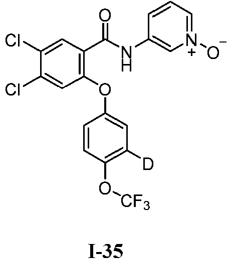 | 3-(4,5-dichloro-2-(4-(trifluoromethoxy)phenoxy-3-deutero)benzamido)pyridine 1-oxide | 460.0[M+H]⁺ |
| I-36 | 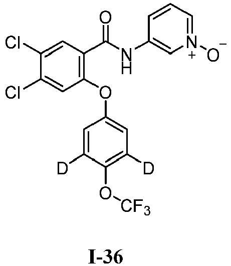 | 3-(4,5-dichloro-2-(4-(trifluoromethoxy)phenoxy-3,5-dideutero)benzamido)pyridine 1-oxide | 461.0[M+H]⁺ |
| I-37 | 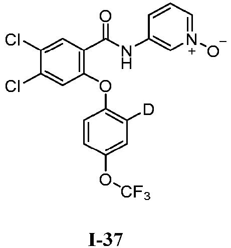 | 3-(4,5-dichloro-2-(4-(trifluoromethoxy)phenoxy-2-deutero)benzamido)pyridine 1-oxide | 460.0[M+H]⁺ |
| I-38 | 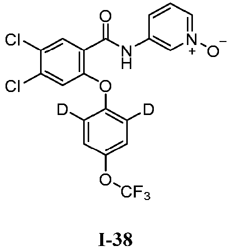 | 3-(4,5-dichloro-2-(4-(trifluoromethoxy)phenoxy-2,6-dideutero)benzamido)pyridine 1-oxide | 461.0[M+H]⁺ |
| I-39 | 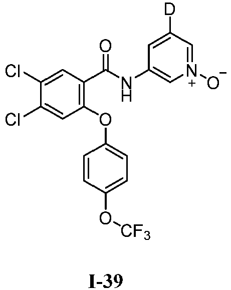 | 3-(4,5-dichloro-2-(4-(trifluoromethoxy)phenoxy)benzamido)pyridine 1-oxide-5-deutero | 460.0[M+H]⁺ |

### Test Example 1: Detection of Inhibitory Activity on Nav1.8 Ion Channel of Compounds

Reagents were purchased from Sigma (St. Louis, MO) except NaOH and KOH for acid-base titration. The final concentrations of the test compounds were all prepared on the day of the test and then dissolved in an extracellular fluid. The extracellular fluid (mM) was NaCl, 137; KCl, 4; CaCl2, 1.8; MgCl2, 1; HEPES, 10; glucose 10; and pH 7.4 (NaOH titration). All test and control compound solutions contained 1 µM TTX. The intracellular fluid (mM) was: aspartic acid, 140; magnesium chloride, 2; ethylene glycol tetraacetic acid (EGTA), 11; and N-2-hydroxyethylpiperazine-N'-2-ethanesulfonic acid (HEPES), 10. The pH was adjusted to 7.4 with cesium hydroxide. The test compounds were dissolved in dimethylsulfoxide (DMSO) at a concentration of 9 mM. On the day of the test, the compounds were then dissolved in the extracellular fluid and prepared into the required concentration.

### The electrophysiological experiment steps:

The cells were transferred to a perfusion chamber and perfusion was carried out using the extracellular fluid. The intracellular fluid was thawed on the day of the experiment. The electrodes were produced by pulling with PC-10 (Narishige, Japan). Whole-cell patch-clamp recording was carried out. Noise was filtered at one fifth of the sampling frequency. The intracellular fluid with 1/4 length of the electrode tube was added into the electrode, and the electrode was installed on a probe. The required Protocol was set, the interface was adjusted to Membrane test, and Stage was adjusted to Bath. A positive pressure was applied in the electrode. The tip of the electrode was contacted with the cell. The three-way valve of the air extractor was adjusted to a three-way state. A negative pressure was then applied to the electrode, so that the electrode and the cell formed a high-resistance seal. Stage was adjusted to Patch, and leak was controlled within -200 pA. A negative pressure was successively applied, so that cell membranes were broken, and a current path was formed. Perfusion was carried out by opening the suction filtration device and the extracellular fluid valve. The cell current was observed, and administration was started when the cell current was stable (at least 3 sweep current curves were overlapped). Administration was performed from low concentration to high concentration. The administration time of each concentration was not less than 2 min, and when the current was stable, the concentration was changed for administration.

For administration of the test samples, perfusion was carried out using a perfusion system utilizing the self gravity. During the initial recording, the peak current amplitude was observed for at least 1 min until it was stable. During the period, the CV% of all peak current amplitudes should be less than 10% to exclude up and down fluctuations of the initial current. The average of the peak current amplitudes recorded for the last 10 times during the initial recording served as the current peak of the negative control. After the initial current was stable, the test samples were dosed starting from a low concentration until the 10 recorded peak currents were again stable or the peak currents were "constant" after and before administration after continuous administration for 5 min. We defined the following two cases as "stable"or "constant": 1) if the absolute average of the peak current for 10 consecutive scans exceeded 200 pA and the CV value was less than 10%, 2) or the average of the peak current for 10 consecutive scans was between 200 pA and 50 pA and the CV value was less than 30%. The next detection of higher concentration was then given.

The average of the peak current of the last 10 scans of each concentration was taken as the peak current for that concentration for data analysis. If the stable state was not reached within 5 min, the peak current average of the last 10 scans at that time was used for data analysis as the peak current for that concentration. At the same time, the cells were discarded and no longer used for detection of higher concentration. At least two cells were tested per concentration of the compounds.

### Voltage pulse program:

The cells were clamped at -80 mV and then depolarized to 10 mV with a 10 millisecond lasting square wave to give a NaV1.8 current. This procedure was repeated every 5 seconds. The maximum current induced by the square wave was detected, and after it was stable, perfusion was carried out using the test compounds. After the reaction was stable, the blocking intensity was calculated.

### Data processing and fitting

Data collection and analysis were performed using pCLAMP 10 (Molecular Devices, Union City, CA). Stable current means that the current varies over time within a limited range. By plotting the dose-effect relationship between the gradient dilution series concentrations of the medicament and the stable current value generated by its action on HEK293/Nav1.8,
the inhibitory activity (IC₅₀) of the medicament on Nav1.8 ion channel was then calculated.

**Table 1. Inhibitory activity of compounds on Nav1.8 ion channel**

| Compound | IC₅₀ (nM) |
|---|---|
| I-1 | 14 |
| I-2 | 11 |
| I-11 | 0.64 |
| I-14 | 10 |
| I-15 | 1.4 |
| I-18 | 2.4 |
| I-19 | 0.98 |
| I-22 | 8.15 |
| I-23 | 2.9 |
| I-24 | 2.2 |
| I-25 | 2.9 |
| I-28 | 1.3 |
| I-31 | 9.75 |
| I-32 | 0.71 |
| I-34 | 1.4 |
| I-35 | 7.3 |
| I-36 | 7.8 |
| I-37 | 6.9 |
| I-38 | 8.2 |

The test results show that the compound of the present disclosure has a relatively strong inhibitory activity on Nav1.8 ion channel.

### Test Example 2: Pharmacokinetic Assay in Mice

Pharmacokinetic assay in mice was performed, wherein the mice used were 3 male ICR mice and fasted overnight. The mice were orally intragastrically dosed with the medicament (10 mg/kg). Blood was collected before administration and at 15 min, 30 min and 1 h, 2 h, 4 h, 6 h, 8 h, and 24 h after administration. Blood samples were centrifuged at 8000 rpm for 6 min at 4 °C, and plasma was collected and stored at -20 °C. The plasma at each time point was taken, and 3-5 times its amount of an acetonitrile solution containing an internal standard was added for mixing. The mixture was vortexed for 1 min and then centrifuged at 13000 rpm at 4 °C for 10 min. The supernatant was taken, and 3 times its amount of water was added for mixing. A proper amount of the resulting mixture was taken for LC-MS/MS analysis. The major pharmacokinetic parameters were analyzed using a non-compartmental model in the WinNonlin 7.0 software.

**Table 2. Results of pharmacokinetic assay in mice of compounds**

| Compound No. | Pharmacokinetic parameters in mice | | | |
|---|---|---|---|---|
| | Oral intragastric administration (10 mg/kg) | | | |
| | Cmax (ng/mL) | Tmax (hr) | AUC0-t (h*ng/mL) | T_{1/2} (h) |
| I-1 | 7370 | 0.25 | 17273 | 2.74 |
| I-11 | 2237 | 1.00 | 9074 | 2.18 |
| I-15 | 30000 | 8.00 | 538725 | 34.0 |
| I-18 | 1393 | 0.50 | 3342 | 1.22 |
| I-19 | 8880 | 0.50 | 19156 | 2.00 |
| I-20 | 8007 | 4.00 | 96162 | 2.87 |
| I-21 | 1397 | 0.50 | 2492 | 1.32 |
| I-22 | 3090 | 1.00 | 12723 | 2.88 |
| I-23 | 3450 | 1.00 | 37901 | 2.53 |
| I-24 | 1867 | 0.50 | 4560 | 1.78 |

The test results show that the compound of the present disclosure has good pharmacokinetic characteristics.

### Test Example 3: Spinal Nerve Ligation Neuropathic Pain Model in Rats

A male SD rat weighing 180-220 g was placed on an operating table in a prone position after anesthesia. An incision was made along the spinal column near the hip bone of the animal, and the fascia and muscle were separated. The L5 transverse process was carefully snapped off with a tweezer. The L5 nerve was separated with a glass needle, and the L5 nerve was ligated with 5-0 ligature. The muscle and the skin were sutured and disinfected with iodophor. After 14 days of modeling, the animals were divided into different groups, 10 animals each group. Different compounds were separately orally administered intragastrically. Mechanical pain thresholds of the animals were detected with Von-Frey fiber filaments at different time points after administration. The specific administration dose and detection time are detailed in Table 3 below.

The mechanical pain threshold detection method: The pelma of the hind limb to be tested of the test animal was continuously stimulated using the Von-Frey fiber filament to bend the fiber filament, and the foot shrinking reaction of the animal was observed. The tested animals were stimulated one by one according to the sequence of the gram number of the fiber filaments from small to large, and each gram number of the fiber filament was continuously used for stimulation for 5 times. If less than 3 positive reactions were present, the procedure described above was repeated using a larger grade of fiber filament, and if 3 or more positive reactions were present for the first time in the test, the fiber filament was the pain threshold for that animal (3 tests per animal for the average). Fiber filament gram number: 0.6, 1.0, 1.4, 2.0, 4.0, 6.0, 8.0, 10.0, and 15.0; the cut value was 15.0 g.

**Table 3: Efficacy of compounds on pain threshold of spinal nerve-ligated rats**

| No. | Mechanical pain threshold (Mean±SD) | | | |
|---|---|---|---|---|
| | Base value before modeling | 1 h after administration | 3 h after administration | 6 h after administration |
| Vehicle | 13.9±2.0 | 4.3±2.0 | 4.4±1.9 | 5.0±1.8 |
| I-1-100mpk | 13.6±1.7 | 11.0±4.0*** | 11.1±3.7*** | NA |
| I-11-100mpk | 13.0±2.8 | NA | 13.6±2.0*** | 12.1±2.5*** |
| Pregabalin-30mpk | 13.6±2.7 | 13.8±2.3*** | 12.3±2.9*** | 12.1±2.3*** |

| | | | | |
|---|---|---|---|---|
| One-way ANOVA, ***P < 0.001, **P < 0.01, *P < 0.05 NA: The time point was not scheduled for detection | | | | |

The test results show that the compound of the present disclosure can significantly improve the mechanical pain threshold reduction of animals caused by rat spinal nerve ligation modeling, and has excellent analgesic efficacy.

### Test Example 4: Incision Pain Model in Rats

A male SD rat weighing 200-250 g was fixed in a prone position after anesthesia. The lateral hind limb sole of the rat was flattened upwards, and the toes were fixed using an operation adhesive tape. Sterilization was performed. A 0.5 cm position of the plantar heel of the animal was incised with a scalpel towards the toe end to incise the fascia and the skin to make a longitudinal incision of about 1 cm. After the flexor digitorum brevis was lifted by the surgical forceps, the abdomen of the muscle was longitudinally incised with a scalpel without completely cutting off the muscle. The skin was sutured and disinfected. On the second day of modeling, the animals were divided into different groups, 8 animals each group. Different compounds were separately orally administered intragastrically. Mechanical pain thresholds of the animals were detected with Von-Frey fiber filaments at different time points after administration. The specific grouping administration dose and detection time are detailed in Table 4 below.

The mechanical pain threshold detection method: The pelma of the hind limb to be tested of the test animal was continuously stimulated using the Von-Frey fiber filament to bend the fiber filament, and the foot shrinking reaction of the animal was observed. The tested animals were stimulated one by one according to the sequence of the gram number of the fiber filaments from small to large, and each gram number of the fiber filament was continuously used for stimulation for 5 times. If less than 3 positive reactions were present, the procedure described above was repeated using a larger grade of fiber filament, and if 3 or more positive reactions were present for the first time in the test, the fiber filament was the pain threshold for that animal (3 tests per animal for the average). Fiber filament gram number: 0.6, 1.0, 1.4, 2.0, 4.0, 6.0, 8.0, 10.0, and 15.0; the cut value was 15.0 g.

**Table 4: Efficacy of compounds on pain threshold of incision pain model rats**

| No. | Mechanical pain threshold (Mean±SD) | | | |
|---|---|---|---|---|
| | Base value before modeling | 1 h after administration | 3 h after administration | 6 h after administration |
| Vehicle | 12.6±1.9 | 2.5±0.5 | 2.9±0.6 | 2.8±0.7 |
| I-1-100mpk | 12.6±1.7 | 12.5±1.9*** | 11.4±2.0*** | 8.4±1.2*** |
| Pregabalin-30mpk | 12.7±1.7 | 12.4±1.9*** | 11.6±1.1*** | 11.3±1.4*** |

| | | | | |
|---|---|---|---|---|
| One-way ANOVA, vs Vehicle group, ***P < 0.001 | | | | |

The test results show that the compound of the present disclosure can significantly improve the mechanical pain threshold reduction of animals caused by rat incision pain modeling, and has excellent analgesic efficacy.

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A compound represented by formula (I), or a tautomer, a stereoisomer, a hydrate, a solvate, a pharmaceutically acceptable salt, or a prodrug thereof: 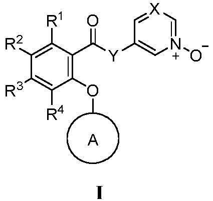 wherein
X is independently selected from N and CR^{A};
R^{A} is selected from H, D, C₁-C₆ alkyl, and C₃-C₆ cycloalkyl;
Y is independently selected from -NH- and 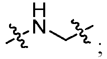
ring A is independently selected from 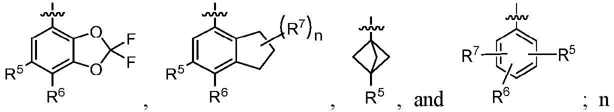
is independently selected from 0, 1, 2, 3, 4, 5, and 6;
R¹, R², R³, R⁴, R⁵*,* R⁶, and R⁷ are each independently selected from H, D, C₁-C₆ alkyl, C₁-C₆ alkyl substituted with one or more R¹¹, C₂-C₆ alkenyl, C₂-C₆ alkenyl substituted with one or more R¹², C₂-C₆ alkynyl, C₂-C₆ alkynyl substituted with one or more R¹³, -O-(C₁-C₆ alkyl), -O-(C₁-C₆ alkyl) substituted with one or more R¹⁴, -S-(C₁-C₆ alkyl), -S-(C₁-C₆ alkyl) substituted with one or more R¹⁵, C₃-C₆ cycloalkyl, C₃-C₆ cycloalkyl substituted with one or more R¹⁶, 4-8 membered heterocycloalkyl, 4-8 membered heterocycloalkyl substituted with one or more R¹⁷, 6-10 membered aryl, 6-10 membered aryl substituted with one or more R¹⁸, 5-8 membered heteroaryl, 5-8 membered heteroaryl substituted with one or more R¹⁹, -NR^{1a}R^{1b}, halogen, hydroxy, cyano, nitro, and -SF₅, and when a plurality of the substituents R¹¹, R¹², R¹³, R¹⁴, R¹⁵, R¹⁶, R¹⁷, R¹⁸, and R¹⁹ are present, the substituents are the same or different;
R^{1a} and R^{1b} are each independently selected from H, C₁-C₆ alkyl, and C₁-C₆ alkyl substituted with one or more R^{1a1}, and when a plurality of the substituents R^{1a}, R^{1b}, and R^{1a1} are present, the substituents are the same or different;
R¹¹, R¹², R¹³, R¹⁴, R¹⁵, R¹⁶, R¹⁷, R¹⁸, R¹⁹, and R^{1a1} are each independently selected from halogen, amino, hydroxy, cyano, deuterium, and nitro.

2. The compound, or the tautomer, the stereoisomer, the hydrate, the solvate, the pharmaceutically acceptable salt, or the prodrug thereof as claimed in claim 1, wherein the compound represented by formula (I) is separately compounds represented by formulas (II) to (VI),

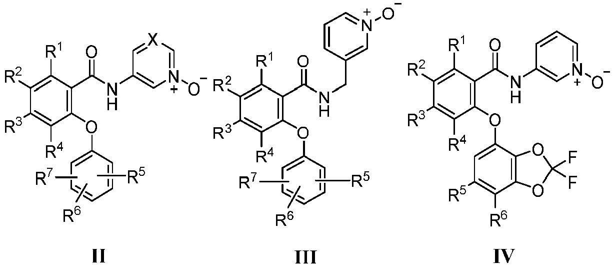

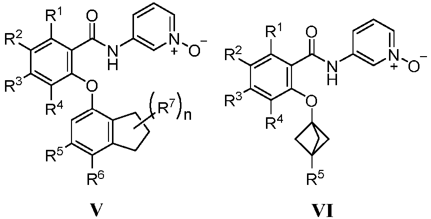

3. The compound, or the tautomer, the stereoisomer, the hydrate, the solvate, the pharmaceutically acceptable salt, or the prodrug thereof as claimed in claim 2, wherein in formula (II),

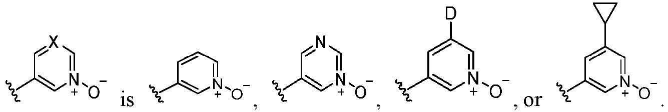

4. The compound, or the tautomer, the stereoisomer, the hydrate, the solvate, the pharmaceutically acceptable salt, or the prodrug thereof as claimed in claim 2, wherein in formula (II), 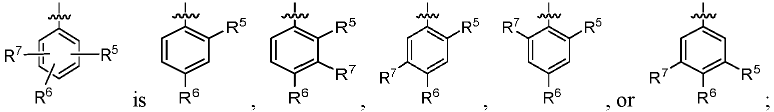
and/or R¹ is H or halogen; preferably, R' is H or F;
and/or R² is halogen, C₂-C₆ alkenyl substituted with one or more R¹², C₂-C₆ alkynyl, C₁-C₆ alkyl substituted with one or more R¹¹, or -S-(C₁-C₆ alkyl) substituted with one or more R¹⁵; preferably, R² is Cl, 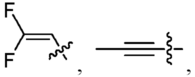 trifluoromethyl, or 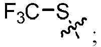
and/or R³ is H, halogen, -S-(C₁-C₆ alkyl) substituted with one or more R¹⁵, or C₁-C₆ alkyl substituted with one or more R¹¹; preferably, R³ is H, Cl, trifluoromethyl, or 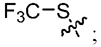
and/or R⁴ is H;
and/or R⁵ is H, D, C₁-C₆ alkyl substituted with one or more R¹¹, C₂-C₆ alkenyl substituted with one or more R¹², - O-(C₁-C₆ alkyl) substituted with one or more R¹⁴, or C₂-C₆ alkynyl; preferably, R⁵ is H, D, methyl, 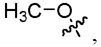 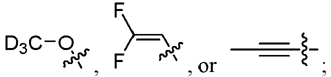
and/or R⁶ is halogen, C₂-C₆ alkenyl substituted with one or more R¹², -O-(C₁-C₆ alkyl) substituted with one or more R¹⁴, or -S-(C₁-C₆ alkyl) substituted with one or more R¹⁵; preferably, R⁶ is F, 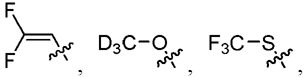 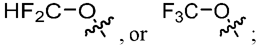
and/or R⁷ is D or halogen; preferably, R⁷ is D or F.

5. The compound, or the tautomer, the stereoisomer, the hydrate, the solvate, the pharmaceutically acceptable salt, or the prodrug thereof as claimed in claim 4, wherein in formula (II), 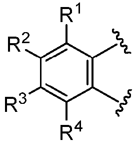 is selected from 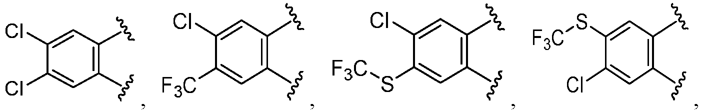 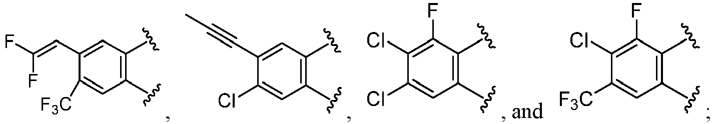 and/or 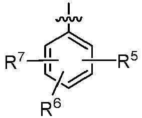 is selected from

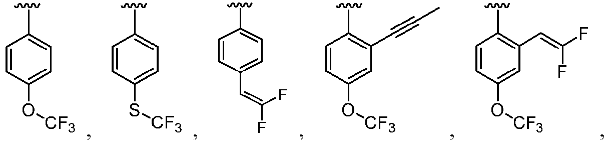

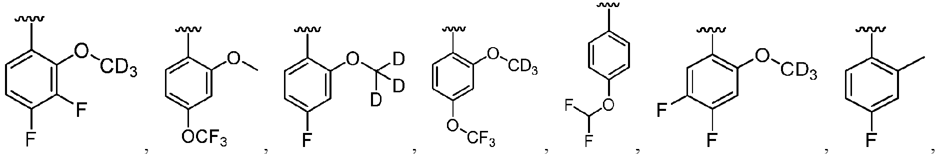

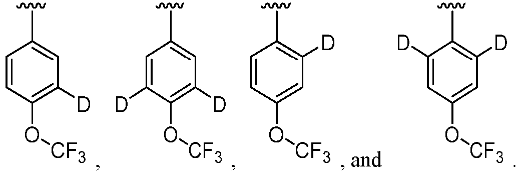

6. The compound, or the tautomer, the stereoisomer, the hydrate, the solvate, the pharmaceutically acceptable salt, or the prodrug thereof as claimed in claim 2, wherein in formula (II), 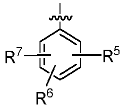 is 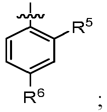
and/or R¹ is H or halogen; preferably, R¹ is H or F;
and/or R² is halogen, C₂-C₆ alkenyl substituted with one or more R¹², C₂-C₆ alkynyl, or -S-(C₁-C₆ alkyl) substituted with one or more R¹⁵; preferably, R² is Cl, 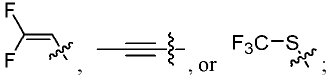
and/or R³ is halogen, -S-(C₁-C₆ alkyl) substituted with one or more R¹⁵, or C₁-C₆ alkyl substituted with one or more R¹¹; preferably, R³ is Cl, trifluoromethyl, or 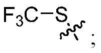
and/or R⁴ is H;
and/or R⁵ is H, C₂-C₆ alkenyl substituted with one or more R¹², or C₂-C₆ alkynyl; preferably, R⁵ is H, 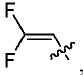 , or 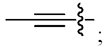
and/or R⁶ is halogen, C₂-C₆ alkenyl substituted with one or more R¹², -O-(C₁-C₆ alkyl) substituted with one or more R¹⁴, or -S-(C₁-C₆ alkyl) substituted with one or more R¹⁵; preferably, R⁶ is F,

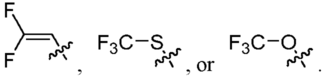

7. The compound, or the tautomer, the stereoisomer, the hydrate, the solvate, the pharmaceutically acceptable salt, or the prodrug thereof as claimed in claim 2, wherein in formula (III), 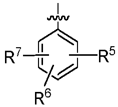 is 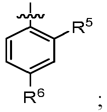
and/or R¹ is H;
and/or R² is halogen; preferably, R² is Cl;
and/or R³ is halogen; preferably, R³ is Cl;
and/or R⁴ is H;
and/or R⁵ is H;
and/or R⁶ is -O-(C₁-C₆ alkyl) substituted with one or more R¹⁴; preferably, R⁶ is

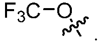

8. The compound, or the tautomer, the stereoisomer, the hydrate, the solvate, the pharmaceutically acceptable salt, or the prodrug thereof as claimed in claim 2, wherein in formula (IV),
R¹ is H or halogen; preferably, R¹ is H or F;
and/or R² is halogen or C₁-C₆ alkyl substituted with one or more R¹¹; preferably, R² is Cl or trifluoromethyl;
and/or R³ is halogen or C₁-C₆ alkyl substituted with one or more R¹¹; preferably, R³ is Cl or trifluoromethyl;
and/or R⁴ is H;
and/or R⁵ is H;
and/or R⁶ is halogen; preferably, R⁶ is F.

9. The compound, or the tautomer, the stereoisomer, the hydrate, the solvate, the pharmaceutically acceptable salt, or the prodrug thereof as claimed in claim 8, wherein in formula (IV), 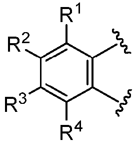 is selected from

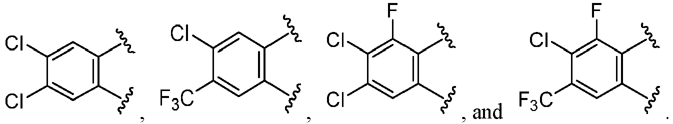

10. The compound, or the tautomer, the stereoisomer, the hydrate, the solvate, the pharmaceutically acceptable salt, or the prodrug thereof as claimed in claim 2, wherein in formula (IV),
R¹ is halogen; preferably, R¹ is F;
and/or R² is halogen; preferably, R² is Cl;
and/or R³ is C₁-C₆ alkyl substituted with one or more R¹¹; preferably, R³ is trifluoromethyl;
and/or R⁴ is H;
and/or R⁵ is H;
and/or R⁶ is halogen; preferably, R⁶ is F.

11. The compound, or the tautomer, the stereoisomer, the hydrate, the solvate, the pharmaceutically acceptable salt, or the prodrug thereof as claimed in claim 2, wherein in formula (V), 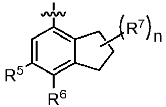 is 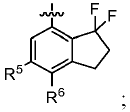
and/or R¹ is halogen; preferably, R¹ is F;
and/or R² is halogen; preferably, R² is Cl;
and/or R³ is C₁-C₆ alkyl substituted with one or more R¹¹; preferably, R³ is trifluoromethyl;
and/or R⁴ is H;
and/or R⁵ is halogen; preferably, R⁵ is F;
and/or R⁶ is halogen; preferably, R⁶ is F.

12. The compound, or the tautomer, the stereoisomer, the hydrate, the solvate, the pharmaceutically acceptable salt, or the prodrug thereof as claimed in claim 2, wherein in formula (VI),
R¹ is H;
and/or R² is halogen; preferably, R² is Cl;
and/or R³ is C₁-C₆ alkyl substituted with one or more R¹¹; preferably, R³ is trifluoromethyl;
and/or R⁴ is H;
and/or R⁵ is -O-(C₁-C₆ alkyl) substituted with one or more R¹⁴; preferably, R⁵ is

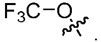

13. The compound, or the tautomer, the stereoisomer, the hydrate, the solvate, the pharmaceutically acceptable salt, or the prodrug thereof as claimed in claim 1, wherein the compound is selected from the following compounds:

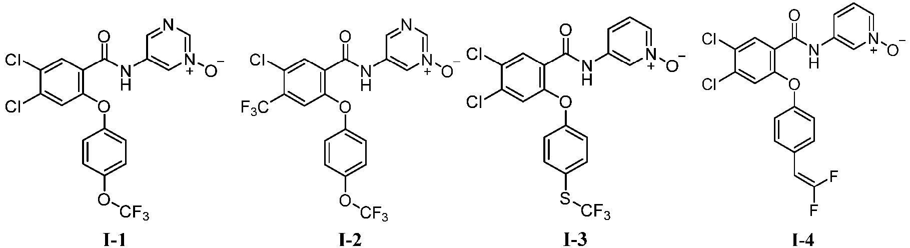

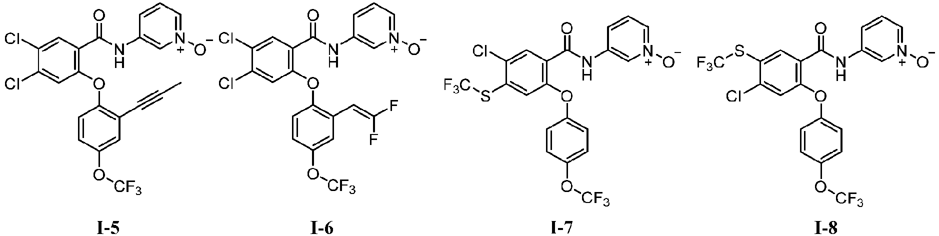

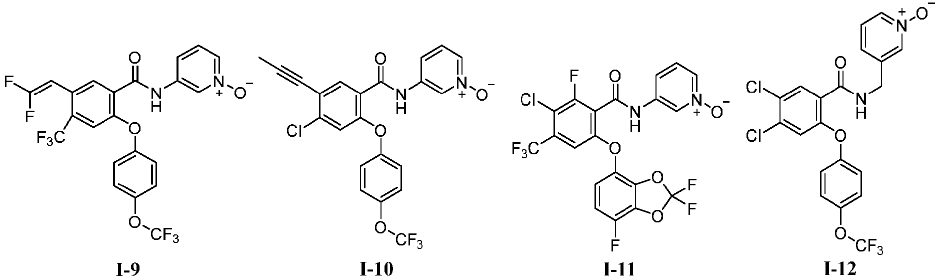

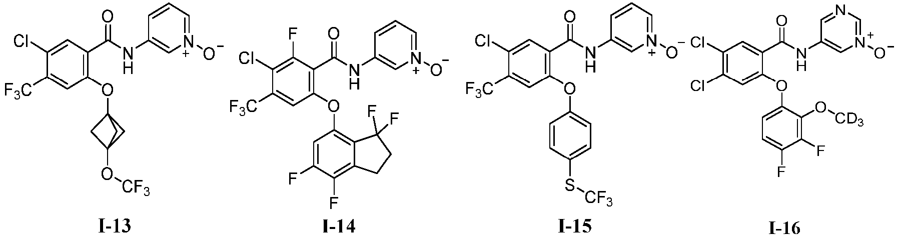

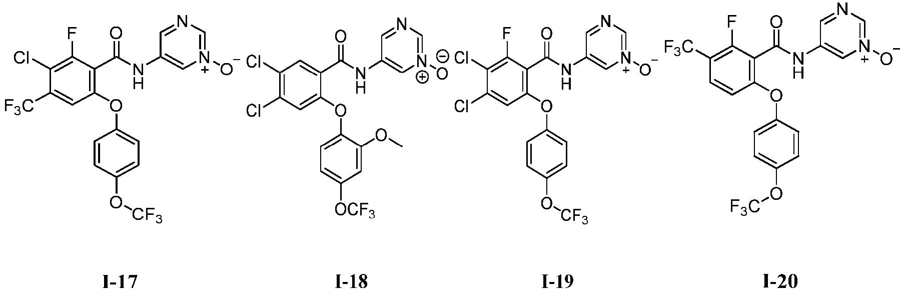

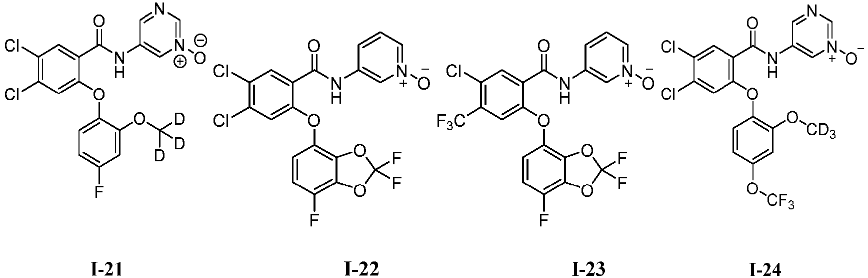

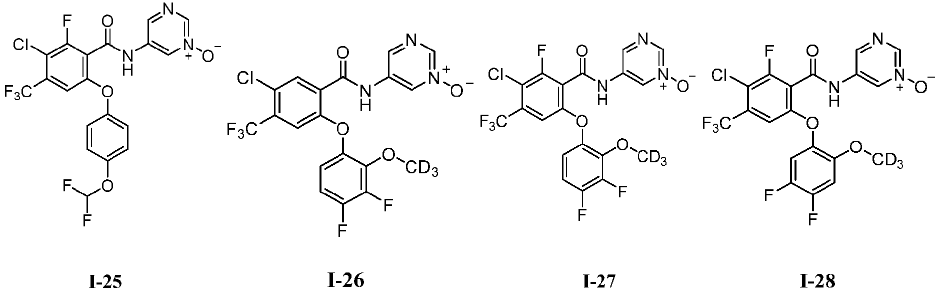

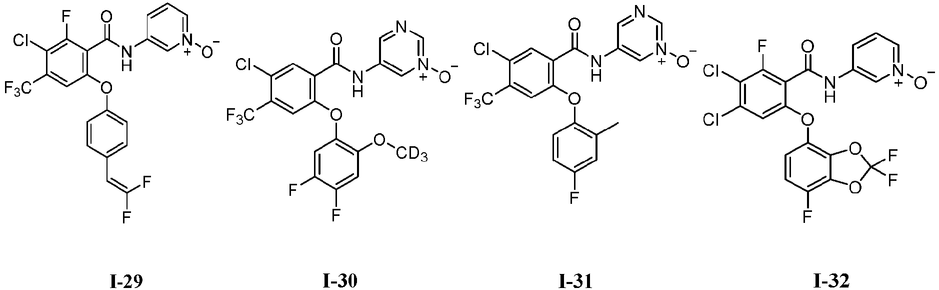

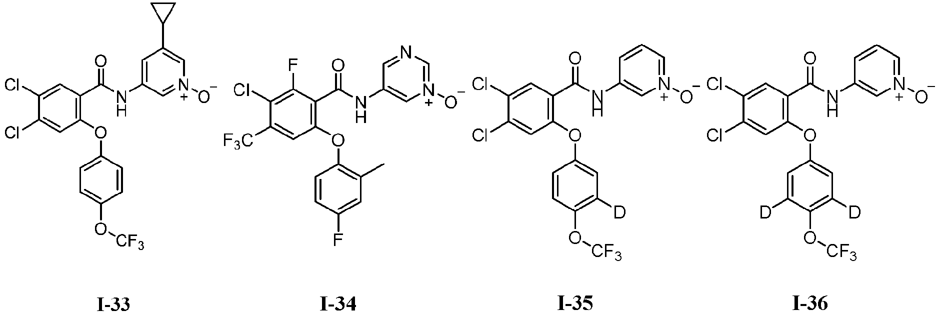

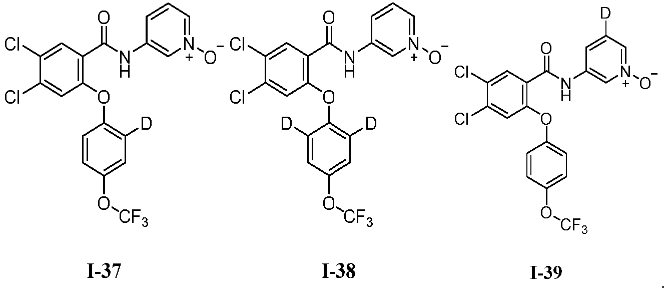

14. A pharmaceutical composition, comprising the compound, or the tautomer, the stereoisomer, the hydrate, the solvate, the pharmaceutically acceptable salt, or the prodrug thereof as claimed in any one of claims 1-13 and a pharmaceutically acceptable excipient.

15. Use of the compound, or the tautomer, the stereoisomer, the hydrate, the solvate, the pharmaceutically acceptable salt, or the prodrug thereof as claimed in any one of claims 1-13 or the composition as claimed in claim 14 for manufacturing a medicament for the inhibition of a voltage-gated sodium channel.

16. The use as claimed in claim 15, wherein the voltage-gated sodium channel is Nav1.8.

17. Use of the compound, or the tautomer, the stereoisomer, the hydrate, the solvate, the pharmaceutically acceptable salt, or the prodrug thereof as claimed in any one of claims 1-13 or the composition as claimed in claim 14 for manufacturing a medicament for the treatment, alleviation, or prevention of pain.

18. The use as claimed in claim 17, wherein the pain comprises acute pain, chronic pain, inflammatory pain, cancer pain, neuropathic pain, musculoskeletal pain, primary pain, intestinal pain, and idiopathic pain.
